# EUROPÄISCHE PATENTSCHRIFT

(11) **EP 2 726 615 B1**
(45) Veröffentlichungstag und Bekanntmachung des Hinweises auf die Patenterteilung: **01.03.2017**
(21) Anmeldenummer: 12728293.7
(22) Anmeldetag: 22.06.2012
(51) Int. Cl.: C12N 9/02, C12P 13/04, C12N 15/77, C12P 13/06, C12P 13/08, C12P 13/10, C12R 1/15, C12N 9/00

(54) **VARIANTEN DES PROMOTORS DES FÜR DIE GLYZERINALDEHYD-3-PHOSPHAT-DEHYDROGENASE KODIERENDEN GAP-GENS**
VARIANTS OF THE PROMOTER OF THE GAP GENE ENCODING GLYCERALDEHYDE-3-PHOSPHATE DEHYDROGENASE
VARIANTS DU PROMOTEUR DU GÈNE GAP CODANT POUR LA GLYCÉRINALDÉHYDE-3-PHOSPHATE-DÉSHYDROGÉNASE

(30) Priorität: 28.06.2011 DE 102011118019
(43) Veröffentlichungstag der Anmeldung: 07.05.2014
(73) Patentinhaber: Evonik Degussa GmbH, 45128 Essen (DE)
(72) Erfinder: RETH, Alexander, 33615 Bielefeld (DE); BATHE, Brigitte, 33154 Salzkotten (DE); HANS, Stephan, 49078 Osnabrück (DE); CLAES, Wilfried, 33619 Bielefeld (DE)
(86) Internationale Anmeldenummer: PCT/EP2012/062046
(87) Internationale Veröffentlichungsnummer: WO 2013/000827

(56) Entgegenhaltungen:
- EP-A1- 1 790 721
- EP-A2- 1 239 040
- WO-A2-03/014330
- WO-A2-2006/138689
- US-A1- 2007 259 408
- US-A1- 2008 050 786
- PÁTEK M ET AL: "Promoters of Corynebacterium glutamicum", JOURNAL OF BIOTECHNOLOGY, ELSEVIER SCIENCE PUBLISHERS, AMSTERDAM, NL, Bd. 104, Nr. 1-3, 4. September 2003 (2003-09-04), Seiten 311-323, XP002323247, ISSN: 0168-1656, DOI: 10.1016/S0168-1656(03)00155-X in der Anmeldung erwähnt
- PÁTEK M ET AL: "Function of Corynebacterium glutamicum promoters in Escherichia coli, Streptomyces lividans, and Bacillus subtilis", JOURNAL OF BIOTECHNOLOGY, ELSEVIER SCIENCE PUBLISHERS, AMSTERDAM, NL, Bd. 104, 1. Januar 2003 (2003-01-01), Seiten 325-334, XP002323103, ISSN: 0168-1656, DOI: 10.1016/S0168-1656(03)00159-7

## Beschreibung

Gegenstand der Erfindung ist ein Verfahren zur Herstellung von Feinchemikalien unter Verwendung von gentechnisch veränderten Mikroorganismen, in denen Varianten des Promotors des für die Glyzerinaldehyd-3-phosphat-Dehydrogenase kodierenden gap-Gens die Überexpression verschiedener Gene ermöglichen.

### Stand der Technik

Feinchemikalien, mit denen insbesondere Aminosäuren, organische Säuren, Vitamine, Nukleoside und Nukleotide gemeint sind, finden in der Humanmedizin, in der pharmazeutischen Industrie, in der Kosmetik, in der Lebensmittelindustrie und in der Tierernährung Anwendung.

Zahlreiche dieser Verbindungen werden durch Fermentation von Stämmen coryneformer Bakterien, insbesondere Corynebacterium glutamicum, hergestellt. Wegen der großen Bedeutung wird ständig an der Verbesserung der Herstellverfahren gearbeitet. Verfahrensverbesserungen können fermentationstechnische Maßnahmen wie zum Beispiel Rührung und Versorgung mit Sauerstoff, oder die Zusammensetzung der Nährmedien wie zum Beispiel die Zuckerkonzentration während der Fermentation, oder die Aufarbeitung zur Produktform durch zum Beispiel Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. das Lysin-Analogon S-(2-Aminoethyl)-Cystein oder das Valin-Analogon 2-Thiazolalanin und chemische Verbindungen beispielsweise L-Aminosäuren wie L-Lysin oder L-Valin produzieren.

Seit einigen Jahren werden ebenfalls Methoden der rekombinanten DNA-Technik zur Stammverbesserung L-Aminosäure produzierender Stämme von Corynebacterium glutamicum eingesetzt, indem man zum Beispiel einzelne Aminosäure-Biosynthesegene verstärkt oder abschwächt und die Auswirkung auf die Produktion der chemischen Verbindung untersucht.

Zusammenfassende Darstellungen zur Biologie, Genetik und Biotechnologie von Corynebacterium glutamicum sind im "Handbook of Corynebacterium glutamicum" (Eds.: L. Eggeling und M. Bott, CRC Press, Taylor & Francis, 2005), in der Spezialausgabe des Journal of Biotechnolgy (Chief Editor: A. Pühler) mit dem Titel "A New Era in Corynebacterium glutamicum Biotechnology" (Journal of Biotechnolgy 104/1-3, (2003)) und im Buch von T. Scheper (Managing Editor) "Microbial Production of L-Amino Acids" (Advances in Biochemical Engineering/Biotechnology 79, Springer Verlag, Berlin, Deutschland, 2003) zu finden.

Die Nukleotidsequenz des Genoms von Corynebacterium glutamicum ist bei Ikeda und Nakagawa (Applied Microbiology and Biotechnology 62, 99-109 (2003)), in der EP 1 108 790 und bei Kalinowski et al. (Journal of Biotechnolgy 104/1-3, (2003)) beschrieben.

Die Nukleotidsequenzen des Genoms von Corynebacterium glutamicum sind ebenfalls in der Datenbank des National Center for Biotechnology Information (NCBI) der National Library of Medicine (Bethesda, MD, USA), in der DNA Data Bank of Japan (DDBJ, Mishima, Japan) oder in der Nukleotidsequenz-Datenbank der European Molecular Biologies Laboratories (EMBL, Heidelberg, Deutschland bzw. Cambridge, UK) verfügbar.

Der natürliche Promotor des für die Glyzerinaldehyd-3-phosphat-Dehydrogenase kodierenden gap-Gens von Corynebacterium glutamicum wurde von Patek et al (Journal of Biotechnology 104, 311-323 (2003)) beschrieben und analysiert.

In der US 2008/0050786 wird in der SEQ ID NO:20 ein 484 bp langes und als seqP-RBS_20 bezeichnetes DNA-Molekül dargestellt, welches den Promotor des gap-Gens einschließlich der Ribosomenbindungsstelle umfasst. Dieser Promotor kann in vorteilhafter Weise zur Expression verschiedener Gene, wie beispielsweise dem für die Aspartatkinase kodierenden Gen lysC, genutzt werden. Der besseren Übersichtlichkeit halber ist die Sequenz unter der SEQ ID NO:1 wiedergegeben.

### Aufgabe der Erfindung

Der Erfindung lag die Aufgabe zugrunde, Varianten des Promotors und/oder der Expressionseinheit des für die Glyzerinaldehyd-3-phosphat-Dehydrogenase kodierenden gap-Gens zur Verfügung zu stellen, unter deren Kontrolle in vorteilhafter Weise verschiedene Gene, wie beispielsweise das für die Aspartatkinase kodierende Gen lysC, exprimiert werden können.

### Beschreibung der Erfindung

Bei den Arbeiten an der vorliegenden Erfindung wurde festgestellt, dass nach Austausch der Nukleobase Guanin an Position 362 des Promotors des gap-Gens von Corynebacterium glutamicum ATCC13032, dargestellt in SEQ ID NO:2, durch die Nukleobase Adenin die Promotoraktivität erhöht ist.

Eine weitere Erhöhung der Promotoraktivität wurde festgestellt nach zusätzlichem Austausch der Nukleobasen Cytosin an Position 265 durch Thymin, Guanin an Position 269 durch Cytosin, Adenin an Position 290 durch Thymin sowie Guanin an Position 292 durch Adenin im Promotor des gap-Gens von Corynebacterium glutamicum ATCC13032, dargestellt in SEQ ID NO:2.

Gegenstand der Erfindung ist daher ein isoliertes Polynukleotid mit Promotoraktivität, welches ein Polynukleotid mit der Nukleotidsequenz dargestellt in SEQ ID NO:3 oder SEQ ID NO:34 umfasst.

Die Ameldung offenbart auch ein isoliertes Polynukleotid mit Promotoraktivität, welches im Wesentlichen aus einem Polynukleotid mit der Nukleotidsequenz dargestellt in SEQ ID NO:3 oder SEQ ID NO:34 besteht. Der Begriff "im Wesentlichen" bedeutet in diesem Zusammenhang, dass am 5'-Ende des Polynukleotids gemäß SEQ ID NO:3 oder SEQ ID NO:34 ein Polynukleotid mit einer Länge von maximal (≤) 1000, maximal (≤) 800, maximal (≤) 700, maximal (≤) 600, maximal (≤) 500 oder maximal (≤) 400 Nukleotiden und am 3'-Ende ein Polynukleotid mit einer Länge von maximal (≤) 15000, maximal (≤) 10000, maximal (≤) 7500, maximal (≤) 5000, maximal (≤) 2500, maximal (≤) 1000, maximal (≤) 800, maximal (≤) 700, maximal (≤) 600, maximal (≤) 500 oder maximal (≤) 400 Nukleotiden angehängt ist.

Erfindungsgemäß ist dabei jede sinnvolle Kombination der Merkmale aus den beiden Listen.
Unter "sinnvolle Kombination" wird zum Beispiel eine Kombination von Merkmalen verstanden, die zur Durchführung einer effizienten Rekombination führt. Der Transfer eines DNA-Bereichs durch homologe Rekombination wird in der experimentellen Durchführung durch die Verwendung von Anhängen gleicher Länge, die den auszutauschenden Bereich flankieren, erleichtert. Für die effiziente Rekombination zwischen ringförmigen DNA-Molekülen sind längere flankierende Homologiebereiche vorteilhaft, die Klonierung des Austauschvektors wird mit Zunahme der Länge der Flanken aber erschwert (Wang et al., Molecular Biotechnology 32:43-53 (2006)). Deshalb ist die spezifische Kombination von Anhängen von jeweils 600 bis maximal (≤) 1000 Nukleotiden bevorzugt, besonders bevorzugt sind Anhänge von jeweils 750 bis 850 Nukleotiden.

Gegenstand der Erfindung ist weiterhin ein isoliertes Polynukleotid mit Promotoraktivität, welches aus der Nukleotidsequenz dargestellt in SEQ ID NO:3 oder SEQ ID NO:34 besteht.

Einzelheiten zur Biochemie bzw. chemischen Struktur von Polynukleotiden, wie sie in Lebewesen, wie beispielsweise Mikroorganismen, vorkommen, findet man unter anderem im Lehrbuch "Biochemie" von Berg et al (Spektrum Akademischer Verlag Heidelberg·Berlin, Deutschland, 2003; ISBN 3-8274-1303-6).

Besteht das Polynukleotid aus Desoxyribonukleotid-Monomeren mit den Nukleobasen bzw. Basen Adenin (A), Guanin (G), Cytosin (C) und Thymin (T) so spricht man von Desoxyribo-Polynukleotiden oder Desoxyribonukleinsäure (DNA). Besteht das Polynukleotid aus Ribonukleotid-Monomeren mit den Nukleobasen bzw. Basen Adenin (A), Guanin (G), Cytosin (C) und Uracil (U) so spricht man von Ribo-Polynukleotiden oder Ribonukleinsäure (RNA). In den genannten Polynukleotiden sind die Monomere durch eine 3'→ 5'-Phosphodiesterbindung miteinander kovalent verbunden.

Unter einem "Polynukleotid mit Promotoraktivität" oder einem "Promotor" wird ein Polynukleotid, bevorzugt Desoxyribo-Polynukleotid, bzw. eine Nukleinsäure, bevorzugt Desoxyribonukleinsäure (DNA) verstanden, das/die in funktioneller Verknüpfung mit einem zu transkribierenden Polynukleotid den Initiationspunkt und die Initiationshäufigkeit der Transkription dieses Polynukleotids festlegt, wodurch die Expressionsstärke des kontrollierten Polynukleotids beeinflußt werden kann.

Aufgrund der doppelsträngigen Struktur der DNA ist der zum Strang des Sequenzprotokolls in SEQ ID NO:3 oder SEQ ID NO:34 komplementäre Strang ebenfalls Gegenstand der Erfindung.

Unter "Transkription" wird der Prozess verstanden, durch den ausgehend von einer DNA-Matrize ein komplementäres RNA-Molekül hergestellt wird. An diesem Prozess sind Proteine wie die RNA-Polymerase, sogenannte Sigma-Faktoren und transkriptionelle Regulatorproteine beteiligt. Die synthetisierte RNA (Messenger-RNA, m-RNA) dient dann als Matrize im Prozess der Translation, der dann zum Polypeptid bzw. Protein führt.

Ein Gen ist chemisch gesehen ein Polynukleotid. Ein Polynukleotid, das ein Protein/Polypeptid kodiert, wird hier synonym zum Begriff "Gen" gebraucht. Demnach werden die beiden Begriffe "Gen" und "Kodierregion" synonym verwendet und gleichermaßen die beiden Begriffe "Protein" und "Polypeptid".

Unter einer "funktionellen Verknüpfung" versteht man in diesem Zusammenhang die sequentielle Anordnung des erfindungsgemässen Polynukleotids mit Promotoraktivität mit einem weiteren Oligo- oder Polynukleotid, die zu einer Transkription des weiteren Polynukleotids führt.

Handelt es sich bei dem weiteren Polynukleotid um ein im Folgenden als zweites Polynukleotid bezeichnetes Polynukleotid, das für ein Polypeptid/Protein kodiert, bestehend aus der Kodierregion für ein Polypeptid beginnend mit einem Start-Kodon, einschließlich des Stop-Kodons und ggf. einschließlich eines Transkriptionsterminators, so bedeutet "funktionelle Verknüpfung" die sequentielle Anordnung des erfindungsgemässen Polynukleotids mit Promotoraktivität mit dem zweiten Polynukleotid, die zu einer Transkription des zweiten Polynukleotids und der Translation der synthetisierten RNA führt.

Das zweite Polynukleotid kodiert für ein oder mehrere Polypeptid(e). Ein für ein Protein/Polypeptid kodierendes Polynukleotid besteht im wesentlichen aus einem Startkodon, ausgewählt aus der Gruppe ATG, GTG und TTG, bevorzugt ATG oder GTG, besonders bevorzugt ATG, einer proteinkodierenden Sequenz und einem oder mehreren Stop-Kodon(en) ausgewählt aus der Gruppe TAA, TAG und TGA.

Kodiert das zweite Polynukleotid für mehrere Proteine/Polypeptide, so kann sich vor jedem Gen eine Ribosomenbindestelle befinden. Hinter dem letzten Gen befindet sich ggf. ein Terminator.

Bevorzugt kodiert das zweite Polynukleotid für ein oder mehrere Polypeptide bzw. Proteine aus dem Biosyntheseweg von Feinchemikalien, bevorzugt ausgewählt aus der Gruppe proteinogene Aminosäuren, nicht proteinogene Aminosäuren, Vitamine, Nukleoside, Nukleotide und organische Säuren. Besonders bevorzugt werden proteinogene Aminosäuren, nicht proteinogene Aminosäuren, und organische Säuren.

Das zweite Polynukleotid besteht vorzugsweise aus einem oder mehreren der in Tabelle 1 von EP 1 108 790 A2 ausgewiesenen Polynukleotide, welche hiermit per Referenz einbezogen wird.

Bevorzugt besteht dabei das zweite Polynukleotid aus einem oder mehreren der Gene bzw. Polynukleotide, die für Enzyme des Pentosephoshatweges kodieren, ausgewählt aus der Gruppe:
a) Polynukleotid (zwf-Gen), das für die Zwf-Untereinheit der Glucose-6-phosphate-1-dehydrogenase (Zwf, EC NR.: 1.1.1.49) kodiert,
b) Polynukleotid (opcA-Gen), das für die OpcA Untereinheit der Glucose-6-phosphate-1-dehydrogenase (OpcA, EC NR.: 1.1.1.49) kodiert,
c) Polynukleotid (devB-Gen), das für eine 6-Phosphogluconolactonase (DevB, EC NR.: 3.1.1.31) kodiert,
d) Polynukleotid (tkt-Gen), das für eine Transketolase (Tkt, EC NR.: 2.2.1.1) kodiert,
e) Polynukleotid (tal-Gen), das für eine Transaldolase (Tal, EC NR.: 2.2.1.2) kodiert,
f) Polynukleotid (gnd-Gen), das für eine 6-Phosphogluconat Dehydrogenase (Gnd, EC NR.: 1.1.1.44) kodiert,
g) Polynukleotid (rpe-Gen), das für eine Ribulose-Phosphat-3-Epimerase (Rpe, EC NR.: 5.1.3.1) kodiert, und
h) Polynukleotid (rpi-Gen), das für eine Ribose-5-phosphat Isomerase B (Rpi, EC NR.: 5.3.1.6) kodiert,
wobei die Gene zwf und opcA besonders bevorzugt werden.

Weiterhin bevorzugt besteht das zweite Nukleotid aus einem oder mehreren der Gene bzw. Polynukleotide, die für Enzyme der Anaplerotik und der Glukoneogenese kodieren, ausgewählt aus der Gruppe:
i) Polynukleotid (ppc-Gen), das für eine Phosphoenolpyruvat Carboxylase (Ppc,EC NR.: 4.1.1.31) kodiert,
j) Polynukleotid (fbp-Gen), das für eine Fructose-1,6-bisphosphatase (Fbp, EC NR.: 3.1.3.11) kodiert, und
k) Polynukleotid (pyc-Gen), das für eine Pyruvat-Carboxylase (Pyc, EC NR.: 6.4.1.1) kodiert,
wobei das pyc-Gen besonders bevorzugt wird.

Im Zusammenhang mit der Herstellung von L-Lysin besteht das zweite Polynukleotid bevorzugt aus einem oder mehreren der Gene bzw. Polynukleotide, die für Enzyme der Biosynthese von L-Lysin kodieren, ausgewählt aus der Gruppe:
1) Polynukleotid (dapA-Gen), das für eine Dihydrodipicolinat-Synthase (DapA, EC Nr.: 4.2.1.52) kodiert,
m) Polynukleotid (asd-Gen), das für eine Aspartatsemialdehyd-Dehydrogenase (Asd, EC Nr.: 1.2.1.11) kodiert,
n) Polynukleotid (ddh-Gen), das für eine meso-Diaminopimelate Dehydrogenase (Ddh, EC Nr.: 1.4.1.16) kodiert,
o) Polynukleotid, (lysA-Gen), das für eine Diaminopimelat-Decarboxylase (LysA, EC Nr.: 4.1.1.20) kodiert,
p) Polynukleotid (aat-Gen), das für eine Aspartat-Aminotransferase (Aat, EC Nr.: 2.6.1.1) kodiert,
q) Polynukleotid (lysE-Gen), das für ein Polypeptid mit L-Lysin-Export Aktivität (LysE, Lysin Efflux Permease) kodiert,
r) Polynukleotid (dapB-Gen), das für eine Dihydrodipicolinat-Reduktase (DapB, EC Nr.: 1.3.1.26) kodiert,
s) Polynukleotid (lysC-Gen), das für eine Aspartatkinase (LysC, EC NR.: 2.7.2.4) kodiert,
t) Polynukleotid (dapC-Gen), das für eine Succinyldiaminopimelate Aminotransferase, AT class I (DapC, EC Nr.: 2.6.1.17) kodiert,
u) Polynukleotid (dapD-Gen), das für eine Tetrahydrodipicolinat Succinylase (DapD, EC NR.: 2.3.1.117) kodiert,
v) Polynukleotid (dapE-Gen), das für eine Succinyldiaminopimelat Desuccinylase (DapE, EC NR.: 3.5.1.18) kodiert, und
w) Polynukleotid (dapF-Gen), das für eine Diaminopimelat Epimerase (DapF, EC NR.: 5.1.1.7) kodiert,
wobei die Gene dapA, asd, ddh, lysA, lysE, dapB und lysC besonders bevorzugt und die Gene asd und lysC ganz besonders bevorzugt werden, da insbesondere mit den beiden letztgenannten eine besondere zusätzliche Verstärkung der Produktion von L-Lysin erreicht wird.

Im Zusammenhang mit der Herstellung von L-Valin, L-Isoleucin, α-Ketoisovaleriansäure, α-Keto-β-methylvaleriansäure oder α-Ketoisocapronsäure besteht das zweite Polynukleotid bevorzugt aus einem oder mehreren der Gene bzw. Polynukleotide, die für Enzyme der Biosynthese von L-Valin, L-Isoleucin, α-Ketoisovaleriansäure, α-Keto-β-methylvaleriansäure oder α-Ketoisocapronsäure kodieren, ausgewählt aus der Gruppe:
x) Polynukleotide (ilvB-Gen und ilvN-Gen), die für die Untereinheiten einer Acetolactate Synthase (IlvBN, EC Nr.: 4.1.3.18) kodieren,
y) Polynukleotid (ilvC-Gen), das für eine Isomeroreductase (IlvC, EC Nr.: 1.1.1.86) kodiert,
z) Polynukleotid (ilvD-Gen), das für eine Dihydroxy-acid Dehydratase (IlvD, EC Nr.: 4.2.1.9) kodiert,
aa) Polynukleotid (ilvE-Gen), das für eine Transaminase (IlvE, EC Nr.: 2.6.1.42) kodiert,
bb) Polynukleotid (ilvA-Gen) das für eine Threonindehydratase (IlvA, EC Nr.: 4.3.1.19) kodiert,
cc) Polynukleotid (hom-Gen) das für eine Homoserindehydrogenase (Hom, EC-Nr.: 1.2.1.11 kodiert
dd) Polynukleotid (thrB-Gen), das für eine Homoserinkinase (ThrB, EC-Nr.: 2.7.1.39) kodiert
ee) Polynukleotid (thrC-Gen), das für eine Threoninsynthase (ThrC, EC-Nr.: 4.2.3.1) kodiert
ff) Polynukleotid (leuA-Gen), das für eine Isopropylmalatsynthase (LeuA, EC-Nr.: 2.3.3.13) kodiert
gg) Polynukleotid (leuB-Gen), das für eine Isopropylmalatdehydrogenase (LeuB, EC-Nr.: 1.1.1.85) kodiert
hh) Polynukleotid (leuC-Gen), das für die große Untereinheit einer Isopropylmalatisomerase (LeuC, EC-Nr.: 4.2.1.33) kodiert
ii) Polynukleotid (leuD-Gen), das für die kleine Untereinheit einer Isopropylmalatisomerase (LeuD, EC-Nr.: 4.2.1.33) kodiert
wobei die Gene ilvBN, hom, ilvA, ilvC, ilvD und ilvE für Isoleucin und Valin besonders bevorzugt werden, wobei die Gene ilvBN, ilvC und ilvD für α-Ketovaleriansäure (KIV) und α-Keto-β-methylvaleriansäure (KMV) besonders bevorzugt werden und wobei die Gene ilvBN, ilvC, ilvD, leuA, leuB, leuC und leuD für α-Ketoisokapronsäure (KIC) besonders bevorzugt werden.

Im Zusammenhang mit der Herstellung von L-Ornithin besteht das zweite Polynukleotid bevorzugt aus einem oder mehreren der Gene bzw. Polynukleotide, die für Enzyme der Biosynthese von L-Ornithin kodieren, ausgewählt aus der Gruppe:
jj) Polynukleotid (lysE-Gen), das für einen Lysin-/Ornithintransporter (DE-Anmeldenummer 102010003419.3) kodiert.
kk) Polynukleotid (argB-Gen), das für eine N-Acetylglutamatkinase (ArgB, EC-NR.: 2.7.2.8) kodiert
11) Polynukleotid (gdh-Gen), das für eine Glutamat-Dehydrogenase (Gdh, EC-Nr.: 1.4.1.3) kodiert,
mm) Polynukleotid (argJ-Gen), das für eine Glutamat N-Acetyltransferase (ArgJ, EC-Nr.: 2.3.1.35 und EC-Nr.: 2.3.1.1) kodiert,
nn) Polynukleotid (argC-Gen), das für eine N-Acetyl-Gamma-Glutamyl-Phosphat-Reduktase (ArgC, EC-Nr.: 1.2.1.38) kodiert, und
oo) Polynukleotid (argD-Gen), das für eine Acetyl-Ornithin Aminotransferase (ArgD, EC-Nr.: 2.6.1.11), kodiert
wobei die Gene lysE und argB besonders bevorzugt werden.

Der erfindungsgemäße Promotor kann somit in jedem der Fälle dazu verwendet werden, das zweite Polynukleotid in Corynebacterium glutamicum zu (über)exprimieren.

Das zweite Polynukleotid wird hinter den erfindungsgemässen Promotor, d.h. am 3'-Ende, positioniert, so dass beide Polynukleotide direkt oder mittels eines Brücken-Oligonukleotids oder Brücken-Polynukleotids funktionsfähig miteinander verbunden sind. Bevorzugt sind beide Polynukleotide mittels eines Brücken-Oligonukleotids oder Brücken-Polynukleotids funktionsfähig miteinander verbunden.

Dieses Brücken-Oligonukleotid oder Brücken-Polynukleotid besteht aus Desoxyribonukleotiden.

Der Ausdruck "direkt funktionsfähig miteinander verbunden" bedeutet in diesem Zusammenhang, dass das Nukleotid mit dem Adenosin-Nukleotid an Position 461 am 3'-Ende von SEQ ID NO:3 oder SEQ ID NO:34 direkt mit dem dem ersten Nukleotid des Startkodons einer Kodierregion verbunden ist. Hieraus resultieren sogenannte "leaderless" mRNAs, welche direkt mit dem 5'-terminalen AUG Startkodon beginnen und somit keine weiteren Translationsinitiationssignale aufweisen.

Der Ausdruck "mittels eines Brücken-Oligonukleotids funktionsfähig miteinander verbunden" bedeutet in diesem Zusammenhang, dass das Nukleotid mit dem Adenosin-Nukleotid an Position 461 am 3'-Ende von SEQ ID NO:3 oder SEQ ID NO:34 durch ein Oligonukleotid mit einer Länge von 1, 2, 3, 4 oder 5 Nukleotiden mit dem ersten Nukleotid des Startkodons einer Kodierregion verbunden ist.

Der Ausdruck "mittels eines Brücken-Polynukleotids funktionsfähig miteinander verbunden" bedeutet in diesem Zusammenhang, dass das Nukleotid mit dem Adenosin-Nukleotid an Position 461 am 3'-Ende von SEQ ID NO:3 oder SEQ ID NO:34 durch ein Polynukleotid mit einer Länge von 6 bis maximal (≤) 600 Nukleotiden mit dem ersten Nukleotid des Startkodons einer Kodierregion verbunden ist.

Der Ausdruck "funktionsfähig miteinander verbunden" bedeutet in diesem Zusammenhang, dass das zweite Polynukleotid derart an das erfindungsgemässe Polynukleotid mit Promotoraktivität gebunden ist, dass die Transkription des zweiten Polynukleotids und die Translation der synthetisierten RNA gewährleistet ist.

Je nach technischer Anforderung hat das Brücken-Polynukleotid eine Länge von
6 - 600, 6 - 500, 6 - 400, 6 - 300, 6 - 200, 6 - 180, 6 - 160, 6 - 140, 6 - 120, 6 - 100, 6 - 80, 6 - 60, 6 - 50, 6 - 40, 6 - 30, 6 - 28, 6 - 27, 6 - 26, 6 - 25 oder
8 - 600, 8 - 500, 8 - 400, 8 - 300, 8 - 200, 8 - 180, 8 - 160, 8 - 140, 8 - 120, 8 - 100, 8 - 80, 8 - 60, 8 - 50, 8 - 40, 8 - 30, 8 - 28, 8 - 27, 8 - 26, 8 - 25 oder
10 - 600, 10 - 500, 10 - 400, 10 - 300, 10 - 200, 10 - 180, 10 - 160, 10 - 140, 10 - 120, 10 - 100, 10 - 80, 10 - 60, 10 - 50, 10 - 40, 10 - 30, 10 - 28, 10 - 27, 10 - 26, 10 - 25 oder
12 - 600, 12 - 500, 12 - 400, 12 - 300, 12 - 200, 12 - 180, 12 - 160, 12 - 140, 12 - 120, 12 - 100, 12 - 80, 12 - 60, 12 - 50, 12 - 40, 12 - 30, 12 - 28, 12 - 27, 12 - 26, 12 - 25 oder
14 - 600, 14 - 500, 14 - 400, 14 - 300, 14 - 200, 14 - 180, 14 - 160, 14 - 140, 14 - 120, 14 - 100, 14 - 80, 14 - 60, 14 - 50, 14 - 40, 14 - 30, 14 - 28, 14 - 27, 14 - 26, 14 - 25 oder
16 - 600, 16 - 500, 16 - 400, 16 - 300, 16 - 200, 16 - 180, 16 - 160, 16 - 140, 16 - 120, 16 - 100, 16 - 80, 16 - 60, 16 - 50, 16 - 40, 16 - 30, 16 - 28, 16 - 27, 16 - 26, 16 - 25 oder
18 - 600, 18 - 500, 18 - 400, 18 - 300, 18 - 200, 18 - 180, 18 - 160, 18 - 140, 18 - 120, 18 - 100, 18 - 80, 18 - 60, 18 - 50, 18 - 40, 18 - 30, 18 - 28, 18 - 27, 18 - 26, 18 - 25 oder
20 - 600, 20 - 500, 20 - 400, 20 - 300, 20 - 200, 20 - 180, 20 - 160, 20 - 140, 20 - 120, 20 - 100, 20 - 80, 20 - 60, 20 - 50, 20 - 40, 20 - 30, 20 - 28, 20 - 27, 20 - 26, 20 - 25 Nukleotiden.

In besonders bevorzugten Ausführungsformen hat das Brücken-Polynukleotid eine Länge von 20, 21, 22, 23, 24 oder 25 Nukleotiden, weil hier vorzugsweise funktionsfähige Konstrukte entstehen, wie auch in den Beispielen 3 bis 5 erkennbar ist.

Bei dem Brücken-Polynukleotid handelt es sich bevorzugt um ein Polynukleotid, welches eine Nukleotidsequenz umfasst die die Translation der synthetisierten RNA gewährleistet. Eine derartige Sequenz wird in der Fachwelt auch als Ribosomenbindestelle (RBS) oder auch als Shine Dalgarno Sequenz bezeichnet.

Ein weiterer Gegenstand der Erfindung ist dementsprechend ein isoliertes Polynukleotid, bestehend aus einem Polynukleotid gemäß SEQ ID NO:3 oder SEQ ID NO:34, welches mit dem Adenosin-Nukleotid an Position 461 am 3'-Ende mit einem zweiten Polynukleotid, welches am 5'-Ende ein ATG oder GTG Startkodon enthält und für ein oder mehrere Polypeptid(e) kodiert, direkt oder mittels eines Brücken-Polynukleotids, welches die Translation von RNA gewährleistet, funktionsfähig miteinander verbunden ist. Bevorzugt sind beide Polynukleotide mittels eines Brücken-Polynukleotids funktionsfähig miteinander verbunden.

Ein weiterer Gegenstand der Erfindung ist auch ein isoliertes Polynukleotid, bestehend aus einem Polynukleotid gemäß SEQ ID NO:3 oder SEQ ID NO:34, welches mit dem Adenosin-Nukleotid an Position 461 am 3'-Ende mit einem Brücken-Oligonukleotid funktionsfähig verbunden ist.

Ein weiterer Gegenstand der Erfindung ist weiterhin ein isoliertes Polynukleotid, bestehend aus einem Polynukleotid gemäß SEQ ID NO:3 oder SEQ ID NO:34, welches mit dem Adenosin-Nukleotid an Position 461 am 3'-Ende mit einem Brücken-Polynukleotid, welches die Translation von RNA gewährleistet, funktionsfähig verbunden ist.

Die Ribosomenbindestellen der Gattung Corynebacterium, bevorzugt der Art Corynebacterium glutamicum, sind gut beschrieben. Von Amador et al. (Microbiology, 145, 915-924 (1999)) wurde die Anti-Shine-Dalgarno Sequenz der 16S rRNA von Corynebacterium glutamicum bestimmt; sie ist in SEQ ID NO:4 dargestellt. Die von Martin et al. (Journal of Biotechnology 104, 41-53 (2003)) angegebene Sequenz ist in SEQ ID NO:5 wiedergegeben.

Weitere Beispiele für geeignete Ribosomenbindestellen sind unter anderem
die Ribosomenbindestelle des Gens ppc von C. glutamicum ATCC13032 (O'Regan et al., Gene 77, 237-251 (1989)), dargestellt in SEQ ID NO:6; die Ribosomenbindestelle des Gens aceA von C. glutamicum ATCC13032 (Reinscheid et al., Journal of Bacteriology 176 (12), 3474-3483 (1994)), dargestellt in SEQ ID NO:7;
die Ribosomenbindestelle des Gens thiX von C. glutamicum ATCC13032 (Reinscheid et al., Journal of Bacteriology 176 (12), 3474-3483 (1994)), dargestellt in SEQ ID NO:8;
die Ribosomenbindestelle des Gens sod von C. glutamicum ATCC13032 (WO2005/059144), dargestellt in SEQ ID NO:9;
die Ribosomenbindestelle des Gens tuf von C. glutamicum ATCC13032 (WO2005/059093), dargestellt in SEQ ID NO:10;
die Ribosomenbindestelle gemäß SEQ ID NO:44 von EP 1918378, dargestellt in SEQ ID NO:11;
die Ribosomenbindestelle des Gens fbp von C. glutamicum ATCC13032, dargestellt in SEQ ID NO:12;
die Ribosomenbindestelle des Gens gap von C. glutamicum ATCC13032 (Eikmanns, Journal of Bacteriology 174 (19), 6076-6086 (1992)) dargestellt in SEQ ID NO:13.

Bevorzugt enthält das Brücken-Polynukleotid die Ribosomenbindestelle des Gens gap dargestellt in SEQ ID NO:13 oder des Gens fbp dargestellt in SEQ ID NO:12.

Besonders bevorzugte Ausführungsformen des Brücken-Polynukleotids, welche die Ribosomenbindestelle des Gens gap enthalten sind in SEQ ID NO:14, SEQ ID NO:15, SEQ ID NO:16 und SEQ ID NO:17 dargestellt.

Eine besonders bevorzugte Ausführungsform des Brücken-Polynukleotids, welche die Ribosomenbindestelle des Gens fbp enthält ist in SEQ ID NO:18 dargestellt.

Diese besonders bevorzugten Ausführungsformen des Brücken-Polynukleotids gewährleisten die Translation von RNA in vorteilhafter Weise.

Zur Erleichterung der chemischen Verknüpfung zwischen dem erfindungsgemäßen Polynukleotid mit der Nukleotidsequenz gemäß SEQ ID NO:3 oder SEQ ID NO:34, dem Brücken-Polynukleotid, welches die Translation von RNA gewährleistet und dem zweiten Polynukleotid, kodierend für ein oder mehrere Polypeptid(e), das am 5'-Ende ein ATG oder GTG Startkodon aufweist, können in diese Polynukleotide an ihren 5'- und 3'-Enden für die Klonierung benötigte funktionelle Nukleotidsequenzen eingebaut werden, die auch nach der Klonierung zumindest teilweise erhalten bleiben.

Der Begriff "für die Klonierung benötigte funktionelle Nukleotidsequenz" steht hier für jede beliebige vorhandene REII (Restriktionsendonuklease des Typs II) -Schnittstelle, deren Sequenz in der Regel aus 4 bis 8 Nukleotiden besteht.

Ergänzend sei an dieser Stelle erwähnt, dass durch ortsspezifische Mutagenese mittels Mutageneseprimern oder bei einer de novo Gensynthese (z.B. durch die Firma GENEART AG (Regensburg, Deutschland)) der Nukleotidsequenzen zur Entfernung von Schnittstellen für Restriktionsendonukleasen stille Mutationen innerhalb der Sequenz eingeführt werden können, damit diese Schnittstellen für spätere Klonierungsschritte vorteilhaft verwendet werden können.

Das Polynukleotid, das durch die funktionelle Verknüpfung des erfindungsgemäßen Promotors mit dem Brücken-Polynukleotid entsteht, welches die Translation von RNA gewährleistet, wird im Folgenden auch als Expressionseinheit bezeichnet.

Gegenstand der Erfindung ist weiterhin die Verwendung des erfindungsgemäßen Promotors oder der erfindungsgemäßen Expressionseinheit zur Expression von gewünschten Genen bzw. Polynukleotiden in Mikroorganismen. Der erfindungsgemäße Promotor oder die erfindungsgemäße Expressionseinheit gewährleistet die Transkription und die Translation der synthetisierten RNA, bevorzugt mRNA, zu einem Polypeptid.

Die Expression wird bevorzugt in Mikroorganismen der Gattung Corynebacterium durchgeführt. Innerhalb der Gattung Corynebacterium werden Stämme bevorzugt, die auf folgenden Arten beruhen: Corynebacterium efficiens, wobei der Typstamm als DSM44549 hinterlegt ist, Corynebacterium glutamicum, wobei der Typstamm als ATCC13032 hinterlegt ist, und Corynebacterium ammoniagenes, wobei der Typstamm als ATCC6871 hinterlegt ist. Die Art Corynebacterium glutamicum wird ganz besonders bevorzugt.

Auf diese Weise können Polynukleotide zur Expression gebracht werden, die für Polypeptide mit einer Eigenschaft, bevorzugt Enzymaktivität, kodieren, die im entsprechenden Wirt nicht vorhanden oder nachweisbar sind. So beschreiben Yukawa et al. zum Beispiel die Expression von Genen aus Escherichia coli zur Verwertung von D-Xylose in Corynebacterium glutamicum R unter Kontrolle des konstitutiven Ptrc-Promotors (Applied Microbiology and Biotechnology 81, 691-699 (2008)).

Der erfindungsgemäße Promotor oder die erfindungsgemäße Expressionseinheit wird weiterhin dazu verwendet gewünschte Gene bzw. Polynukleotide in Mikroorganismen zu überexprimieren (Überexpression).

Unter Überexpression versteht man allgemein eine Erhöhung der intrazellulären Konzentration oder Aktivität einer Ribonukleinsäure, eines Proteins (Polypeptids) oder eines Enzyms im Vergleich zum Ausgangsstamm (Elternstamm) oder Wildtypstamm, wenn dies der Ausgangsstamm ist. Unter einem Ausgangsstamm (Elternstamm) versteht man den Stamm, an dem die zur Überexpression führende Maßnahme durchgeführt wurde.

Bei der Überexpression werden die Methoden der rekombinanten Überexpression bevorzugt. Hierunter werden alle Methoden zusammengefasst bei denen ein Mikroorganismus unter Verwendung eines in-vitro bereitgestellten DNA-Moleküls hergestellt wird. Derartige DNA-Moleküle umfassen beispielsweise Promotoren, Expressionskassetten, Gene, Allele, Kodierregionen etc.. Diese werden durch Methoden der Transformation, Konjugation, Transduktion oder gleichartige Methoden in den gewünschten Mikroorganismus überführt.

Im Falle der vorliegenden Erfindung handelt es sich bei den Promotoren bevorzugt um ein Polynukleotid gemäß SEQ ID NO:3 oder SEQ ID NO:34 und bei den Expressionskassetten bevorzugt um ein Polynukleotid gemäß SEQ ID NO:3 oder SEQ ID NO:34, welches mit dem Adenosin-Nukleotid an Position 461 am 3'-Ende mit einem Brücken-Polynukleotid, welches die Translation von RNA gewährleistet, funktionsfähig verbunden ist.

Durch die Maßnahmen der Überexpression unter Verwendung des erfindungsgemäßen Promotors oder der erfindungsgemäßen Expressionseinheit wird die Aktivität oder Konzentration des entsprechenden Polypeptids im Allgemeinen um mindestens 10%, 25%, 50%, 75%, 100%, 150%, 200%, 300%, 400% oder 500%, bevorzugt maximal bis 1000%, 2000%, 4000%, 10000% oder 20000% bezogen auf die Aktivität oder Konzentration des Polypeptids im Stamm vor der zur Überexpression führenden Maßnahme erhöht.

Das Ausmaß der Expression beziehungsweise Überexpression kann durch Messung der Menge der vom Gen transkribierten mRNA, durch Bestimmung der Menge des Polypeptids und durch Bestimmung der Enzymaktivität festgestellt werden.

Für die Bestimmung der Menge an mRNA können unter anderem die Methode des "Northern Blotting's" und der quantitativen RT-PCR verwendet werden. Bei der quantitativen RT-PCR wird der Polymerase-Kettenreaktion eine reverse Transkription vorgeschaltet. Hierzu kann das LightCycler^{™} System der Firma Roche Diagnostics (Boehringer Mannheim GmbH, Roche Molecular Biochemicals, Mannheim, Deutschland) verwendet werden, wie beispielsweise bei Jungwirth et al. (FEMS Microbiology Letters 281, 190-197 (2008)) beschrieben. Die Konzentration des Proteins kann über 1- und 2-dimensionale Proteingelauftrennung und anschließende optische Identifizierung der Proteinkonzentration mit entsprechender Auswertesoftware im Gel bestimmt werden. Eine gebräuchliche Methode zur Präparation der Proteingele bei coryneformen Bakterien und zur Identifizierung der Proteine ist die von Hermann et al. (Electrophoresis, 22:1712-23 (2001)) beschriebene Vorgehensweise. Die Proteinkonzentration kann ebenfalls durch Western-Blot-Hybridisierung mit einem für das nachzuweisende Protein spezifischen Antikörper (Sambrook et al., Molecular cloning: a laboratory manual. 2nd Ed. Cold Spring Harbor Laboratory Press, Cold Spring Harbor, N.Y., 1989) und anschließender optischer Auswertung mit ensprechender Software zur Konzentrationsbestimmung (Lohaus und Meyer (1998) Biospektrum 5:32-39; Lottspeich, Angewandte Chemie 321: 2630-2647 (1999)) bestimmt werden. Die statistische Signifikanz der erhobenen Daten wird mittels eines T-Tests (Gosset ,Biometrika 6(1): 1-25 (1908)) ermittelt.

Die Maßnahme zur Überexpression gewünschter Gene unter Verwendung des erfindungsgemäßen Promotors kann in geeigneter Weise mit weiteren Maßnahmen zur Überexpression kombiniert werden.

Zur Erzielung einer Überexpression stehen im Stand der Technik eine Vielzahl von Methoden zur Verfügung. Hierzu gehört neben der Modifikation der Nukleotidsequenzen, die die Expression des Gens steuern bzw. kontrollieren auch die Erhöhung der Kopienzahl.

Die Erhöhung der Kopienzahl kann durch Plasmide erfolgen, die im Cytoplasma des Mikroorganismus replizieren. Hierzu sind im Stand der Technik eine Fülle von Plasmiden für die verschiedensten Gruppen von Mikroorganismen beschrieben, mit denen man die gewünschte Erhöhung der Kopienzahl des Gens einstellen kann. Geeignete Plasmide für die Gattung Corynebacterium sind beispielsweise bei Tauch et al. (Journal of Biotechnology 104 (1-3), 27-40, (2003)), oder bei Stansen et al. (Applied and Environmental Microbiology 71, 5920-5928 (2005)) beschrieben.

Die Erhöhung der Kopienzahl um mindestens eine (1) Kopie kann weiterhin durch Einfügen weiterer Kopien in das Chromosom des Mikroorganismus erfolgen. Geeignete Methoden für die Gattung Corynebacterium sind beispielsweise in der Patentschrift WO 03/014330, WO 03/040373 und WO 04/069996 beschrieben.

Die Erhöhung der Genexpression kann weiterhin dadurch erfolgen, dass man mehrere Promotoren vor das gewünschte Gen positioniert bzw. funktionell mit dem zu exprimierenden Gen verknüpft und auf diese Weise zu einer erhöhten Expression gelangt. Beispiele hierfür werden in der Patentschrift WO 2006/069711 beschrieben.

Die Transkription eines Gens wird gegebenenfalls durch Proteine, welche die Transkription unterdrücken (Repressorproteine) oder fördern (Aktivatorproteine) kontrolliert. Zur Erzielung einer Überexpression ist es demnach ebenfalls möglich, die Expression von Aktivatorproteinen zu steigern oder die Expression von Repressorproteinen zu verringern oder auszuschalten oder auch die Bindestellen der Repressorproteine zu eliminieren.

Die Geschwindigkeit der Elongation wird durch die Kodon-Verwendung beeinflusst, durch den Gebrauch von Kodons für im Ausgangsstamm häufig vorkommenden t(transfer)-RNAs kann die Translation verstärkt werden. Desweiteren kann der Austausch eines Start-Kodons zu dem in vielen Mikroorganismen (77% in Escherichia coli) am häufigsten vorkommenden Kodon ATG die Translation erheblich verbessern, da auf RNA-Ebene das Kodon AUG zwei- bis dreimal effektiver ist als zum Beispiel die Kodons GUG und UUG (Khudyakov et al., FEBS Letters 232(2):369-71 (1988); Reddy et al., Proceedings of the National Academy of Sciences of the USA 82(17):5656-60 (1985)). Auch die Sequenzumgebung des Start-Kodons kann optimiert werden, da zusammenwirkende Effekte zwischen dem Start-Kodon und den flankierenden Bereichen beschrieben sind (Stenstrom et al., Gene 273(2):259-65 (2001); Hui et al., EMBO Journal 3(3):623-9 (1984) ).

Anleitungen zum Umgang mit DNA, Verdauung und Ligation von DNA, Transformation und Selektion von Transformanten findet man unter anderem in dem bekannten Handbuch von Sambrook et al. "Molecular Cloning: A Laboratory Manual, Second Edition (Cold Spring Harbor Laboratory Press, 1989).

Gegenstand der Erfindung sind auch Vektoren, die das erfindungsgemäße Polynukleotid enthalten.

Kirchner und Tauch (Journal of Biotechnology 104:287-299 (2003)) beschreiben eine Auswahl der in Corynebacterium glutamicum anzuwendenden Vektoren.

Die homologe Rekombination erlaubt unter Verwendung der erfindungsgemäßen Vektoren den Austausch von DNA-Abschnitten auf dem Chromosom gegen erfindungsgemäße Polynukleotide, welche durch den Vektor in die Zelle transportiert werden. Für die effiziente Rekombination zwischen dem ringförmigen DNA-Molekül des Vektors und der Ziel-DNA auf dem Chromosom wird der auszutauschende DNA-Bereich mit dem erfindungsgemäßen Polynukleotid an den Enden mit Nukleotidsequenzen homolog zum Zielort versehen, diese bestimmen den Ort der Integration des Vektors bzw. des Austauschs der DNA.

So kann das erfindungsgemäße Polynukleotid mit Promotoraktivität
1. gegen den nativen Promotor am nativen Genort des zweiten Polynukleotids im Chromosom ausgetauscht werden oder
2. mit dem zweiten Polynukleotid an dessen nativem Genort oder an einem weiteren Genort integriert werden.

Unter "Austausch des nativen Promotors am nativen Genort des zweiten Polynukleotids" versteht man den Sachverhalt, dass der natürlich vorkommende Promotor desjenigen Gens ausgetauscht wird, welches im allgemeinen natürlicherweise in einer Kopie an seinem Genort im entsprechenden Wildtyp oder entsprechenden Ausgangsorganismus in Form seiner Nukleotidsequenz vorkommt.

Unter "einem weiteren Genort" versteht man einen Genort, dessen Nukleotidsequenz verschieden ist von der Sequenz des zweiten Polynukleotids. Dieser weitere Genort, beziehungsweise die an dem weiteren Genort vorhandene Nukleotidsequenz liegt bevorzugt im Chromosom und ist im allgemeinen für das Wachstum und für die Produktion der gewünschten chemischen Verbindungen nicht essentiell. Weiterhin können intergenische Bereiche im Chromosom, das heißt Nukleotidsequenzen ohne kodierende Funktion verwendet werden.

Die Expression oder Überexpression wird bevorzugt in Mikroorganismen der Gattung Corynebacterium durchgeführt. Innerhalb der Gattung Corynebacterium werden Stämme bevorzugt, die auf folgenden Arten beruhen: Corynebacterium efficiens, wobei der Typstamm als DSM44549 hinterlegt ist, Corynebacterium glutamicum, wobei der Typstamm als ATCC13032 hinterlegt ist, und Corynebacterium ammoniagenes, wobei der Typstamm als ATCC6871 hinterlegt ist. Die Art Corynebacterium glutamicum wird ganz besonders bevorzugt.

Einige Vertreter der Art Corynebacterium glutamicum sind im Stand der Technik auch unter anderen Bezeichnungen bekannt. Hierzu gehören beispielsweise: Stamm ATCC13870, der als Corynebacterium acetoacidophilum bezeichnet wurde, Stamm DSM20137, der als Corynebacterium lilium bezeichnet wurde, Stamm ATCC17965, der als Corynebacterium melassecola bezeichnet wurde, Stamm ATCC14067, der als Brevibacterium flavum bezeichnet wurde, Stamm ATCC13869, der als Brevibacterium lactofermentum bezeichnet wurde, und Stamm ATCC14020, der als Brevibacterium divaricatum bezeichnet wurde.

Der Begriff "Micrococcus glutamicus" für Corynebacterium glutamicum war ebenfalls gebräuchlich. Einige Vertreter der Art Corynebacterium efficiens wurden im Stand der Technik auch als Corynebacterium thermoaminogenes bezeichnet, wie zum Beispiel der Stamm FERM BP-1539.

Die für die erfindungsgemäßen Maßnahmen der Expression oder Überexpression eingesetzten Mikrorganismen bzw. Stämme (Ausgangsstämme) besitzen bevorzugt bereits die Fähigkeit eine gewünschte Feinchemikalie in das sie umgebende Nährmedium auszuscheiden und dort zu akkumulieren. Hierfür wird im Folgenden auch der Ausdruck "produzieren" verwendet. Insbesondere besitzen die für die Maßnahmen der Überexpression eingesetzten Stämme die Fähigkeit ≥ (mindestens) ≥ 0,10 g/l, 0,25 g/l, ≥ 0,5 g/l, ≥ 1,0 g/l, ≥ 1,5 g/l, ≥ 2,0 g/l, ≥ 4 g/l oder ≥ 10 g/l der gewünschten Feinchemikalie in ≤ (maximal) 120 Stunden, ≤ 96 Stunden, 48 Stunden, ≤ 36 Stunden, ≤ 24 Stunden oder ≤ 12 Stunden in der Zelle oder im Nährmedium anzureichern bzw. zu akkumulieren. Bei den Ausgangsstämmen handelt es sich bevorzugt um Stämme, die durch Mutagenese und Selektion, durch rekombinante DNA-Techniken oder durch eine Kombination beider Methoden hergestellt wurden.

Es ist für den Fachmann verständlich, dass man zu einem für die Maßnahmen der Erfindung geeignetem Mikroorganismus auch dadurch gelangen kann, indem man in einem Wildstamm, wie zum Beispiel in dem Corynebacterium glutamicum Typstamm ATCC 13032 oder in dem Stamm ATCC 14067, zunächst den erfindungsgemäßen Promotor gemäß SEQ ID NO:3 oder SEQ ID NO:34 zur Überexpression der gewünschten Gene einsetzt und anschließend durch weitere, im Stand der Technik beschriebene, genetische Maßnahmen, den Mikrorganismus veranlasst, die gewünschte(n) Feinchemikalien zu produzieren.

Unter dem Begriff "Feinchemikalien" versteht man für die Maßnahmen der Erfindung Aminosäuren, organische Säuren, Vitamine, Nukleoside und Nukleotide. Besonders bevorzugt werden proteinogene Aminosäuren, nicht proteinogene Aminosäuren, und organische Säuren.

Unter "proteinogenen Aminosäuren" versteht man die Aminosäuren die in natürlichen Proteinen, das heißt in Proteinen von Mikroorganismen, Pflanzen, Tieren und Menschen vorkommen. Sie dienen als Struktureinheiten für Proteine, in denen sie über Peptidbindungen miteinander verknüpft sind.

Die Begriffe Protein und Polypeptid sind gegenseitig austauschbar.

Werden im folgenden proteinogene L-Aminosäuren erwähnt, sind damit eine oder mehrere der Aminosäuren einschließlich ihrer Salze, ausgewählt aus der Gruppe L-Asparaginsäure, L-Asparagin, L-Threonin, L-Serin, L-Glutaminsäure, L-Glutamin, L-Glycin, L-Alanin, L-Cystein, L-Valin, L-Methionin, L-Isoleucin, L-Leucin, L-Tyrosin, L-Phenylalanin, L-Histidin, L-Lysin, L-Tryptophan, L-Arginin, L-Prolin und gegebenenfalls L-Selenocystein und L-Pyrrolysin gemeint. Besonders bevorzugt sind die L-Aminosäuren L-Lysin, L-Tryptophan, L-Methionin, L-Valin, L-Isoleucin und L-Prolin. Ganz besonders bevorzugt sind L-Lysin und L-Valin.

Wird im Folgenden L-Lysin oder Lysin erwähnt, sind damit nicht nur die Basen, sondern auch die Salze wie z.B. Lysin-Monohydrochlorid oder Lysin-Sulfat gemeint.

Zu den nicht proteinogenen Aminosäuren gehören unter anderem L-Ornithin und L-Homoserin.

Zu den organischen Säuren gehören unter anderem α-Ketosäuren, besonders bevorzugt sind die α-Ketoisokapronsäure (KIC), α-Ketovaleriansäure (KIV) und α-Keto-β-methylvaleriansäure (KMV).

L-Lysin ausscheidende bzw. produzierende Stämme der Art Corynebacterium glutamicum sind beispielsweise:
Corynebacterium glutamicum MH20-22B (= DSM16835) beschrieben in Menkel et al. (Applied and Environmental Microbiology 55(3), 684-688 (1989)) und hinterlegt als DSM16835,
Corynebacterium glutamicum DM1729 beschrieben bei Georgi et al. (Metabolic Engineering 7, 291-301 (2005)) und in EP 1 717 616 A2 und hinterlegt als DSM17576,
Corynebacterium glutamicum DSM13994 beschrieben in US 6,783,967, und
Corynebacterium glutamicum DM1933 beschrieben bei Blombach et al. (Appl Environ Microbiol. 2009 Jan;75(2):419-27).

Ein L-Lysin ausscheidender bzw. produzierender Stamm der Art Corynebacterium efficiens ist beispielsweise:
Corynebacterium thermoaminogenes AJ12521 (= FERM BP-3304) beschrieben in US 5,250,423.

L-Lysin produzierende Mikrorganismen besitzen typischerweise eine "feed back" resistente bzw. desensibilisierte Aspartatkinase. Unter "feed back" resistenten Aspartatkinasen versteht man Aspartatkinasen (LysC), die im Vergleich zur Wildform (Wildtyp) eine geringere Empfindlichkeit gegenüber der Hemmung durch Mischungen von Lysin und Threonin oder Mischungen von AEC (Aminoethylcystein) und Threonin oder Lysin allein oder AEC allein aufweisen. Die für diese im Vergleich zum Wildtyp desensibilisierten Aspartatkinasen kodierenden Gene bzw. Allele werden auch als lysC^{FBR}-Allele bezeichnet. Im Falle der Aspartatkinasen der Art Corynebacterium glutamicum ist der Stamm ATCC13032 der geeignete Wildtyp. Im Stand der Technik sind zahlreiche lysC^{FBR}-Allele beschrieben, die für Aspartatkinasevarianten kodieren, welche Aminosäureaustausche im Vergleich zum Wildtypprotein besitzen. Bei Bakterien der Gattung Corynebacterium wird das lysC-Gen auch als ask-Gen bezeichnet. Bei Enterobacteriaceae wird die vom lysC-Gen kodierte Aspartatkinase auch als Aspartokinase III bezeichnet.

Eine ausführliche Liste mit Angaben, welche Aminosäureaustausche im Aspatatkinaseprotein von Corynebacterium glutamicum zu einer Desensibilisierung führen, ist unter anderem in der WO2009141330A1 (Seite 10, Zeile 21 bis Seite 13, Zeile 17) enthalten, welche hier per Referenz miteinbezogen wird. Bevorzugt werden Aspartatkinasevarianten, welche folgende Aminosäureaustausche tragen, ausgewählt aus der Gruppe: an Position 380 der Aminosäuresequenz L-Isoleucin anstelle von L-Threonin und gegebenenfalls an Position 381 L-Phenylalanin anstelle L-Serin, an Position 311 L-Isoleucin anstelle L-Threonin und an Position 279 L-Threonin anstelle L-Alanin.

Ein L-Methionin ausscheidender bzw. produzierender Stamm der Art Corynebacterium glutamicum ist beispielsweise
Corynebacterium glutamicum M1179 (=DSM 17322) beschrieben in WO 2007/011939.

Bekannte Vertreter L- Tryptophan produzierender bzw. ausscheidender Stämme coryneformer Bakterien sind beispielsweise:
Corynebacterium glutamicum K76 (=Ferm BP-1847) beschrieben in US 5,563,052,
Corynebacterium glutamicum BPS13 (=Ferm BP-1777) beschrieben in US 5,605,818, und
Corynebacterium glutamicum Ferm BP-3055 beschrieben in US 5,235,940.

Bekannte Vertreter L-Valin produzierender bzw. ausscheidender Stämme coryneformer Bakterien sind beispielsweise:
Brevibacterium lactofermentum FERM BP-1763 beschrieben in US 5,188,948,
Brevibacterium lactofermentum FERM BP-3007 beschrieben in US 5,521,074,
Corynebacterium glutamicum FERM BP-3006 beschrieben in US 5,521,074, und
Corynebacterium glutamicum FERM BP-1764 beschrieben in US 5,188,948.

L-Valin produzierende Mikrorganismen besitzen typischerweise eine "feed back" resistente bzw. desensibilisierte Acetolactate Synthase (AHAS, EC 4.1.3.18), welche das erste Enzym der parallelen Stoffwechselwege zur Synthese von Isoleucin, Valin und Leucin (Umbarger, H. E. 1987. Biosynthesis of the branched-chain amino acids, p. 352-367. In F. C. Neidhardt, J. L. Ingraham, K. B. Low, B. Magasanik, M. Schaechter, and H.E. Umbarger (ed.), Escherichia coli and Salmonella typhimurium: Cellular and Molecular Biology. American Society for Microbiology, Washington, D.C.) ist. Das Holoenzym besteht immer aus 2 großen Untereinheiten und 2 kleinen Untereinheiten. Die große AHAS-Untereinheit bildet die katalytische Domäne und wird von ilvB kodiert, die kleine Untereinheit, welche als regulatorische Domäne fungiert, wird von ilvN kodiert. Unter "feed back" resistenten Acetolactate Synthasen versteht man Acetolactate Synthasen, die im Vergleich zur Wildform (Wildtyp) eine geringere Empfindlichkeit gegenüber der Hemmung durch die verzweigtkettigen Aminosäuren Valin, Leucin und Isoleucin bzw. Mischungen dieser aufweisen. Im Falle der Acetolactate Synthasen der Art Corynebacterium glutamicum ist der Stamm ATCC13032 der geeignete Wildtyp, unter der Art Brevibacterium flavum ist der Stamm ATCC14067 der geeignete Wildtyp.

Die in Corynebacterium glutamicum für Acetolactate Synthase kodierenden Gene ilvBN sind beispielsweise von Keilhauer et al. (Journal of Bacteriology 175(17):5595-603 (1993)) oder in der EP1108790 beschrieben. Die Zugangsnummer L09232 stellt die Sequenz der Gene dar.

Enzymvarianten der AHAS, die nicht mehr der Feedbackhemmung durch die verzweigtkettigen Aminosäuren (Leucin, Valin, Isoleucin) unterliegen, sind beispielsweise bei Mendel et al. (Journal of Molecular Biology 325, 275-284 (2003)), Elisakova et al. (Applied and Environmental Microbiology 71, 207-213 (2005)), Wada et al. (Bioscience Biotechnology & Biochemistry, 72 (11), 2959-2965, (2008)) und in der EP1491634 beschrieben. Bevorzugt werden Varianten einer "feed back" resistenten Acetolactate Synthase, welche einen oder mehrere der folgenden Aminosäureaustausche in der durch ilvN kodierten kleinen Untereinheit tragen, ausgewählt aus der Gruppe: an Position 20 der Aminosäuresequenz L-Aparaginsäure anstelle von Glycin, an Position 21 der Aminosäuresequenz L-Aparaginsäure anstelle von L-Isoleucin, an Position 22 der Aminosäuresequenz L-Phenylalanin anstelle von L-Isoleucin, an Position 42 jede proteinogene Aminosäure ausgenommen L-Alanin, bevorzugt L-Valin, L-Isoleucin und L-Leucin, besonders bevorzugt L-Valin und gegebenenfalls an Position 47 L-Leucin anstelle von L-Histidin.

Bekannte Vertreter L-Isoleucin produzierender bzw. ausscheidender Stämme coryneformer Bakterien sind beispielsweise:
Brevibacterium flavum FERM BP-760 beschrieben in US 4,656,135,
Brevibacterium flavum FERM BP-2215 beschrieben in US 5,294,547, und
Corynebacterium glutamicum FERM BP-758 beschrieben in US 4,656,135.

Bekannte Vertreter L-Homoserin produzierender bzw. ausscheidender Stämme coryneformer Bakterien sind beispielsweise:
Micrococcus glutamicus ATCC 14296 beschrieben in US 3,189,526 und
Micrococcus glutamicus ATCC 14297 beschrieben in US 3,189,526.

Bekannte Vertreter L-Ornithin ausscheidender bzw. produzierender Stämme der Art Corynebacterium glutamicum sind beispielsweise:
Brevibacterium lactofermentum FERM-BP 2344 beschrieben in US 5,188,947 und
Corynebacterium glutamicum FERM-BP 2345 beschrieben in US 5,188,947.

α-Ketosäure ausscheidende bzw. produzierende Stämme beruhen beispielsweise auf:
Corynebacterium glutamicum, Stamm ATCC13032,
Brevibacterium flavum, Stamm ATCC 14067 und
Brevibacterium lactofermentum, Stamm ATCC 13869.

Die vorliegende Erfindung stellt einen Mikroorganismus bereit, der eine Feinchemikalie produziert, wobei der Mikroorganismus durch die Verwendung des erfindungsgemäßen Promotors gemäß SEQ ID NO:3 oder SEQ ID NO:34 eine erhöhte Expression von einem oder mehreren Genen im Vergleich zu dem jeweiligen Ausgangsstamm aufweist.

Weiterhin stellt die vorliegende Erfindung ein Verfahren bereit zur fermentativen Herstellung einer Feinchemikalie enthaltend die Schritte:
a) Kultivieren des oben beschriebenen Mikroorganismus gemäß der vorliegenden Erfindung in einem geeignetem Medium, wobei eine Fermentationsbrühe erhalten wird, und
b)Anreichern der Feinchemikalie in der Fermentationsbrühe aus a) und/oder in den Zellen des Mikroorganismus.

Dabei ist bevorzugt, dass man aus der Feinchemikaliehaltigen Fermentationsbrühe die Feinchemikalie oder ein flüssiges oder festes Feinchemikalie-haltiges Produkt gewinnt.

Die hergestellten Mikroorganismen können kontinuierlich - wie beispielsweise in der WO 05/021772 beschrieben- oder diskontinuierlich im batch - Verfahren (Satzkultivierung bzw. Satzverfahren) oder im fed batch (Zulaufverfahren) oder repeated fed batch Verfahren (repetitives Zulaufverfahren) zum Zwecke der Produktion der gewünschten organisch chemischen Verbindung kultiviert werden. Eine Zusammenfassung allgemeiner Art über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Das zu verwendende Kulturmedium beziehungsweise Fermentationsmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Kulturmedium und Fermentationsmedium beziehungsweise Medium sind gegenseitig austauschbar.

Als Kohlenstoffquelle können Zucker und Kohlehydrate wie z.B. Glucose, Saccharose, Lactose, Fructose, Maltose, Melasse, Saccharose-haltige Lösungen aus der Zuckerrüben- oder Zuckerrohrverarbeitung, Stärke, Stärkehydrolysat und Cellulose, Öle und Fette, wie zum Beispiel Sojaöl, Sonnenblumenöl, Erdnußöl und Kokosfett, Fettsäuren, wie zum Beispiel Palmitinsäure, Stearinsäure und Linolsäure, Alkohole wie zum Beispiel Glycerin, Methanol und Ethanol und organische Säuren, wie zum Beispiel Essigsäure oder Milchsäure verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Das Kulturmedium muß weiterhin Salze beispielsweise in Form von Chloriden oder Sulfaten von Metallen wie beispielsweise Natrium, Kalium, Magnesium, Calcium und Eisen enthalten, wie zum Beispiel Magnesiumsulfat oder Eisensulfat, die für das Wachstum notwendig sind. Schließlich können essentielle Wuchsstoffe wie Aminosäuren beispielsweise Homoserin und Vitamine beispielsweise Thiamin, Biotin oder Pantothensäure zusätzlich zu den oben genannten Stoffen eingesetzt werden.

Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak beziehungsweise Ammoniakwasser oder saure Verbindungen wie Phosphorsäure oder Schwefelsäure in geeigneter Weise eingesetzt. Der pH wird im Allgemeinen auf einen Wert von 6,0 bis 8,5 vorzugsweise 6,5 bis 8 eingestellt. Zur Kontrolle der Schaumentwicklung können Antischaummittel, wie zum Beispiel Fettsäurepolyglykolester eingesetzt werden. Zur Aufrechterhaltung der Stabilität von Plasmiden können dem Medium geeignete, selektiv wirkende Stoffe wie zum Beispiel Antibiotika hinzugefügt werden. Die Fermentation wird bevorzugt unter aeroben Bedingungen durchgeführt. Um diese aufrechtzuerhalten, werden Sauerstoff oder Sauerstoff-haltige Gasmischungen, wie zum Beispiel Luft in die Kultur eingetragen. Die Verwendung von Flüssigkeiten, die mit Wasserstoffperoxid angereichert sind, ist ebenfalls möglich. Gegebenenfalls wird die Fermentation bei Überdruck, beispielsweise bei einem Überdruck von 0,03 bis 0,2 MPa, gefahren. Die Temperatur der Kultur liegt normalerweise bei 20°C bis 45°C und vorzugsweise bei 25°C bis 40°C, besonders bevorzugt bei 30° bis 37°C. Bei batch- oder fed-batch-Verfahren wird die Kultivierung bevorzugt solange fortgesetzt, bis sich eine für die Maßnahme der Gewinnung der gewünschten organisch chemischen Verbindung ausreichende Menge, gebildet hat. Dieses Ziel wird normalerweise innerhalb von 10 Stunden bis 160 Stunden erreicht. Bei kontinuierlichen Verfahren sind längere Kultivierungszeiten möglich. Durch die Tätigkeit der Mikroorganismen kommt es zu einer Anreicherung (Akkumulation) der organisch chemischen Verbindung im Fermentationsmedium und/oder in den Zellen der Mikroorganismen.

Beispiele für geeignete Fermentationsmedien finden sich unter anderem in den Patentschriften 5,770,409, US 5,990,350, US 5,275,940, WO 2007/012078, US 5,827,698, WO 2009/043803, US 5,756,345 oder US 7,138,266.

Die Analyse von L-Aminosäuren zur Bestimmung der Konzentration zu einem oder mehreren Zeitpunkt(en) im Verlauf der Fermentation kann durch Trennung der L-Aminosäuren mittels Ionenaustauschchromatographie vorzugsweise Kationenaustauschchromatographie mit anschließender Nachsäulenderivatisierung unter Verwendung von Ninhydrin erfolgen, so wie bei Spackman et al. (Analytical Chemistry 30: 1190-1206 (1958)) beschrieben. Anstelle von Ninhydrin kann auch ortho-Phtadialdehyd zur Nachsäulenderivatisierung eingesetzt werden. Einen Übersichtsartikel zur Ionenaustauschchromatographie findet man bei Pickering (LC·GC (Magazine of Chromatographic Science) 7(6), 484-487 (1989)).

Es ist ebenfalls möglich eine Vorsäulenderivatisierung beispielsweise unter Verwendung von ortho-Phtadialdehyd oder Phenylisothiocyanat vorzunehmen und die entstandenen Aminosäurederivate durch Reversed-Phase-Chromatographie (RP) vorzugsweise in Form der Hochleistungsflüssigkeitschromatographie (HPLC) aufzutrennen. Eine derartige Methode ist beispielsweise bei Lindroth et al. (Analytical Chemistry 51: 1167-1174 (1979)) beschrieben.

Die Detektion erfolgt photometrisch (Absorption, Fluoreszenz).

Eine zusammenfassende Darstellung zur Aminosäureanalyse findet man unter anderem im Lehrbuch "Bioanalytik" von Lottspeich und Zorbas (Spektrum Akademischer Verlag, Heidelberg, Deutschland 1998).

Die Analyse von α-Ketosäuren zur Bestimmung der Konzentration zu einem oder mehreren Zeitpunkt(en) im Verlauf der Fermentation kann durch Trennung der Ketosäuren und anderen Ausscheidungsprodukten mittels Ionenaustauschchromatographie, vorzugsweise Kationenaustauschchromatographie an einem sulfonierten Styrol-Divinylbenzol Polymeren in der H+ Form, z.B. mittels 0,025 N Schwefelsäure mit anschließender UV-Detektion bei 215nm (alternativ auch bei 230 oder 275 nm) erfolgen. Vorzugsweise kann eine REZEX RFQ - Fast Fruit H+ Säule (Phenomenex) eingesetzt werden, andere Lieferanten für die Trennphase (z.B. Aminex von BioRad) sind möglich. Analoge Trennungen sind in entsprechenden Applikationsbeispielen der Lieferanten beschrieben.

Die Leistung der Verfahren bzw. Fermentationsprozesse in denen die erfindungsgemäße Promotorvariante vorliegt bezüglich eines oder mehrerer der Parameter ausgewählt aus der Gruppe der Konzentration (gebildete Verbindung pro Volumen), der Ausbeute (gebildete Verbindung pro verbrauchter Kohlenstoff-Quelle), der Bildung (gebildete Verbindung pro Volumen und Zeit) und der spezifischen Bildung (gebildete Verbindung pro Zelltrockenmasse bzw. Biotrockenmasse und Zeit oder gebildete Verbindung pro Zellprotein und Zeit) oder auch anderer Prozess-Parameter und Kombinationen davon, wird erhöht um mindestens 0,5%, mindestens 1%, mindestens 1,5% oder mindestens 2% im Vergleich zu Verfahren bzw. Fermentationsprozessen mit Mikroorganismen, in denen die erfindungsgemäße Promotorvariante nicht vorliegt. Dies ist im Rahmen eines großtechnischen Prozesses als sehr wertvoll anzusehen.

Durch die Maßnahmen der Fermentation erhält man eine Fermentationsbrühe, welche die gewünschte Feinchemikalie, bevorzugt Aminosäure oder organische Säure, enthält.

Anschließend erfolgt die Bereitstellung bzw. Herstellung oder Gewinnung eines die Feinchemikalie enthaltenden Produktes in flüssiger oder fester Form.

Unter einer Fermentationsbrühe versteht man ein Fermentationsmedium bzw. Nährmedium, in dem ein Mikroorganismus für eine gewisse Zeit und bei einer gewissen Temperatur kultiviert wurde. Das Fermentationsmedium beziehungsweise die während der Fermentation eingesetzten Medien enthält/enthalten sämtliche Substanzen beziehungsweise Komponenten, die eine Produktion der gewünschten Verbindung und typischerweise die Vermehrung bzw. Lebensfähigkeit sicherstellen.

Bei Abschluss der Fermentation enthält die entstandene Fermentationsbrühe dementsprechend
a) die infolge der Vermehrung der Zellen des Mikroorganismus entstandene Biomasse (Zellmasse) des Mikroorganismus,
b) die im Laufe der Fermentation gebildete gewünschte Feinchemikalie,
c) die gegebenenfalls im Laufe der Fermentation gebildeten organischen Nebenprodukte, und
d) die durch die Fermentation nicht verbrauchten Bestandteile des eingesetzten Fermentationsmediums beziehungsweise der Einsatzstoffe wie beispielsweise Vitamine wie Biotin oder Salze wie Magnesiumsulfat.

Zu den organischen Nebenprodukten gehören Stoffe, die von den bei der Fermentation eingesetzten Mikroorganismen neben der jeweiligen gewünschten Verbindung erzeugt und gegebenenfalls ausgeschieden werden.

Die Fermentationsbrühe wird dem Kulturgefäß beziehungsweise dem Fermentationsbehälter entnommen, gegebenenfalls gesammelt, und dazu verwendet, ein die Feinchemikalie enthaltendes Produkt in flüssiger oder fester Form bereitzustellen. Hierfür wird auch der Ausdruck "Gewinnen des Feinchemikalie haltigen Produktes" verwendet. Im einfachsten Fall stellt die dem Fermentationsbehälter entnommene Feinchemikalie-haltige Fermentationsbrühe selbst das gewonnene Produkt dar.

Durch eine oder mehrere der Maßnahmen ausgewählt aus der Gruppe
a) teilweise (> 0% bis < 80%) bis vollständige (100%) oder nahezu vollständige (≥ 80%, ≥ 90%, ≥ 95%, ≥ 96%, ≥ 97%, ≥ 98%, ≥ 99%) Entfernung des Wassers,
b) teilweise (> 0% bis < 80%) bis vollständige (100%) oder nahezu vollständige (≥ 80%, ≥ 90%, ≥ 95%, ≥ 96%, ≥ 97%, ≥ 98%, ≥ 99%) Entfernung der Biomasse, wobei diese gegebenenfalls vor der Entfernung inaktiviert wird,
c) teilweise (> 0% bis < 80%) bis vollständige (100%) oder nahezu vollständige (≥ 80%, ≥ 90%, ≥ 95%, ≥ 96%, ≥ 97%, ≥ 98%, ≥ 99%, ≥ 99, 3%, ≥ 99,7%) Entfernung der im Laufe der Fermentation gebildeten organischen Nebenprodukte, und
d) teilweise (> 0%) bis vollständige (100%) oder nahezu vollständige (≥ 80%, ≥ 90%, ≥ 95%, ≥ 96%, ≥ 97%, ≥ 98%, ≥ 99%, ≥ 99,3%, ≥ 99,7%) Entfernung der durch die Fermentation nicht verbrauchten Bestandteile des eingesetzten Fermentationsmediums beziehungsweise der Einsatzstoffe,
aus der Fermentationsbrühe erzielt man eine Konzentrierung bzw. Reinigung der gewünschten organisch chemischen Verbindung. Auf diese Weise werden Produkte isoliert, die einen gewünschten Gehalt an der Verbindung aufweisen.

Die teilweise (> 0% bis < 80%) bis vollständige (100%) oder nahezu vollständige (≥ 80% bis < 100%) Entfernung des Wassers (Maßnahme a)) wird auch als Trocknung bezeichnet.

In einer Variante des Verfahrens gelangt man durch vollständige oder nahezu vollständige Entfernung des Wassers, der Biomasse, der organischen Nebenprodukte und der nicht verbrauchten Bestandteile des eingesetzten Fermentationsmediums zu reinen ((≥ 80 Gew.-%, ≥ 90 Gew.-%) oder hochreinen (≥ 95 Gew.-%, ≥ 97 Gew.-%, ≥ 99% Gew.-%) Produktformen der gewünschten organisch chemischen Verbindung, bevorzugt L-Aminosäuren. Für die Maßnahmen gemäß a), b), c) oder d) sind im Stand der Technik eine Fülle von technischen Anleitungen verfügbar.

Im Falle der Aminosäure L-Lysin sind im Stand der Technik im Wesentlichen vier verschiedene Produktformen bekannt.

Eine Gruppe L-Lysin-haltiger Produkte umfasst konzentrierte, wässrige, alkalische Lösungen von aufgereinigtem L-Lysin (EP-B-0534865). Eine weitere Gruppe, wie beispielsweise in der US 6,340,486 und US 6,465,025 beschrieben, umfasst wässrige, saure, Biomasse-haltige Konzentrate von L-Lysin-haltigen Fermentationsbrühen. Die bekannteste Gruppe fester Produkte umfasst pulverförmige oder kristalline Formen von aufgereinigtem beziehungsweise reinem L-Lysin, das typischerweise in Form eines Salzes wie zum Beispiel L-Lysin-Monohydrochlorid vorliegt. Eine weitere Gruppe fester Produktformen ist beispielsweise in der EP-B-0533039 beschrieben. Die dort beschriebene Produktform enthält neben L-Lysin den größten Teil der während der fermentativen Herstellung verwendeten und nicht verbrauchten Einsatzstoffe und gegebenfalls die Biomasse des eingesetzten Mikroorganismus mit einem Anteil von >0% - 100%.

Entsprechend der verschiedenen Produktformen kennt man verschiedenste Verfahren, bei denen aus der Fermentationsbrühe das L-Lysin-haltige Produkt oder das gereinigte L-Lysin hergestellt wird.

Zur Herstellung von festem, reinen L-Lysin werden im Wesentlichen Methoden der Ionenaustauschchromatographie gegebenfalls unter Verwendung von Aktivkohle und Methoden der Kristallisierung angewendet. Auf diese Weise erhält man die entsprechende Base oder ein entsprechendes Salz wie beispielsweise das Monohydrochlorid (Lys-HCl) oder das Lysinsulfat (Lys₂-H₂SO₄).

In der EP-B-0534865 ist ein Verfahren zur Herstellung wässriger, basischer L-Lysin-haltiger Lösungen aus Fermentationsbrühen beschrieben. Bei dem dort beschriebenen Verfahren wird die Biomasse aus der Fermentationsbrühe abgetrennt und verworfen. Mittels einer Base wie beispielsweise Natrium-, Kalium- oder Ammoniumhydroxid wird ein pH-Wert zwischen 9 bis 11 eingestellt. Die mineralischen Bestandteile (anorganischen Salze) werden nach Konzentrierung und Abkühlung durch Kristallisation aus der Brühe abgetrennt und entweder als Dünger verwendet oder verworfen.

Bei Verfahren zur Herstellung von Lysin unter Verwendung von Bakterien der Gattung Corynebacterium werden solche Verfahren bevorzugt, bei denen Produkte erhalten werden, die Bestandteile der Fermentationsbrühe enthalten. Diese werden insbesondere als Tierfuttermitteladditive verwendet.

Je nach Anforderung kann die Biomasse ganz oder teilweise durch Separationsmethoden wie z.B. der Zentrifugation, der Filtration, dem Dekantieren oder einer Kombination hieraus aus der Fermentationsbrühe entfernt oder vollständig in ihr belassen werden. Gegebenenfalls wird die Biomasse beziehungsweise die Biomasse enthaltene Fermentationsbrühe während eines geeigneten Verfahrensschrittes inaktiviert beispielsweise durch thermische Behandlung (Erhitzen) oder durch Säurezugabe.

In einer Verfahrensweise wird die Biomasse vollständig oder nahezu vollständig entfernt, sodass keine (0 %) oder höchstens 30%, höchstens 20%, höchstens 10%, höchstens 5%, höchstens 1% oder höchstens 0,1% Biomasse im hergestellten Produkt verbleibt. In einer weiteren Verfahrensweise wird die Biomasse nicht oder nur in geringfügigen Anteilen entfernt, sodass sämtliche (100%) oder mehr als 70%, 80%, 90%, 95%, 99% oder 99,9% Biomasse im hergestellten Produkt verbleibt. In einem erfindungsgemäßen Verfahren wird dementsprechend die Biomasse in Anteilen ≥ 0% bis ≤ 100% entfernt.

Schließlich kann die nach der Fermentation erhaltene Fermentationsbrühe vor oder nach der vollständigen oder teilweisen Entfernung der Biomasse mit einer anorganischen Säure wie beispielsweise Salzsäure, Schwefelsäure oder Phosphorsäure oder organischen Säure wie beispielsweise Propionsäure zur Verbesserung der Handhabungseigenschaften des Endprodukts auf einen sauren pH Wert eingestellt werden (GB 1,439,728 oder EP 1 331 220). Es ist gleichfalls möglich, die Fermentationsbrühe mit der vollständig enthaltenen Biomasse anzusäuern. Schließlich kann die Brühe auch durch Zusatz von Natriumbisulfit (NaHSO₃, GB 1,439,728) oder einem anderen Salz beispielsweise Ammonium-, Alkali- oder Erdalkalisalz der schwefligen Säure stabilisiert werden.

Bei der Abtrennung der Biomasse werden gegebenenfalls in der Fermentationsbrühe enthaltene organische oder anorganische Feststoffe teilweise oder ganz entfernt. Die in der Fermentationsbrühe gelösten organischen Nebenprodukte und die gelösten nicht verbrauchten Bestandteile des Fermentationsmediums (Einsatzstoffe) bleiben mindestens teilweise (> 0%), bevorzugt zu mindestens 25%, besonders bevorzugt zu mindestens 50% und ganz besonders bevorzugt zu mindestens 75% im Produkt. Gegebenenfalls bleiben diese auch vollständig (100%) oder nahezu vollständig, das heißt > 95% oder > 98% oder größer 99%, im Produkt. Enthält ein Produkt in diesem Sinn mindestens einen Teil der Bestandteile der Fermentationsbrühe, so wird dies auch mit dem Begriff "Produkt auf Fermentationsbrühebasis" umschrieben.

Anschließend wird der Brühe mit bekannten Methoden wie z.B. mit Hilfe eines Rotationsverdampfers, Dünnschichtverdampfers, Fallfilmverdampfers, durch Umkehrosmose oder durch Nanofiltration Wasser entzogen beziehungsweise eingedickt oder konzentriert. Diese aufkonzentrierte Fermentationsbrühe kann anschließend durch Methoden der Gefriertrocknung, der Sprühtrocknung, der Sprühgranulation oder durch anderweitige Verfahren wie zum Beispiel in der zirkulierenden Wirbelschicht gemäß PCT/EP2004/006655 beschrieben, zu rieselfähigen Produkten insbesondere zu einem feinteiligen Pulver oder vorzugsweise grobkörnigem Granulat aufgearbeitet werden. Gegebenenfalls wird aus dem erhaltenen Granulat durch Sieben oder Staubabtrennung ein gewünschtes Produkt isoliert.

Es ist ebenfalls möglich, die Fermentationsbrühe direkt, d. h. ohne vorherige Aufkonzentrierung durch Sprühtrocknung oder Sprühgranulation, zu trocknen.

Unter "rieselfähig" versteht man Pulver, die aus einer Serie von Glasauslaufgefäßen mit verschieden großen Auslauföffnungen mindestens aus dem Gefäß mit der Öffnung 5 mm (Millimeter) ungehindert auslaufen (Klein: Seifen, Öle, Fette, Wachse 94, 12 (1968)).

Mit "feinteilig" ist ein Pulver mit überwiegendem Anteil (> 50 %) einer Korngröße von 20 bis 200 µm Durchmesser gemeint.

Mit "grobkörnig" ist ein Produkt mit einem überwiegendem Anteil (> 50 %) einer Korngröße von 200 bis 2000 µm Durchmesser gemeint.

Die Korngrößenbestimmung kann mit Methoden der Laserbeugungsspektrometrie durchgeführt werden. Die entsprechenden Methoden sind im Lehrbuch zur "Teilchengrößenmessung in der Laborpraxis" von R. H. Müller und R. Schuhmann, Wissenschaftliche Verlagsgesellschaft Stuttgart (1996) oder im Lehrbuch "Introduction to Particle Technology" von M. Rhodes, Verlag Wiley & Sons (1998) beschrieben.

Das rieselfähige, feinteilige Pulver kann wiederum durch geeignete Kompaktier- oder Granulier-Verfahren in ein grobkörniges, gut rieselfähiges, lagerfähiges und weitgehend staubfreies Produkt überführt werden.

Der Begriff "staubfrei" bedeutet, daß das Produkt lediglich geringe Anteile (< 5 %) an Korngrößen unter 100 µm Durchmesser enthält.

"Lagerfähig", im Sinne dieser Erfindung, bedeutet ein Produkt, das mindestens ein (1) Jahr oder länger, bevorzugt mindestens 1,5 Jahre oder länger, besonders bevorzugt zwei (2) Jahre oder länger in trockener und kühler Umgebung gelagert werden kann, ohne dass ein wesentlicher Verlust der jeweiligen Aminosäure auftritt. Unter wesentlichem Verlust wird ein Verlust von >5% verstanden.

Ein weiterer Gegenstand der Erfindung ist ein Verfahren, das in seinen Grundzügen in der WO 2007/042363 A1 beschrieben ist. Hierfür wird unter Verwendung der erfindungsgemäß erhaltenen Fermentationsbrühe, aus der die Biomasse gegebenenfalls ganz oder teilweise abgetrennt wurde, ein Verfahren durchgeführt, das folgende Schritte umfasst:
a) den pH-Wert durch Zugabe von Schwefelsäure auf 4,0 bis 5,2, insbesondere 4,9 bis 5,1, absenkt, und ein molares Sulfat/L-Lysin-Verhältnis von 0,85 bis 1,2, bevorzugt 0,9 bis 1,0, besonders bevorzugt >0,9 bis <0,95, in der Brühe einstellt, gegebenenfalls durch Zugabe von einer weiteren oder mehreren Sulfat-haltigen Verbindung(en) und
b) das so erhaltene Gemisch durch Wasserentzug aufkonzentriert und gegebenenfalls granuliert,
wobei gegebenenfalls vor Schritt a) eine oder beide der folgenden Maßnahmen durchgeführt wird/werden:
c) Messung des molaren Verhältnisses von Sulfat/L-Lysin zur Ermittlung der benötigten Menge an Sulfat-haltigen Verbindung(n)
d) Zusatz einer Sulfat-haltigen Verbindung ausgewählt aus der Gruppe Ammoniumsulfat, Ammoniumhydrogensulfat und Schwefelsäure in entsprechenden Verhältnissen.

Gegebenenfalls wird weiterhin vor Schritt b) ein Salz der schwefligen Säure, bevorzugt Alkalihydrogensulfit, besonders bevorzugt Natriumhydrogensulfit in einer Konzentration von 0,01 bis 0,5 Gew.-%, bevorzugt 0,1 bis 0,3 Gew.-%, besonders bevorzugt 0,1 bis 0,2 Gew.-% bezogen auf die Fermentationsbrühe hinzugesetzt.

Als bevorzugte Sulfat-haltige Verbindungen im Sinne der oben genannten Verfahrensschritte sind insbesondere Ammoniumsulfat und/oder Ammoniumhydrogensulfat oder entsprechende Mischungen von Ammoniak und Schwefelsäure und Schwefelsäure selbst zu nennen.

Das molare Sulfat/L-Lysin-Verhältnis V wird nach der Formel: V = 2 x [SO₄²⁻] / [L-Lysin] berechnet. Diese Formel berücksichtigt die Tatsache, dass das SO₄²- Anion, bzw. die Schwefelsäure zweiwertig ist. Ein Verhältnis V = 1 bedeutet, dass ein stöchiometrisch zusammengesetztes LYS₂-H₂SO₄) vorliegt, während bei einem Verhältnis von V = 0,9 ein 10%iger Sulfatmangel und bei einem Verhältnis von V = 1,1 ein 10% Sulfatüberschuss gefunden wird.

Vorteilhaft bei der Granulation oder Kompaktierung ist der Einsatz von üblichen organischen oder anorganischen Hilfsstoffen, beziehungsweise Trägern wie Stärke, Gelatine, Cellulosederivaten oder ähnlichen Stoffen, wie sie üblicherweise in der Lebensmittel- oder Futterverarbeitung als Binde-, Gelier-, oder Verdickungsmittel Verwendung finden, oder von weiteren Stoffen wie zum Beispiel Kieselsäuren, Silikaten (EP0743016A) und Stearaten.

Weiterhin ist es vorteilhaft, die Oberfläche der erhaltenen Granulate mit Ölen oder Fetten zu behandeln so wie es in der WO 04/054381 beschrieben ist. Als Öle können Mineralöle, pflanzliche Öle oder Mischungen pflanzlicher Öle verwendet werden. Beispiele für derartige Öle sind Sojaöl, Olivenöl, Sojaöl/Lecithingemische. In gleicher Weise sind auch Silikonöle, Polyethylenglykole oder Hydroxyethylcellulose geeignet. Durch die Behandlung der Oberflächen mit den genannten Ölen erzielt man eine erhöhte Abriebfestigkeit des Produktes und einen Verringerung des Staubanteils. Der Gehalt an Öl im Produkt beträgt 0,02 bis 2,0 Gew.-%, bevorzugt 0,02 bis 1,0 Gew.-%, und ganz besonders bevorzugt 0,2 bis 1,0 Gew.-% bezogen auf die Gesamtmenge des Futtermitteladditivs.

Bevorzugt sind Produkte mit einem Anteil von ≥ 97 Gew.-% einer Korngröße von 100 bis 1800 µm oder einem Anteil von ≥ 95 Gew.-% einer Korngröße von 300 bis 1800 µm Durchmesser. Der Anteil an Staub, d. h. Partikeln mit einer Korngrösse < 100 µm, liegt bevorzugt bei > 0 bis 1 Gew.- besonders bevorzugt bei maximal 0,5 Gew.-%.

Alternativ kann das Produkt aber auch auf einen in der Futtermittelverarbeitung bekannten und üblichen organischen oder anorganischen Trägerstoff wie zum Beispiel Kieselsäuren, Silikate, Schrote, Kleien, Mehle, Stärken, Zucker oder andere aufgezogen und/oder mit üblichen Verdickungs- oder Bindemitteln vermischt und stabilisiert werden. Anwendungsbeispiele und Verfahren hierzu sind in der Literatur (Die Mühle + Mischfuttertechnik 132 (1995) 49, Seite 817) beschrieben.

Schließlich kann das Produkt auch durch Beschichtungsverfahren ("Coating") mit Filmbildnern wie beispielsweise Metallcarbonaten, Kieselsäuren, Silikaten, Alginaten, Stearaten, Stärken, Gummis und Celluloseether, wie in der DE-C-4100920 beschrieben, in einen Zustand gebracht werden, in dem es stabil gegenüber der Verdauung durch Tiermägen, insbesondere dem Magen von Wiederkäuern, ist.

Zur Einstellung einer gewünschten L-Lysin Konzentration im Produkt kann je nach Anforderung das L-Lysin während des Verfahrens in Form eines Konzentrates oder gegebenenfalls einer weitgehend reinen Substanz beziehungsweise dessen Salz in flüssiger oder fester Form hinzugefügt werden. Diese können einzeln oder als Mischungen zur erhaltenen oder aufkonzentrierten Fermentationsbrühe, oder auch während des Trocknungs- oder Granulationsprozesses, hinzugefügt werden.

Die Anmeldung offenbart eine Kombination mit einem Verfahren zur Herstellung eines festen Lysin-haltigen Produktes, das in seinen Grundzügen in der US 20050220933 beschrieben ist. Hierbei wird unter Verwendung der erfindungsgemäß erhaltenen Fermentationsbrühe ein Verfahren durchgeführt, das folgende Schritte umfasst:
a) Filtration der Fermentationsbrühe, bevorzugt mit einem Membranfilter, so dass ein Biomasse-haltiger Schlamm und ein Filtrat erhalten wird,
b) Aufkonzentration des Filtrates, bevorzugt so, dass ein Feststoffgehalt von 48 bis 52 Gew.-% erhalten wird,
c) Granulation des in Schritt b) erhaltenen Konzentrates, bevorzugt bei einer Temperatur von 50°C bis 62°C, und
d) Beschichtung des in c) erhaltenen Granulates mit einem oder mehreren der Beschichtungsmittel (coating agent(s))

Zur Beschichtung in Schritt d) werden bevorzugt Beschichtungsmittel verwendet, welche ausgewählt werden aus der Gruppe, bestehend aus
d1) der in Schritt a) erhaltenen Biomasse,
d2) einer L-Lysin-haltigen Verbindung, bevorzugt ausgewählt aus der Gruppe L-Lysinhydrochlorid oder L-Lysinsulfat,
d3) einem im wesentlichen L-Lysin-freien Stoff mit L-Lysingehalt < 1 Gew.-%, bevorzugt < 0,5 Gew.-%, bevorzugt ausgewählt aus der Gruppe Stärke, Karageenan, Agar, Kieselsäuren, Silikate, Schrote, Kleien und Mehle, und
d4) einem wasserabstoßenden Stoff, bevorzugt ausgewählt aus der Gruppe Öle, Polyethylenglykole und flüssige Paraffine.

Durch die Maßnahmen entsprechend den Schritten d1) bis d4), insbesondere d1) bis d3), wird der Gehalt an L-Lysin auf einen gewünschten Wert eingestellt.

Bei der Herstellung L-Lysin-haltiger Produkte wird das Verhältnis der Ionen bevorzugt so eingestellt, dass das molare Ionenverhältnis entsprechend nachstehender Formel 2x[SO₄²⁻] + [Cl⁻] - [NH₄⁺] - [Na⁺] - [K⁺] -2x [Mg²⁺] -2x [Ca²⁺] / [L-Lys]

0,68 bis 0,95, bevorzugt 0,68 bis 0,90, besonders bevorzugt 0,68 bis 0,86 ergibt, so wie von Kushiki et al. in der US 20030152633 beschrieben.

Im Falle des L-Lysins hat das auf diese Weise hergestellte feste Produkt auf Fermentationsbrühebasis einen Lysingehalt (als Lysinbase) von 10 Gew.-% bis 70 Gew.-% oder 20 Gew.-% bis 70 Gew.-%, bevorzugt 30 Gew.-% bis 70 Gew.-% und ganz besonders bevorzugt von 40 Gew.-% bis 70 Gew.-% bezogen auf die Trockenmasse des Produkts. Maximale Gehalte an Lysinbase von 71 Gew.-%, 72 Gew.-%, 73 Gew.-% sind ebenfalls möglich.

Der Wassergehalt des L-Lysin-haltigen, festen Produktes beträgt bis zu 5 Gew.-%, bevorzugt bis zu 4 Gew.-%, und besonders bevorzugt weniger als 3 Gew.-%.

### Beispiel 1

### Sequenzierung des Promotorbereiches des Gens gap aus DM1547

Der Corynebacterium glutamicum Stamm DM1547 wurde durch mehrfache, ungerichtete Mutagenese, Selektion und Mutantenauswahl aus C. glutamicum ATCC13032 hergestellt. Der Stamm ist resistent gegen das Lysin-Analogon S-(2-Aminoethyl)-L-Cystein.

Eine Reinkultur des Stammes DM1547 wurde bei der Deutschen Sammlung für Mikrorganismen und Zellkulturen (DSMZ, Braunschweig, Deutschland) am 16. Januar 2001 gemäß Budapester Vertrag als DSM 13994 hinterlegt. Der Stamm DSM 13994 ist in der EP 1 239 040 aufgeführt.

Die Nukleotidsequenz des Genoms von Corynebacterium glutamicum ATCC13032 ist bei Ikeda und Nakagawa (Applied Microbiology and Biotechnology 62, 99-109 (2003)), in der EP 1 108 790 und bei Kalinowski et al. (Journal of Biotechnolgy 104(1-3), (2003)) beschrieben. Die Nukleotidsequenz des Genoms von Corynebacterium glutamicum R ist bei Yukawa et al. (Microbiology 153(4):1042-1058 (2007)) beschrieben.

Die Nukleotidsequenz des Genoms von Corynebacterium efficiens ist bei Nishio et al (Genome Research. 13 (7), 1572-1579 (2003)) beschrieben.

Die Nukleotidsequenzen des Genoms von Corynebacterium glutamicum und Corynebacterium efficiens sind ebenfalls in der Datenbank des National Center for Biotechnology Information (NCBI) der National Library of Medicine (Bethesda, MD, USA), in der DNA Data Bank of Japan (DDBJ, Mishima, Japan) oder in der Nukleotidsequenz-Datenbank der European Molecular Biologies Laboratories (EMBL, Heidelberg, Deutschland bzw. Cambridge, UK) verfügbar.

Aus dem Stamm DM1547 wurde mit der Methode von Eikmanns et al. (Microbiology 140: 1817 - 1828 (1994)) chromosomale DNA isoliert. Mit Hilfe der Polymerase-Kettenreaktion wurde ein DNA-Abschnitt amplifiziert, welcher den stromaufwärts liegenden Bereich des gap-Gens enthält. Aufgrund der für C.glutamicum bekannten Sequenz des gap-Gens wurden folgende Oligonukleotide als Primer verwendet:
Pgap3-1 (SEQ ID NO:31):
   5' cgtttggggtcaatgtccat 3'
Pgap3-2 (SEQ ID NO:32):
   5' cccagtccaggttctttg 3'

Die dargestellten Primer wurden von der Firma MWG Biotech (Ebersberg, Deutschland) synthetisiert. Sie ermöglichen die Amplifizierung eines 529 bp langen DNA-Abschnittes. Der Primer Pgap3-2 bindet an die Region entsprechend Position 742 bis 759 des zu SEQ ID NO:30 (und SEQ ID NO:33) komplementären Stranges. Der Primer Pgap3-1 bindet an die Region entsprechend Position 231 bis 250 des Stranges gemäß SEQ ID NO:30 (und SEQ ID NO:33).

Die PCR-Reaktion wurde mit der Phusion High Fidelity DNA Polymerase (New England Biolabs, Frankfurt, Deutschland) durchgeführt. Der Reaktionsansatz wurde nach Herstellerangaben angesetzt und enthielt bei 50 µl GesamtVolumen 10 µl des mitgelieferten 5 x Phusion HF Buffer, Desoxynucleosidtriphosphate in einer Konzentration von jeweils 200 µM, Primer in einer Konzentration von 0,5 µM, ungefähr 50 ng Template-DNA und 2 Units Phusion Polymerase. Durch Zugabe von H₂O wurde das Volumen auf 50 µl eingestellt.

Der PCR-Ansatz wurde zuerst einer einleitenden Denaturierung bei 98°C für 30 Sekunden unterzogen. Darauf folgten sich 35x wiederholend ein Denaturierungsschritt bei 98°C für 20 Sekunden, ein Schritt zum Binden der Primer an die vorgelegte DNA bei 60°C für 20 Sekunden und der Extensionsschritt zur Verlängerung der Primer bei 72°C für 60 Sekunden. Nach dem abschliessenden Extensionsschritt für 5 Minuten bei 72°C wurde der PCR-Ansatz einer Agarosegel-Elektrophorese (0,8% Agarose) unterworfen. Ein DNA-Fragment von ca. 0,53 kb Länge wurde identifiziert, aus dem Gel isoliert und unter Verwendung des QIAquick Gel Extraction Kit der Firma Qiagen, (Hilden, Deutschland) aufgereinigt.

Die Nukleotidsequenz des amplifizierten DNA-Fragmentes bzw. PCR-Produktes wurde von der Firma Agowa (Berlin, Deutschland) bestimmt.

Die Nukleotidsequenz des Promotorbereichs des gap-Gens aus dem Stamm DM1547 enthält an Position 379 die Nukleobase Adenin (siehe SEQ ID NO:33). Der Promotorbereich des Wildtyps (siehe SEQ ID NO:30) enthält an dieser Position die Nukleobase Guanin. Diese Mutation wird im Folgenden als Pgap3 bezeichnet.

### Beispiel 2

### Konstruktion des Vektors pK18mobsacB-IBcg0054

Zur Charakterisierung der erfindungsgemäßen Mutation wird ein stromaufwärts liegender Bereich des gap-Gens, enthaltend den Promotor, zur Verstärkung eines synthethischen Operons verwendet. Das synthethische Operon besteht dabei aus einer desensibilisierten Aspartat-Kinase aus Corynebacterium glutamicum, kodiert durch ein Allel des Gens lysC, welches an Position 311 des kodierten Aspartatkinaseproteins anstelle der wildtypischen Nukleobase Threonin die Nukleobase Isoleucin besitzt (siehe WO 00/63388 und US 6,893,848), und einer

Aspartatsemialdehyd-Dehydrogenase aus Corynebacterium glutamicum, kodiert durch das Gen asd. Die Integration des synthetischen Operons erfolgt über homologe Rekombination in einen intergenischen Bereich im Chromosom von Corynebacterium glutamicum, so dass die Insertion in einer Erhöhung der Kopienzahl der Gene lysC und asd resultiert. Eine homologe DNA-Sequenz, die den Einbau des synthethischen Operons ermöglicht, wurde von der Firma Geneart AG (Regensburg, Deutschland) synthetisiert. Diese DNA-Sequenz ist in SEQ ID NO:35 dargestellt, und trägt die Bezeichnung IBcg0054. Die DNA-Sequenz enthält einen 383 bp umfassenden intergenischen Bereich flankiert von einem 595 bp Bereich (Gen cg0055 kodierend ein putatives Membranprotein und ein Teil des Gens cg0057 kodierend für eine Serin/Threonin-Proteinkinase) sowie einem 663 bp Bereich (Teil des Gens cg0054 kodierend ein Eisen-Aufnahme-Protein).

Zusätzlich zu den Restriktionsschnittstellen der Enzyme AvrII und NsiI zentral innerhalb des intergenischen Bereiches zur Insertion verschiedener DNA-Sequenzen wurden für die Restriktionsendonukleasen MunI und HindIII flankierende Schnittstellen für Umklonierungen des gesamten Fragmentes erstellt.

Das 1660 bp lange Geneart-Fragment (SEQ IDNO:35) wurde mit den Restriktionsenzymen MunI und HindIII verdaut und anschließend in den von Schäfer et al. (Gene, 145, 69-73 (1994)) beschriebenen, mobilisierbaren Vektor pK18mobsacB umkloniert. Hierzu wurde pK18mobsacB mit den Restriktionsenzymen EcoRI und HindIII verdaut. Der so vorbereitete Vektor wurde mit dem IBcg0054-Fragment gemischt und der Ansatz mit dem Ready-To-Go T4 DNA Ligase Kit (Amersham-Pharmacia, Freiburg, Deutschland) behandelt.

Anschließend wurde der E.coli Stamm S17-1 (Simon et al.,Bio/Technologie 1: 784-791, 1993) mit dem Ligationsansatz transformiert (Hanahan, In. DNA cloning. A practical approach. Vol.1. ILR-Press, Cold Spring Habor, New York, 1989). Die Selektion auf Plasmid-tragende Zellen erfolgte durch Ausplattieren des Tansformationsansatzes auf LB-Agar (Sambrock et al., Molecular Cloning: a laboratory manual. 2nd Ed. Cold Spring Habor, New York, 1989), der mit 50 mg/l Kanamycin supplementiert worden war.

Plasmid-DNA wurde aus einer Transformante mit Hilfe des QIAprep Spin Miniprep Kit der Firma Qiagen isoliert und durch Restriktionsspaltung mit den Enzymen EcoRI und HindIII, sowie anschließender Agarosegel-Elektrophorese überprüft. Das Plasmid trägt die Bezeichnung pK18mobsacB_IBcg0054.

### Beispiel 3

**Konstruktion der Austauschvektoren**
**pK18mobsacB_PgapWT_lysCT311I_asd,**
**pK18mobsacB_Pgap3_lysCT311I_asd und**
**pK18mobsacB_Pg3N3_lysCT311I_asd**

Die Charakterisierung der Mutation im stromaufwärts liegenden Bereich des gap-Gens erfolgt anhand einer DNA-Kassette, enthaltend ein synthetisches Operon der Gene lysC und asd unter Kontrolle der jeweiligen Promotoren PgapWT, Pgap3 bzw. Pg3N3, welches in den in Beispiel 2 beschriebenen intergenischen Bereich insertiert wird. Die synthetischen Operons wurden von der Firma Geneart AG (Regensburg, Deutschland) synthetisiert und tragen die Bezeichnungen PgapWT_lysCT311I_asd, Pgap3_lysCT311I_asd bzw. Pg3N3_lysCT311I_asd. Die DNA-Sequenzen sind in Seq ID No:36 für PgapWT_lysCT311I_asd, in Seq ID No:37 für Pgap3_lysCT311I_asd bzw. in Seq ID No:38 für Pg3N3_lysCT311I_asd dargestellt.

Der Promotorbereich des gap-Gens umfasst die Positionen 9 bis 469, wobei die Sequenz der Konstrukte in diesem Bereich jeweils den in Seq ID No:2 (für PgapWT), in Seq ID No:3 (für Pgap3) bzw. Seq ID No:34 (für Pg3N3) dargestellten Sequenzen entspricht. Stromabwärts schließt sich die kodierende Sequenz einer desensibilisierten Aspartat-Kinase aus Corynebacterium glutamicum, kodiert durch ein Allel des Gens lysC, welches an Position 311 des kodierten Aspartatkinaseproteins anstelle der wildtypischen Nukleobase Threonin die Nukleobase Isoleucin besitzt (siehe WO 00/63388 und US 6,893,848), an. Zusätzlich wurde die Nukleobase Guanin durch die Nukleobase Adenin innerhalb des Startcodons von lysC ausgetauscht. Daran schließt sich wiederum stromabwärts die kodierende Sequenz der Aspartatsemialdehyd-Dehydrogenase aus Corynebacterium glutamicum, kodiert das Gen asd, an. Stromaufwärts der beiden Gene lysC und asd befindet sich jeweils die Ribosomenbindestelle des gap-Gens (vor lysC in Form der Seq ID No:16, vor asd in Form der Seq ID No:26) sowie stromabwärts von asd der Terminator des gap-Gens.

Es wurden für Umklonierungen Restriktionsschnittstellen der Enzyme AvrII und NsiI das synthetische Operon flankierend erstellt.

Die Fragmente wurden mit den Restriktionsenzymen AvrII und NsiI gespalten und anschließend in den unter Beispiel 2 beschriebenen, mobilisierbaren Vektor pK18mobsacB_IBcg0054 umkloniert. Hierzu wurde pK18mobsacB_IBcg0054 mit den Restriktionsenzymen AvrII und NsiI verdaut. Der so vorbereitete Vektor wurde mit den PgapWT_lysCT311I_asd, Pgap3_lysCT311I_asd bzw. Pg3N3_lysCT311I_asd-Fragmenten gemischt und der Ansatz mit dem Ready-To-Go T4 DNA Ligase Kit (Amersham-Pharmacia, Freiburg, Deutschland) behandelt.

Anschließend wurde der E.coli Stamm S17-1 (Simon et al.,Bio/Technologie 1: 784-791, 1993) mit dem Ligationsansatz transformiert (Hanahan, In. DNA cloning. A practical approach. Vol.1. ILR-Press, Cold Spring Habor, New York, 1989). Die Selektion auf Plasmid-tragende Zellen erfolgte durch Ausplattieren des Tansformationsansatzes auf LB-Agar (Sambrock et al., Molecular Cloning: a laboratory manual. 2nd Ed. Cold Spring Habor, New York, 1989), der mit 50 mg/l Kanamycin supplementiert worden war.

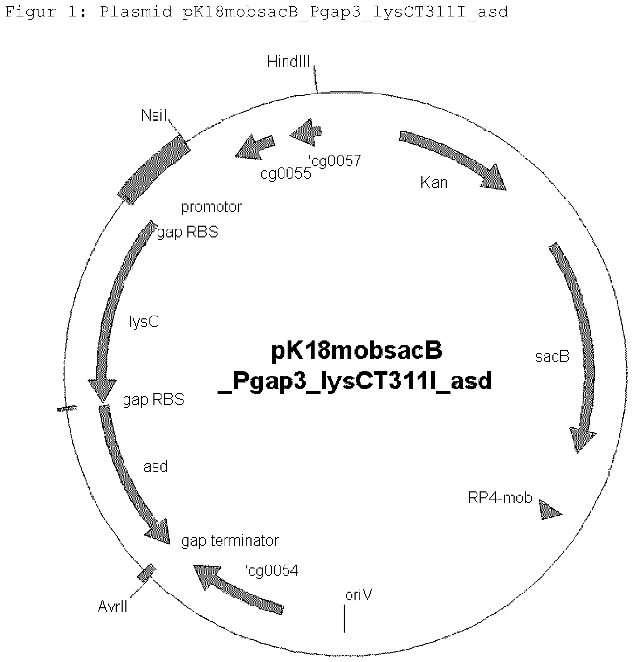

Plasmid-DNA wurde aus einer Transformante mit Hilfe des QIAprep Spin Miniprep Kit der Firma Qiagen isoliert und durch Restriktionsspaltung mit den Enzymen AvrII und NsiI, sowie anschließender Agarosegel-Elektrophorese überprüft. Die Plasmide tragen je nach gap-Promotor Allel die Bezeichnung pK18mobsacB_PgapWT_lysCT311I_asd, pK18mobsacB_Pgap3_lysCT311I_asd bzw. pK18mobsacB_Pg3N3_lysCT311I_asd. Beispielhaft ist pK18mobsacB_Pgap3_lysCT311I_asd in Figur 1 dargestellt.

### Beispiel 4

**Herstellung der C.glutamicum Stämme DM1729_**
**PgapWT_lysCT311I_asd, DM1729_ Pgap3_lysCT311I_asd und**
**DM1729_ Pg3N3_lysCT311I_asd**

Die Mutationen PgapWT_lysCT311I_asd, Pgap3_lysCT311I_asd bzw. Pg3N3_lysCT311I_asd sollen in den Stamm Corynebacterium glutamicum DM1729 eingebracht werden. Der Stamm DM1729 ist eine Aminoethylcystein-resistente Mutante von Corynebacterium glutamicum ATCC13032 und in der Patentanmeldung EP 1 717 616 A2 und durch Georgi et al. (Metabolic Engineering 7, 291-301 (2005)) beschrieben. Sie ist unter der Bezeichnung DSM17576 bei der Deutschen Sammlung für Mikroorganismen und Zellkulturen (DSMZ, Braunschweig, Deutschland) hinterlegt.

Die in Beispiel 3 beschriebenen Vektoren pK18mobsacB_PgapWT_lysCT311I_asd, pK18mobsacB_Pgap3_lysCT311I_asd bzw. pK18mobsacB_Pg3N3_lysCT311I_asd wurden nach dem Protokoll von Schäfer et al. (Journal of Microbiology 172: 1663-1666 (1990)) in den C. glutamicum Stamm DM1729 durch Konjugation transferiert. Der Vektor kann in DM1729 nicht selbständig replizieren und bleibt nur dann in der Zelle erhalten, wenn er als Folge eines Rekombinationsereignisses im Chromosom integriert vorliegt. Die Selektion von Transkonjuganten, d.h. von Klonen mit integriertem pK18mobsacB_PgapWT_lysCT311I_asd, pK18mobsacB_Pgap3_lysCT311I_asd bzw. pK18mobsacB_Pg3N3_lysCT311I_asd erfolgte durch Ausplattieren des Konjugationsansatzes auf LB-Agar, der mit 25 mg/l Kanamycin und 50 mg/l Nalidixinsäure supplementiert worden war. Kanamycin-resistente Transkonjuganten wurden anschließend auf mit Kanamycin (25 mg/1) supplementierten LB-Agarplatten ausgestrichen und für 24 Stunden bei 33°C inkubiert. Zur Selektion von Mutanten, bei denen als Folge eines zweiten Rekombinationsereignisses die Exzision des Plasmides stattgefunden hatte, wurden die Klone 30 Stunden unselektiv in LB-Flüssigmedium kultiviert, anschließend auf LB-Agar, der mit 10% Sucrose supplementiert worden war ausgestrichen und 24 Stunden bei 33°C bebrütet.

Die Plasmide pK18mobsacB_PgapWT_lysCT311I_asd, pK18mobsacB_Pgap3_lysCT311I_asd bzw. pK18mobsacB_Pg3N3_lysCT311I_asd enthalten ebenso wie das Ausgangsplasmid pK18mobsacB neben dem Kanamycin-Resistenzgen eine Kopie des für die Levan-Sucrase aus Bacillus subtilis kodierenden sacB-Gens. Die durch Sucrose induzierbare Expression des sacB-Gens führt zur Bildung der Levan-Sucrase, welche die Synthese des für C. glutamicum toxischen Produktes Levan katalysiert. Auf mit Sucrose supplementiertem LB-Agar wachsen daher nur solche Klone, bei denen das integrierte pK18mobsacB_PgapWT_lysCT311I_asd, pK18mobsacB_Pgap3_lysCT311I_asd bzw. pK18mobsacB_Pg3N3_lysCT311I_asd als Folge eines zweiten Rekombinationsereignisses exzisiert hat. In Abhängigkeit von der Lage des zweiten Rekombinationsereignisses in bezug auf den Mutationsort findet bei der Exzision der Allelaustausch bzw. der Einbau der Mutation statt oder es verbleibt die ursprüngliche Kopie im Chromosom des Wirtes.

Anschließend wurde jeweils ein Klon gesucht, bei dem der gewünschte Austausch, d. h. der Einbau der Kassette PgapWT_lysCT311I_asd, Pgap3_lysCT311I_asd bzw. Pg3N3_lysCT311I_asd in den intergenischen Bereich, erfolgt war.

Hierzu wurden jeweils 20 Klone mit dem Phänotyp "Wachstum in Gegenwart von Sucrose" und "Nicht-Wachstum in Gegenwart von Kanamycin" auf Integration der Kassette PgapWT_lysCT311I_asd, Pgap3_lysCT311I_asd bzw. Pg3N3_lysCT311I_asd unter Anwendung der Polymerase-Kettenreaktion (PCR) überprüft.

Hierfür wurden folgende synthetische Oligonukleotide (Primer) verwendet:
Primer cg0054_9.p (SEQ ID No.39): 5' CCACCCATCACCCTCACTTC 3'
Primer cg0054_10.p (SEQ ID No.40): 5' GCACTCTCGTTTGGCAGTTC 3'
Primer QlysC_WT_P2 (SEQ ID No.41): 5'AAGTGCCGGGATCATTACTA 3'

Die dargestellten Primer wurden von der Firma MWG Biotech (Ebersberg, Deutschland) synthetisiert. Die Primer cg0054_9.p und cg0054_10.p ermöglichen die Amplifikation eines 1032 bp großen DNA-Fragmentes bei vorliegen der wildtypischen Anordnung im intergenischen Bereich. Durch die Verwendung eines weiteren Primers (QlysC_WT_P2) in demselben Reaktionsansatz, der sich an das Gen lysC anlagern kann, wird die Amplifikation eines 1330 bp großen DNA-Fragmentes ermöglicht, wodurch die Integration des synthetischen Operons PgapWT_lysCT311I_asd, Pgap3_lysCT311I_asd bzw. Pg3N3_lysCT311I_asd in den intergenischen Bereich IBcg0054 spezifisch nachgewiesen wird. Die Kombination der Primer cg0054_9.p und cg0054_10.p ergibt im Falle der Integration in den intergenischen Bereich unter den gewählten Bedingungen für die PCR-Reaktion kein Amplifikat. Mit Hilfe der beschriebenen Nachweisreaktionen, werden außerdem PCR-Reaktionen identifiziert, welche aus technischen Gründen kein Amplifikat ergeben haben.

Die PCR-Reaktionen wurde mit dem Taq PCR Core Kit von Quiagen (Hilden, Deutschland), enthaltend die Taq DNA Polymerase aus *Thermus aquaticus,* in einem Mastercycler der Firma Eppendorf (Hamburg, Deutschland)durchgeführt. Die Bedingungen im Reaktionsansatz wurden nach Angaben des Herstellers eingestellt. Der PCR-Ansatz wurde zuerst einer einleitenden Denaturierung bei 94°C für 2 Minuten unterzogen. Darauf folgten sich 35x wiederholend ein Denaturierungsschritt bei 94°C für 30 Sekunden, ein Schritt zum Binden der Primer an die DNA bei 57°C für 30 Sekunden und der Extensions-Schritt zur Verlängerung der Primer bei 72°C für 60 sec. Nach dem abschliessenden Extensions-Schritt für 5 min bei 72°C wurden die so amplifizierten Produkte in einem Agarosegel elektrophoretisch geprüft.

Auf diese Weise wurden Mutanten identifiziert, welche die Kassette PgapWT_lysCT311I_asd, Pgap3_lysCT311I_asd bzw. Pg3N3_lysCT311I_asd in Form einer Integration vorliegen haben, wobei jeweils einer der erhaltenen Stämme C. glutamicum DM1729_ PgapWT_lysCT311I_asd, DM1729_ Pgap3_lysCT311I_asd und DM1729_ Pg3N3_lysCT311I_asd genannt wurde.

### Beispiel 5

### Herstellung von L-Lysin

Die in Beispiel 4 erhaltenen *C*. *glutamicum* Stämme DM1729_ PgapWT_lysCT311I_asd, DM1729_ Pgap3_lysCT311I_asd und DM1729_ Pg3N3_lysCT311I_asd und der Ausgangsstamm DM1729 wurden in einem zur Produktion von Lysin geeigneten Nährmedium kultiviert und der Lysingehalt im Kulturüberstand bestimmt.

Dazu wurden die Klone zunächst auf Hirn-Herz-Agarplatten (Merck, Darmstadt, Deutschland) für 24 Stunden bei 33°C vermehrt. Ausgehend von diesen Agarplattenkulturen wurde je eine Vorkultur angeimpft (10 ml Medium im 100 ml Erlenmeyerkolben). Als Medium für die Vorkultur wurde das Medium MM verwendet. Die Vorkultur wurde 24 Stunden bei 33°C und 240 rpm auf einem Schüttler inkubiert. Von dieser Vorkultur wurde eine Hauptkultur angeimpft, so dass die Anfangs-OD (660 nm) der Hauptkultur 0,1 OD betrug. Für die Hauptkultur wurde ebenfalls das Medium MM verwendet.

| Medium MM | |
|---|---|
| CSL | 5 g/l |
| MOPS | 20 g/l |
| Glucose (getrennt autoklaviert) | 50 g/l |
| Salze: | |
| (NH₄)₂SO₄) | 25 g/l |
| KH₂PO₄ | 0,1 g/l |
| MgSO₄ * 7 H₂O | 1,0 g/l |
| CaCl₂ * 2 H₂O | 10 mg/l |
| FeSO₄ * 7 H₂O | 10 mg/l |
| MnSO₄ * H₂O | 5,0mg/l |
| Biotin (sterilfiltriert) | 0,3 mg/l |
| Thiamin * HCl (sterilfiltriert) | 0,2 mg/l |
| CaCO₃ | 25 g/l |

CSL (Corn Steep Liquor), MOPS (Morpholinopropansulfonsäure) und die Salzlösung wurden mit Ammoniakwasser auf pH 7 eingestellt und autoklaviert. Anschließend wurden die sterilen Substrat- und Vitaminlösungen sowie das trocken autoklavierte CaCO₃ zugesetzt.

Die Kultivierung erfolgte in Volumina von 10 ml, die in 100 ml Erlenmeyerkolben mit Schikanen enthalten waren. Die Temperatur betrug bei 33°C, die Umdrehungszahl war 250 rpm und die Luftfeuchte 80%.

Nach 24 Stunden wurde die optische Dichte (OD) bei einer Meßwellenlänge von 660 nm mit dem Biomek 1000 (Beckmann Instruments GmbH, München) bestimmt. Die gebildete Lysinmenge wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt.

In Tabelle 1 ist das Ergebnis des Versuchs dargestellt.

**Tabelle 1: Herstellung von L-Lysin**

| Stamm | L-Lysin HCl (g/l) | OD (660nm) |
|---|---|---|
| DM1729 (Ausgangsstamm) | 9,9 | 14,9 |
| DM1729_ PgapWT_lysCT311I_asd | 12,5 | 13,9 |
| DM1729_ Pgap3_lysCT311I_asd | 12,8 | 13,2 |
| DM1729_ Pg3N3_lysCT311I_asd | 13,2 | 12,9 |

Alle Werte sind Mittelwerte von 3 unabhängigen Experimenten mit den genannten Stämmen

Das Ergebnis zeigt den positiven Effekt auf die Bildung des gewünschten Produktes (L-Lysin) durch Verwendung der erfindungsgemäßen Promotor-Varianten Pgap3 und Pg3N3 in Bezug auf die vorhergenannten Ausgangsstämme.

### Beispiel 6

**Konstruktion des Austauschvektors**
**pK18mobsacB_IBcg0054_Pg3_argJB**

Ausgehend von der Genomsequenz von Corynebacterium glutamicum ATCC13032 wurde ein 2722 bp großes DNA-Fragment bei der Firma GeneArt (Regensburg, Deutschland) synthetisiert (Seq ID: 29) welches den Pgap3 Promotor und die Gene argJ (kodierend für eine Glutamat-N-acetyltransferase) und argB (kodierend für eine Acetylglutamatkinase) trägt. Der Promotorbereich des gap-Gens umfasst die Positionen 10 bis 470, wobei die Sequenz des Konstruktes in diesem Bereich der in Seq ID No:3 (für Pgap3) dargestellten Sequenz entspricht. Stromabwärts schließen sich die kodierenden Sequenzen für eine Glutamat-N-acetyltransferase und eine Acetylglutamatkinase aus Corynebacterium glutamicum an. Das Fragment wurde über die terminal eingeführten Schnittstellen SphI und BlnI durch SphI- und BlnI-Spaltung geschnitten und anschließend in den ebenfalls SphI-/BlnI-geschnittenen Vektor pK18mobsacB_IBcg0054 (aus Beispiel 2) kloniert. Das Plasmid trägt die Bezeichnung pK18mobsacB_IBcg0054_Pg3_argJB.

### Beispiel 7

**Herstellung des C. glutamicum Stammes Corynebacterium**
**glutamicum ATCC13032-DargFRGH-Pg3-argJB**

Der in Beispiel 6 genannte Vektor pK18mobsacB_IBcg0054_Pg3_argJB wurde analog zu Beispiel 4 mittels Konjugation nach einem Protokoll von Schäfer et al. (Journal of Microbiology 172: 1663-1666 (1990)) in den Corynebacterium glutamicum Stamm Corynebacterium glutamicum ATCC13032_DargFRGH (aus Patentanmeldung DE102010003419.3) transferiert.

Dazu wurde der Vektor zuvor in Stamm E.coli S17-1 transformiert (Simon et al., Biotechnolgy 1:784-791). Der Vektor pK18mobsacB bzw. pK18mobsacB_IBcg0054_Pg3_argJB kann in C.glutamicum ATCC13032 nicht selbständig replizieren und bleibt nur dann in der Zelle erhalten, wenn er als Folge eines Rekombinationsereignisses ins Chromosom integriert hat. Die Selektion von Klonen mit integriertem pK18mobsacB_IBcg0054_Pg3_argJB erfolgt durch Ausplattieren des Konjugationsansatzes auf LB-Agar (Sambrook et al.,Molecular Cloning: A Laboratory Manual, 2nd Ed., Cold Spring Habor, New York, 1989), der mit 15 mg/l Kanamycin und 50 mg/ml Nalidixinsäure supplementiert worden ist. 15 Angewachsene Klone werden auf LB-Agarplatten mit 25 mg/l Kanamycin ausgestrichen und für 16 Stunden bei 33°C inkubiert. Zur Selektion von Mutanten, bei denen als Folge eines zweiten Rekombinationsereignisses die Exzision des Plasmides stattgefunden hat, werden die Klone 20 Stunden 20 unselektiv in LB-Flüssigmedium kultiviert, anschließend auf LB-Agar mit 10% Sucrose ausgestrichen und 24 Stunden bebrütet.Das Plasmid pK18mobsacB_IBcg0054_Pg3_argJB enthält ebenso wie das Ausgangsplasmid pK18mobsacB neben dem Kanamycin Resistenzgen eine Kopie des für die Levan-Sucrase aus Bacillus subtilis kodierenden sacB-Gens. Die durch Sucrose induzierbare Expression führt zur Bildung der Levan-Sucrase, die die Synthese des für C. glutamicum toxischen Produktes Levan katalysiert. Auf LB-Agar mit Sucrose wachsen daher nur solche Klone an, bei denen das integrierte pK18mobsacB_IBcg0054_Pg3_argJB wiederum exzisiert hat. Bei der Exzision kann zusammen mit dem Plasmid entweder die vollständige Kassette IBcg0054_Pg3_argJB exzisieren, oder der chromosomale intergenische Bereich IBcg0054. Ungefähr 40 bis 50 Kolonien wurden auf den Phänotyp "Wachstum in Gegenwart von Sucrose" und "Nicht-Wachstum in Gegenwart von Kanamycin" geprüft. Um nachzuweisen, dass die Kassette IBcg0054_Pg3_argJB im Chromosom verblieben ist, wurden ungefähr 20 Kolonien, die den Phänotyp 5 "Wachstum in Gegenwart von Sucrose" und "Nicht-Wachstum in Gegenwart von Kanamycin" aufweisen, nach der Standard-PCR-Methode von Innis et al. (PCR Protocols. A Guide to Methods and Applications, 1990, Academic Press) mit Hilfe der Polymerase-Kettenreaktion untersucht. Hierbei wurde aus der chromosomalen DNA der Kolonien ein DNA-Fragment amplifiziert, welches den umgebenden Bereich des intergenischen Bereiches IBcg0054 und einen Teil des inserierten arg-Gens trägt. Es wurden die folgenden Primer-Oligonukleotide für die PCR ausgewählt.
argA-E1: TGTCGCGGAAGTGCTGCAAC
cg0054_10.p: GCACTCTCGTTTGGCAGTTC

Der Nachweis der richtigen Klone erfolgte durch Erzeugung eines 1949 bp DNA-Fragmentes, welches im Agarosegel nachgewiesen wurde.

### Beispiel 8

### Herstellung von L-Ornithin mit Corynebacterium glutamicum

Zur Untersuchung ihrer Fähigkeit L-Ornithin zu produzieren wurden jeweils drei Klone des Stammes C. glutamicum ATCC13032_DargFRGH_Pg3_argJB und drei Klone des Stammes ATCC 13032_Delta_argFRGH in jeweils 10 ml Testmedium für 16 h bei 33°C vorkultiviert. Für den Produktionstest wurden mit der erhaltenen Vorkultur je 10 ml Testmedium so angeimpft, dass die Start-OD₆₀₀ (optische Dichte bei 600 nm) 0,1 betrug. Jeder Klon wurde in drei Schüttelkolben geprüft, sodass jeder Stamm durch insgesamt neun Schüttelkolben repräsentiert ist.

Das Testmedium war identisch mit dem bei Keilhauer et al. (Journal of Bacteriology (1993) 175: 5593-5603) beschriebenen CgXII-Medium, enthielt aber zusätzlich 7,5 g/l Hefeextract (Difco), 25 µg/ml Kanamycin, 1 mM IPTG (isopropyl-beta-D-thiogalactopyranoside) und 40 g/l Saccharose anstelle Glucose. Der Einfachheit halber ist die Zusammensetzung des Testmediums in der nachfolgenden Tabelle 2 zusammengefasst.

**Tabelle 2**

| **Komponente** | **Gehalt pro l** |
|---|---|
| (NH₄)₂SO₄ | 20 g |
| Harnstoff | 5 g |
| KH₂PO₄ | 1 g |
| K₂HPO₄ | 1 g |
| MgSO₄ x 7 H₂O | 0,25 g |
| 3-Morpholinopropansulfonsäure (MOPS) | 42 g |
| CaCl₂ | 0,01 g |
| FeSO₄ x 7H₂O | 0,01 g |
| MnSO₄ x H₂O | 0,01 g |
| ZnSO₄ x 7 H₂O | 0,001 g |
| CuSO₄ | 0,0002 g |
| NiCl₂ x 6H₂O | 0,00002 g |
| Biotin | 0,0002 g |
| Protokatechusäure | 0,03 g |
| Saccharose | 40 g |
| Hefeextrakt | 7,5 g |
| pH (mit NaOH) | 7 |

Die Kultivierung erfolgte bei 33°C und 200 rpm in 100 ml Schüttelkolben. Die Auslenkung des Schüttlers war 5 cm. Nach 24 und 48 Stunden wurden Proben aus den Kulturen entnommen und die optische Dichte der Gehalt an Saccharose und der Gehalt an L-Ornithin bestimmt und die Zellen kurz abzentrifugiert (Tischzentrifuge Typ 5415D (Eppendorf) bei 13000 rpm, 10 min, Raumtemperatur).

Die Bestimmung der optischen Dichte erfolgte bei einer Wellenlänge von 660 nm mit einem GENios Mikrotiterplatten Photometer (Tecan, Reading UK). Die Proben wurden vor der Messung 1:100 mit demineralisiertem Wasser verdünnt.

Die Bestimmung der Saccharose erfolgte mit einem Testsystem (Cat. Nr. 10 716 251 035) der Firma R-Biopharm AG (Darmstadt, Deutschland). Hierbei wird Saccharose invertiert und die gebildete Glukose mit einem gekoppeltem Enzymtest (Hexokinase/Glukose-6-Phosphat Dehydrogenase) via NADH-Bildung nachgewiesen.

Die quantitative Bestimmung der extrazellulären Aminosäurekonzentrationen aus dem Kulturüberstand erfolgte mittels reversed phase HPLC (Lindroth et al., Analytical chemistry (1979) 51: 1167-1174). Es wurde ein HPLC-Gerät der Serie HP1100 (Hewlett-Packard, Waldbronn, Deutschland) mit angeschlossenem Fluoreszenzdetektor (G1321A) verwendet; die Systemsteuerung und die Auswertung der Daten erfolgte mit einer HP-Chem-Station (Hewlett-Packard). 1 µL der zu analysierenden Aminosäurelösung wurde in einer automatischen Vorsäulenderivatisierung mit 20 µL ortho-Phthalaldehyd/2-Mercaptoethanol-Fertigreagenz (Pierce Europe BV, Oud-Beijerland, Niederlande) gemischt. Die dabei entstehenden fluoreszierenden, thiosubstituierten Isoindole (Jones et al., Journal of Chromatography (1983) 266: 471-482) wurden über eine kombinierte Vorsäule (40x4 mm Hypersil ODS 5) und Hauptsäule (Hypersil ODS 5, beide Säulen von der Firma CS-Chromatographie Service GmbH, Langerwehe, Deutschland) mit einem Gradientenprogramm mit zunehmend unpolarer Phase (Methanol) aufgetrennt. Das polare Eluenz war Natriumacetat (0,1 M; pH 7,2); die Flußrate betrug 0,8 mL pro Minute. Die Fluoreszenzdetektion der derivatisierten Aminosäuren erfolgte bei einer Anregungswellenlänge von 230 nm und einer Emissionswellenlänge von 450 nm. Die L-Ornithin bzw. die L-Ornithinhydrochlorid Konzentrationen wurden durch einen Vergleich mit einem externen Standard und L-Asparagin als zusätzlichen internen Standard berechnet.

Das Molekulargewicht von L-Ornithinhydrochlorid beträgt 168,6 g x mol⁻¹ und von L-Ornithin 132,1 g x mol⁻¹.

Zur Berechnung der Ausbeute wurde die Menge an gebildetem L-Ornithin (gemessen als L-Ornithinhydrochlorid) durch die Menge an verbrauchter Saccharose geteilt.

Die Ergebnisse sind in Tabelle 3 dargestellt.

Tabelle 3: L-Ornithin Bildung nach 24 Stunden (Tabelle 3A) und 48 Stunden (Tabelle 3B) Inkubation. Abkürzungen: Orn-HCl: L-Ornithinhydrochlorid.

**Tabelle 3A:**

| Zeit | 24 Stunden | | |
|---|---|---|---|
| Stamm | Orn-HCl g/l | Ausbeute g/g | OD |
| ATCC 13032 _Delta_argFRGH | 9,85 ± 0,10 | 0,39 ± 0,01 | 10,83 ± 0,25 |
| ATCC13032_Darg FRGH_Pg3_argJB | 10,53 ± 0,16 | 0,42 ± 0,01 | 10,50 ± 0,45 |

**Tabelle 3B:**

| Zeit | 48 Stunden | | |
|---|---|---|---|
| Stamm | Orn-HCl g/l | Ausbeute g/g | OD |
| ATCC 13032 _Delta_argFRGH | 15,10 ± 0,65 | 0,35 ± 0,01 | 11,59 ± 1,20 |
| ATCC13032_Darg FRGH_Pg3_argJB | 16,28 ± 0, 41 | 0,38 ± 0,01 | 10, 88 ± 0,43 |

### Beispiel 9

**Herstellung der Austauschkonstrukte**
**pK18mobsacB_homUP_Pg3_hom, pK18mobsacB_ilvAUP_Pg3_ilvA und**
**pK18mobsacB_pycUP_Pg3_pyc**

### Konstruktion des Austauschvektors pK18mobsacB_homUP_Pg3_hom

Ausgehend von der Genomsequenz von Corynebacterium glutamicum ATCC13032 wurde ein 2549 bp großes DNA-Fragment bei der Firma GeneArt (Regensburg, Deutschland) synthetisiert (SEQ ID No. 42) welches einen Teil der stromaufwärts des hom-Gens befindlichen Genregion, den Pgap3 Promotor und einen Teil des hom-Gens trägt. Das Fragment wurde über die terminal eingeführten Schnittstellen BamHI und XbaI durch BamHI- und XbaI-Spaltung geschnitten und anschließend in den ebenfalls BamHI-/XbaI-geschnittenen Vektor pK18mobsacB kloniert. Das Plasmid trägt die Bezeichnung pK18mobsacB_homUP_Pg3_hom.

**Konstruktion des Austauschvektors**
**pK18mobsacB-ilvAUP-Pg3_i1vA**

Analog wurde bei der Firma GeneArt ein 2442 bp großes DNA-Fragment synthetisiert (SEQ ID No. 43) welches einen Teil der stromaufwärts des ilvA-Gens befindlichen Genregion, den Pgap3 Promotor und einen Teil des ilvA-Gens trägt. Das Fragment wurde über die terminal eingeführten Schnittstellen BamHI und XbaI durch BamHI- und XbaI-Spaltung geschnitten und anschließend in den ebenfalls BamHI-/XbaI-geschnittenen Vektor pK18mobsacB kloniert. Das Plasmid trägt die Bezeichnung pK18mobsacB_ilvAUP_Pg3_ilvA.

### Konstruktion des Austauschvektors pK18mobsacB_pycUP_Pg3_pyc

Analog wurde bei der Firma GeneArt ein 2072 bp großes DNA-Fragment synthetisiert (SEQ ID No. 44) welches einen Teil der stromaufwärts des pyc-Gens befindlichen Genregion, den Pgap3 Promotor und einen Teil des pyc-Gens trägt. Das Fragment wurde über die terminal eingeführten Schnittstellen BamHI und HindIII durch BamHI- und HindIII-Spaltung geschnitten und anschließend in den ebenfalls BamHI-/HindIII-geschnittenen Vektor pK18mobsacB kloniert. Das Plasmid trägt die Bezeichnung pK18mobsacB_pycUP_Pg3_pyc.

### Beispiel 10

**Herstellung der C. glutamicum Stämme**
**ATCC14310_homUP_Pg3_hom, ATCC14310_ ilvAUP_Pg3_ilvA und**
**ATCC14310_pycUP_Pg3_pyc**

Die in Beispiel 9 genannten Vektoren **pK18mobsacB_homUP_Pg3_hom pK18mobsacB_ilvAUP_Pg3_ilvA und pK18mobsacB_pycUP_Pg3_pyc** wurden mittels Konjugation nach einem Protokoll von Schäfer et al. (Journal of Microbiology 172: 1663-1666 (1990)) jeweils einzeln durch Konjugation in den Corynebacterium glutamicum Stamm **C. glutamicum ATCC14310** (analog zu Beispiel 4) transferiert.

Es wurden die folgenden Primer-Oligonukleotide für die Nachweis-PCR der Stämme verwendet.

### Primer für den Nachweis von homUP Pg3 hom in ATCC14310:

Hom_Xl-A1 GATCTAGACGTCCAGGAGTTCCTCTACG (SEQ ID No. 45)
LC-hom2 TGGCGTCCAAGATGAAGTTG (SEQ ID No. 46)

Der Nachweis der richtigen Klone erfolgte durch Erzeugung eines 1502 bp DNA-Fragmentes, welches im Agarosegel nachgewiesen und anschließend durch Sequenzierung bestätigt wurde.

### Primer für den Nachweis von ilvAUP Pg3 ilvA in ATCC14310:

Pgap3_1.p CGAGTACTATGCATTGAAGCCTAAAAACGACCG (SEQ ID No. 47)
ilvA-int-rev2 ACCGCGGATCTTGTAGGAAC (SEQ ID No. 48)

Der Nachweis der richtigen Klone erfolgte durch Erzeugung eines 712 bp DNA-Fragmentes, welches im Agarosegel nachgewiesen und anschließend durch Sequenzierung bestätigt wurde.

### Primer für den Nachweis von pycUP Pg3 pyc in ATCC14310:

pycUP_3.p AGCACGTCAGCTGGTTCA (SEQ ID No. 49)
2xpyc-1 AGGTACGCCTTGACTGGTGA (SEQ ID No. 50)

Der Nachweis der richtigen Klone erfolgte durch Erzeugung eines 1004 bp DNA-Fragmentes, welches im Agarosegel nachgewiesen und anschließend durch Sequenzierung bestätigt wurde.

### Beispiel 11

### Herstellung von Isoleucin

Je 10 ml Caso-Bouillon mit 0.5 % Glucose wurden in einem Erlenmeyerkolben mit Schikanen (100 ml Volumen) mit 100 µl einer Bakterienkultur aus Beispiel 10 (ATCC 14310 Derivate) beimpft und als Vorkultur bei 33°C für 16 h bei 200 rpm geschüttelt (5 cm Amplitude). Aus der Vorkultur wurden 10 ml Hauptkultur in je drei 100 ml Erlenmeyerkolben mit Schikanen mit 1% Animpfvolumen inokuliert. Für die Hauptkultur diente ein Medium, welches 40 g Glukose, 20 g Ammoniumsulfat, 0,5 g Kaliumdihydrogenphosphat, 0,5 g Dikaliumhydrogenphosphat, 20 g Calciumcarbonat, 0,25 g Magnesiumsulfat (Heptahydrat), 10 mg Eisensulfat (Heptahydrat), 10 mg Mangansulfat (x 4 H2O), 1 mg Zinksulfat (Heptahydrat), 0,2 mg Kupfersulfat und 300 mg/l L-Leucin enthielt. Die Hauptkultur wurde 48 h bei 33°C und 200 rpm Schüttelgeschwindigkeit (5 cm Amplitude) inkubiert. Die erhaltene Kulturflüssigkeit wurde abzentrifugiert und die Isoleucinkonzentration anschließend im Überstand bestimmt. Die Ergebnisse sind in Tabelle 4 dargestellt.

**Tabelle 4: L-Isoleucin Bildung nach 48 Stunden Inkubation.**

| | **Isoleucinkonz. (g/l) nach 48 h** | | |
|---|---|---|---|
| **Stamm** | Kultur 1 | Kultur 2 | Kultur 3 |
| **ATCC14310** | 3,51 | 3,43 | 3,48 |
| **ATCC14310_homUP_Pg3_hom** | 3,91 | 3,95 | 3,97 |
| **ATCC14310_ilvAUP_Pg3_ilvA** | 4,1 | 4,02 | 4,05 |
| **ATCC14310_pycUP_Pg3_pyc** | 3,75 | 3,81 | 3,84 |

Figur 1: Karte des Plasmids pK18mobsacB_Pgap3_lysCT311I_asd

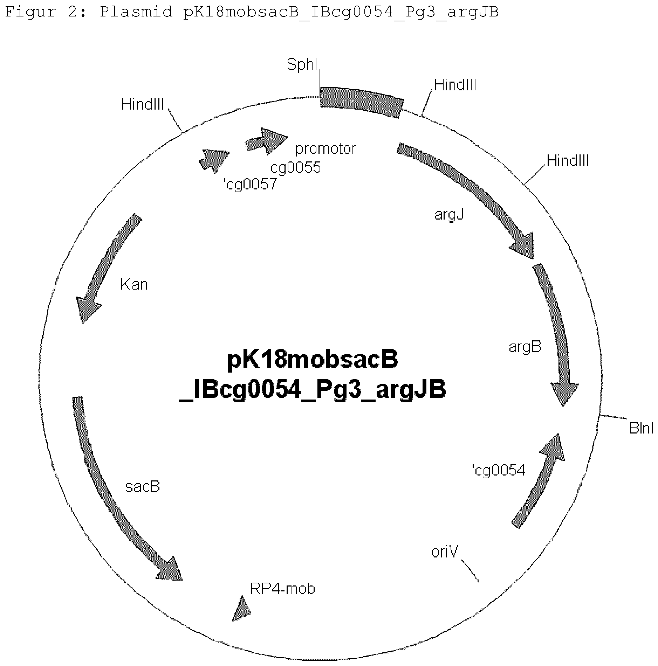

Figur 2: Karte des Plasmids pK18mobsacB_IBcg0054_Pg3_argJB

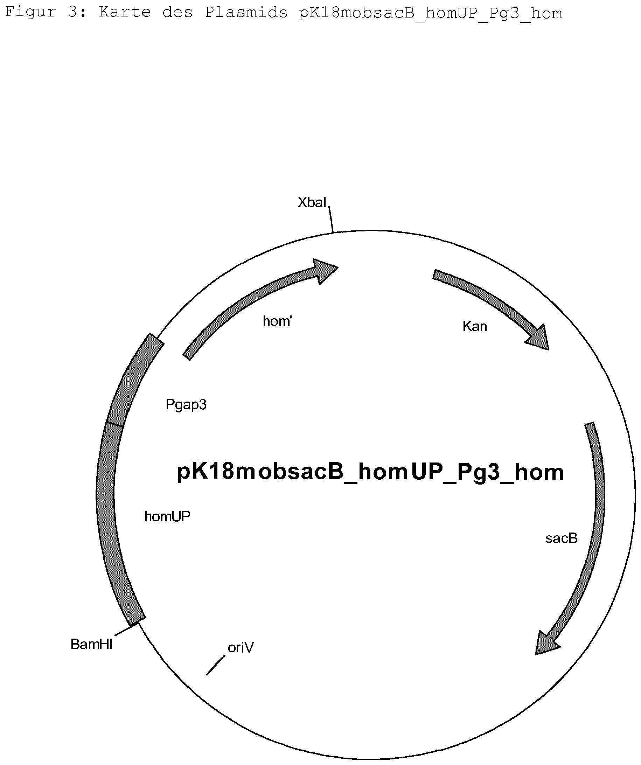

Figur 3: Karte des Plasmids pK18mobsacB_homUP_Pg3_hom

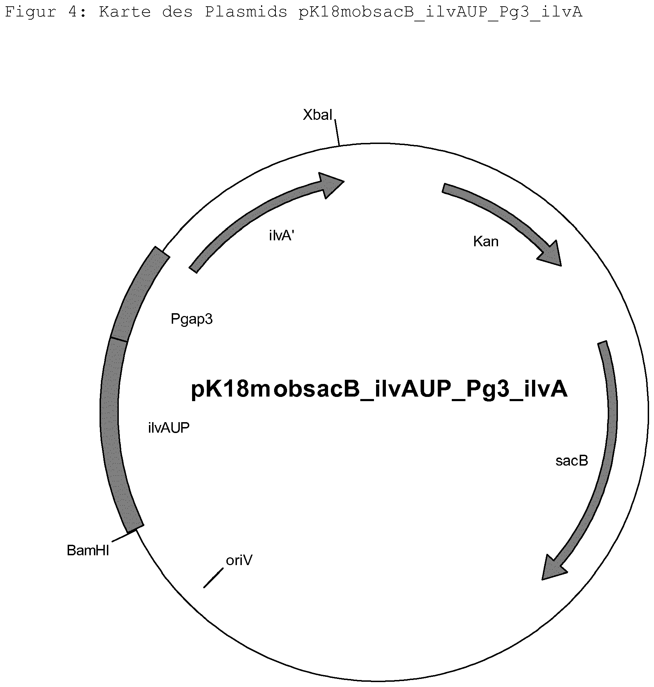

Figur 4: Karte des Plasmids pK18mobsacB_ilvAUP_Pg3_ilvA

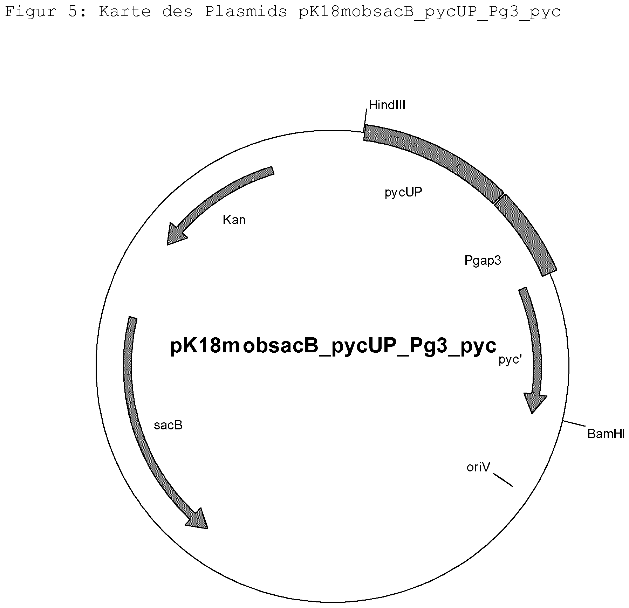

Figur 5: Karte des Plasmids pK18mobsacB_pycUP_Pg3_pyc

Die verwendeten Abkürzungen und Bezeichnungen haben folgende Bedeutung.
- oriV:: ColE1-ähnlicher Origin aus pMB1
- sacB: das für das Protein Levansucrose kodierende sacB-Gen
- RP4mob:: RP4-Mobilisierungs-Site
- Kan:: Resistenzgen für Kanamycin
- 'cg0057:: Teil des Gens cg0057 kodierend für eine Serin/Threonin-Proteinkinase
- cg0055:: Gen cg0055 kodierend ein putatives Membranprotein
- 'cg0054:: Teil des Gens cg0054 kodierend ein Eisen-Aufnahme-Protein
- gap Terminator:: Terminator des gap-Gens
- promotor:: Promotor des gap-Gens entsprechend SEQ ID Nr.3 (auch Pgap3)
- gap RBS:: Ribosomenbindestelle des gap-Gens
- lysC:: Allel des Gens lysC, kodierend für eine desensibilisierte Aspartat-Kinase
- asd:: Gen asd , kodierend für eine Aspartatsemialdehyd-Dehydrogenase
- argJ:: Gen argJ, kodierend für eine Glutamat-N-acetyltransferase
- argB:: Gen argB, kodierend für eine Acetylglutamatkinase
- homUP:: Teil der stromaufwärts des hom-Gens befindlichen Genregion
- hom' :: Teil des hom-Gens, kodierend für eine Homoserin-Dehydrogenase
- ilvAUP:: Teil der stromaufwärts des ilvA-Gens befindlichen Genregion
- ilva':: Teil des ilvA-Gens, kodierend für eine Threonin-Dehydratase
- pycUP:: Teil der stromaufwärts des pyc-Gens befindlichen Genregion
- pyc':: Teil des pyc-Gens, kodierend für eine Pyruvat Carboxylase
- HindIII:: Schnittstelle des Restriktionsenzyms HindIII
- BamHI:: Schnittstelle des Restriktionsenzyms BamHI
- XbaI:: Schnittstelle des Restriktionsenzyms XbaI
- AvrII:: Schnittstelle des Restriktionsenzyms AvrII
- NsiI:: Schnittstelle des Restriktionsenzyms NsiI
- SphI:: Schnittstelle des Restriktionsenzyms SphI
- BlnI:: Schnittstelle des Restriktionsenzyms BlnI

## Patentansprüche

1. Ein isoliertes Polynukleotid mit Promotoraktivität, welches ein Polynukleotid mit der Nukleotidsequenz dargestellt in SEQ ID NO:3 oder SEQ ID NO:34 umfasst.

2. Das Polynukleotid gemäß Anspruch 1 **dadurch gekennzeichnet, dass** es aus der Nukleotidsequenz dargestellt in SEQ ID NO:3 oder SEQ ID NO:34 besteht.

3. Das Polynukleotid gemäß Anspruch 2, **dadurch gekennzeichnet, dass** es am 3'-Ende mit einem zweiten Polynukleotid, welches am 5'-Ende ein ATG oder GTG Startkodon enthält und für ein oder mehrere Polypeptid(e) kodiert, direkt, mittels eines Brücken-Oligonukleotids oder mittels eines Brücken-Polynukleotids, bevorzugt mittels eines Brücken-Polynukleotids, funktionsfähig miteinander verbunden ist.

4. Das Polynukleotid gemäß Anspruch 3, **dadurch gekennzeichnet, dass** das Polynukleotid gemäß SEQ ID NO:3 oder SEQ ID NO:34 mit dem Adenosin-Nukleotid an Position 461 am 3'-Ende durch ein Brücken-Oligonukleotid mit einer Länge von 1, 2, 3, 4 oder 5 Nukleotiden mit dem ersten Nukleotid des Startkodons des zweiten Polynukleotids verbunden ist.

5. Das Polynukleotid gemäß Anspruch 3, **dadurch gekennzeichnet, dass** das Polynukleotid gemäß SEQ ID NO:3 oder SEQ ID NO:34 mit dem Adenosin-Nukleotid am 3'-Ende durch ein Brücken-Polynukleotid mit einer Länge von 6 bis maximal (≤) 600 Nukleotiden, vorzugsweise mit einer Länge von 20, 21, 22, 23, 24 oder 25 Nukleotiden, mit dem ersten Nukleotid des Startkodons des zweiten Polynukleotids verbunden ist.

6. Das Polynukleotid gemäß Anspruch 5, **dadurch gekennzeichnet, dass** das Brücken-Polynukleotid eine Nukleotidsequenz umfasst, die die Translation der synthetisierten RNA gewährleistet.

7. Das Polynukleotid gemäß den Ansprüchen 5 bis 6,
**dadurch gekennzeichnet, dass** die Sequenz des Brücken-Polynukleotids im Wesentlichen aus einer Nukleotidsequenz dargestellt in SEQ ID NO:12 oder SEQ ID NO:13 besteht.

8. Das Polynukleotid gemäß den Ansprüchen 5 bis 6,
**dadurch gekennzeichnet, dass** die Sequenz des Brücken-Polynukleotids aus einer der Nukleotidsequenzen dargestellt in SEQ ID NO:14, SEQ ID NO:15, SEQ ID NO:16, SEQ ID NO:17 oder SEQ ID NO:18 besteht.

9. Ein Polynukleotid gemäß Anspruch 2, **dadurch gekennzeichnet, dass** es mit dem Adenosin-Nukleotid an Position 461 am 3'-Ende von SEQ ID NO:3 oder SEQ ID NO:34 mit einem Brücken-Oligonukleotid oder mit einem Brücken-Polynukleotid, bevorzugt mit einem Brücken-Polynukleotid, welches die Translation von RNA gewährleistet, funktionsfähig verbunden ist.

10. Das Polynukleotid gemäß den Ansprüchen 3 bis 8,
**dadurch gekennzeichnet, dass** das zweite Polynukleotid, welches für ein oder mehrere Polypeptid(e) kodiert und welches mit dem Polynukleotid gemäß Anspruch 2 funktionsfähig verbunden ist, aus einem oder mehreren der Polynukleotide besteht, ausgewählt aus der Gruppe:
a) Polynukleotid (zwf-Gen), das für die Zwf-Untereinheit der Glucose-6-phosphate-1-dehydrogenase (Zwf, EC NR.: 1.1.1.49) kodiert,
b) Polynukleotid (opcA-Gen), das für die OpcA Untereinheit der Glucose-6-phosphate-1-dehydrogenase (OpcA, EC NR.: 1.1.1.49) kodiert,
c) Polynukleotid (devB-Gen), das für eine 6-Phosphogluconolactonase (DevB, EC NR.: 3.1.1.31) kodiert,
d) Polynukleotid (tkt-Gen), das für eine Transketolase (Tkt, EC NR.: 2.2.1.1) kodiert,
e) Polynukleotid (tal-Gen), das für eine Transaldolase (Tal, EC NR.: 2.2.1.2) kodiert,
f) Polynukleotid (gnd-Gen), das für eine 6-Phosphogluconat Dehydrogenase (Gnd, EC NR.: 1.1.1.44) kodiert,
g) Polynukleotid (rpe-Gen), das für eine Ribulose-Phosphat-3-Epimerase (Rpe, EC NR.: 5.1.3.1) kodiert,
h) Polynukleotid (rpi-Gen), das für eine Ribose-5-phosphat Isomerase B (Rpi, EC NR.: 5.3.1.6) kodiert,
i) Polynukleotid (ppc-Gen), das für eine Phosphoenolpyruvat Carboxylase (Ppc,EC NR.: 4.1.1.31) kodiert,
j) Polynukleotid (fbp-Gen), das für eine Fructose-1,6-bisphosphatase (Fbp, EC NR.: 3.1.3.11) kodiert,
k) Polynukleotid (pyc-Gen), das für eine Pyruvat-Carboxylase (Pyc, EC NR.: 6.4.1.1) kodiert,
l) Polynukleotid (dapA-Gen), das für eine Dihydrodipicolinat-Synthase (DapA, EC Nr.: 4.2.1.52) kodiert,
m) Polynukleotid (asd-Gen), das für eine Aspartatsemialdehyd-Dehydrogenase (Asd, EC Nr.: 1.2.1.11) kodiert,
n) Polynukleotid (ddh-Gen), das für eine meso-Diaminopimelate Dehydrogenase (Ddh, EC Nr.: 1.4.1.16) kodiert,
o) Polynukleotid, (lysA-Gen), das für eine Diaminopimelat-Decarboxylase (LysA, EC Nr.: 4.1.1.20) kodiert,
p) Polynukleotid (aat-Gen), das für eine Aspartat-Aminotransferase (Aat, EC Nr.: 2.6.1.1) kodiert,
q) Polynukleotid (lysE-Gen), das für ein Polypeptid mit L-Lysin-Export Aktivität (LysE, Lysin Efflux Permease) kodiert,
r) Polynukleotid (dapB-Gen), das für eine Dihydrodipicolinat-Reduktase (DapB, EC Nr.: 1.3.1.26) kodiert,
s) Polynukleotid (lysC-Gen), das für eine Aspartatkinase (LysC, EC NR.: 2.7.2.4) kodiert,
t) Polynukleotid (dapC-Gen), das für eine Succinyldiaminopimelate Aminotransferase, AT class I (DapC, EC Nr.: 2.6.1.17) kodiert,
u) Polynukleotid (dapD-Gen), das für eine Tetrahydrodipicolinat Succinylase (DapD, EC NR.: 2.3.1.117) kodiert,
v) Polynukleotid (dapE-Gen), das für eine Succinyldiaminopimelat Desuccinylase (DapE, EC NR.: 3.5.1.18) kodiert,
w) Polynukleotid (dapF-Gen), das für eine Diaminopimelat Epimerase (DapF, EC NR.: 5.1.1.7) kodiert,
x) Polynukleotide (ilvB-Gen und ilvN-Gen), die für die Untereinheiten einer Acetolactate Synthase (IlvBN, EC Nr.: 4.1.3.18) kodieren,
y) Polynukleotid (ilvC-Gen), das für eine Isomeroreductase (IlvC, EC Nr.: 1.1.1.86) kodiert,
z) Polynukleotid (ilvD-Gen), das für eine Dihydroxy-acid Dehydratase (IlvD, EC Nr.: 4.2.1.9) kodiert,
aa) Polynukleotid (ilvE-Gen), das für eine Transaminase (IlvE, EC Nr.: 2.6.1.42) kodiert,
bb) Polynukleotid (ilvA-Gen) das für eine Threonindehydratase (IlvA, EC Nr.: 4.3.1.19) kodiert,
cc) Polynukleotid (hom-Gen) das für eine Homoserindehydrogenase (Hom, EC-Nr.: 1.2.1.11 kodiert,
dd) Polynukleotid (thrB-Gen), das für eine Homoserinkinase (ThrB, EC-Nr.: 2.7.1.39) kodiert,
ee) Polynukleotid (thrC-Gen), das für eine Threoninsynthase (ThrC, EC-Nr.: 4.2.3.1) kodiert,
ff) Polynukleotid (leuA-Gen), das für eine Isopropylmalatsynthase (LeuA, EC-Nr.: 2.3.3.13) kodiert,
gg) Polynukleotid (leuB-Gen), das für eine Isopropylmalatdehydrogenase (LeuB, EC-Nr.: 1.1.1.85) kodiert,
hh) Polynukleotid (leuC-Gen), das für die große Untereinheit einer Isopropylmalatisomerase (LeuC, EC-Nr.: 4.2.1.33) kodiert,
ii) Polynukleotid (leuD-Gen), das für die kleine Untereinheit einer Isopropylmalatisomerase (LeuD, EC-Nr.: 4.2.1.33) kodiert,
jj) Polynukleotid (lysE-Gen), das für einen Lysin-/Ornithintransporter (DE-Anmeldenummer 102010003419.3) kodiert,
kk) Polynukleotid (argB-Gen), das für eine N-Acetylglutamatkinase (ArgB, EC-NR.: 2.7.2.8) kodiert,
ll) Polynukleotid (gdh-Gen), das für eine Glutamat-Dehydrogenase (Gdh, EC-Nr.: 1.4.1.3) kodiert,
mm) Polynukleotid (argJ-Gen), das für eine Glutamat N-Acetyltransferase (ArgJ, EC-Nr.: 2.3.1.35 und EC-Nr.: 2.3.1.1) kodiert,
nn) Polynukleotid (argC-Gen), das für eine N-Acetyl-Gamma-Glutamyl-Phosphat-Reduktase (ArgC, EC-Nr.: 1.2.1.38) kodiert, und
oo) Polynukleotid (argD-Gen), das für eine Acetyl-Ornithin Aminotransferase (ArgD, EC-Nr.: 2.6.1.11), kodiert.

11. Ein Vektor enthaltend das Polynukleotid gemäß einem der Ansprüche 1 bis 10.

12. Ein Mikroorganismus enthaltend das Polynukleotid gemäß SEQ ID NO:34.

13. Der Mikroorganismus enthaltend den Vektor gemäß Anspruch 11.

14. Der Mikroorganismus gemäß Anspruch 12, **dadurch gekennzeichnet, dass** das Polynukleotid im Chromosom integriert ist.

15. Der Mikroorganismus gemäß einem der Ansprüche 12 bis 14, **dadurch gekennzeichnet, dass** es sich um ein Bakterium der Gattung Corynebacterium, vorzugsweise Corynebacterium glutamicum, handelt.

16. Ein Mikroorganismus enthaltend das isolierte Polynukleotid gemäß einem der Ansprüche 1 bis 10, **dadurch gekennzeichnet, dass** das isolierte Polynukleotid mit Promotoraktivität gemäß einem der Ansprüche 1 bis 10 im Chromosom
1. gegen den nativen Promotor am nativen Genort des zweiten Polynukleotids ausgetauscht ist oder
2. mit dem zweiten Polynukleotid an dessen nativen Genort oder an einem weiteren Genort integriert ist, wobei das zweite Polynukleotid, welches für ein oder mehrere Polypeptid(e) kodiert, aus einem oder mehreren der Polynukleotide besteht, ausgewählt aus der Gruppe:
a) Polynukleotid (zwf-Gen), das für die Zwf-Untereinheit der Glucose-6-phosphate-1-dehydrogenase (Zwf, EC NR.: 1.1.1.49) kodiert,
b) Polynukleotid (opcA-Gen), das für die OpcA Untereinheit der Glucose-6-phosphate-1-dehydrogenase (OpcA, EC NR.: 1.1.1.49) kodiert,
c) Polynukleotid (devB-Gen), das für eine 6-Phosphogluconolactonase (DevB, EC NR.: 3.1.1.31) kodiert,
d) Polynukleotid (tkt-Gen), das für eine Transketolase (Tkt, EC NR.: 2.2.1.1) kodiert,
e) Polynukleotid (tal-Gen), das für eine Transaldolase (Tal, EC NR.: 2.2.1.2) kodiert,
f) Polynukleotid (gnd-Gen), das für eine 6-Phosphogluconat Dehydrogenase (Gnd, EC NR.: 1.1.1.44) kodiert,
g) Polynukleotid (rpe-Gen), das für eine Ribulose-Phosphat-3-Epimerase (Rpe, EC NR.: 5.1.3.1) kodiert,
h) Polynukleotid (rpi-Gen), das für eine Ribose-5-phosphat Isomerase B (Rpi, EC NR.: 5.3.1.6) kodiert,
i) Polynukleotid (ppc-Gen), das für eine Phosphoenolpyruvat Carboxylase (Ppc,EC NR.: 4.1.1.31) kodiert,
j) Polynukleotid (fbp-Gen), das für eine Fructose-1,6-bisphosphatase (Fbp, EC NR.: 3.1.3.11) kodiert,
k) Polynukleotid (pyc-Gen), das für eine Pyruvat-Carboxylase (Pyc, EC NR.: 6.4.1.1) kodiert,
l) Polynukleotid (dapA-Gen), das für eine Dihydrodipicolinat-Synthase (DapA, EC Nr.: 4.2.1.52) kodiert,
m) Polynukleotid (asd-Gen), das für eine Aspartatsemialdehyd-Dehydrogenase (Asd, EC Nr.: 1.2.1.11) kodiert,
n) Polynukleotid (ddh-Gen), das für eine meso-Diaminopimelate Dehydrogenase (Ddh, EC Nr.: 1.4.1.16) kodiert,
o) Polynukleotid, (lysA-Gen), das für eine Diaminopimelat-Decarboxylase (LysA, EC Nr.: 4.1.1.20) kodiert,
p) Polynukleotid (aat-Gen), das für eine Aspartat-Aminotransferase (Aat, EC Nr.: 2.6.1.1) kodiert,
q) Polynukleotid (lysE-Gen), das für ein Polypeptid mit L-Lysin-Export Aktivität (LysE, Lysin Efflux Permease) kodiert,
r) Polynukleotid (dapB-Gen), das für eine Dihydrodipicolinat-Reduktase (DapB, EC Nr.: 1.3.1.26) kodiert,
s) Polynukleotid (lysC-Gen), das für eine Aspartatkinase (LysC, EC NR.: 2.7.2.4) kodiert,
t) Polynukleotid (dapC-Gen), das für eine Succinyldiaminopimelate Aminotransferase, AT class I (DapC, EC Nr.: 2.6.1.17) kodiert,
u) Polynukleotid (dapD-Gen), das für eine Tetrahydrodipicolinat Succinylase (DapD, EC NR.: 2.3.1.117) kodiert,
v) Polynukleotid (dapE-Gen), das für eine Succinyldiaminopimelat Desuccinylase (DapE, EC NR.: 3.5.1.18) kodiert,
w) Polynukleotid (dapF-Gen), das für eine Diaminopimelat Epimerase (DapF, EC NR.: 5.1.1.7) kodiert,
x) Polynukleotide (ilvB-Gen und ilvN-Gen), die für die Untereinheiten einer Acetolactate Synthase (IlvBN, EC Nr.: 4.1.3.18) kodieren,
y) Polynukleotid (ilvC-Gen), das für eine Isomeroreductase (IlvC, EC Nr.: 1.1.1.86) kodiert,
z) Polynukleotid (ilvD-Gen), das für eine Dihydroxy-acid Dehydratase (IlvD, EC Nr.: 4.2.1.9) kodiert,
aa) Polynukleotid (ilvE-Gen), das für eine Transaminase (IlvE, EC Nr.: 2.6.1.42) kodiert,
bb) Polynukleotid (ilvA-Gen) das für eine Threonindehydratase (IlvA, EC Nr.: 4.3.1.19) kodiert,
cc) Polynukleotid (hom-Gen) das für eine Homoserindehydrogenase (Hom, EC-Nr.: 1.2.1.11 kodiert,
dd) Polynukleotid (thrB-Gen), das für eine Homoserinkinase (ThrB, EC-Nr.: 2.7.1.39) kodiert,
ee) Polynukleotid (thrC-Gen), das für eine Threoninsynthase (ThrC, EC-Nr.: 4.2.3.1) kodiert,
ff) Polynukleotid (leuA-Gen), das für eine Isopropylmalatsynthase (LeuA, EC-Nr.: 2.3.3.13) kodiert,
gg) Polynukleotid (leuB-Gen), das für eine Isopropylmalatdehydrogenase (LeuB, EC-Nr.: 1.1.1.85) kodiert,
hh) Polynukleotid (leuC-Gen), das für die große Untereinheit einer Isopropylmalatisomerase (LeuC, EC-Nr.: 4.2.1.33) kodiert,
ii) Polynukleotid (leuD-Gen), das für die kleine Untereinheit einer Isopropylmalatisomerase (LeuD, EC-Nr.: 4.2.1.33) kodiert,
jj) Polynukleotid (lysE-Gen), das für einen Lysin-/Ornithintransporter (DE-Anmeldenummer 102010003419.3) kodiert,
kk) Polynukleotid (argB-Gen), das für eine N-Acetylglutamatkinase (ArgB, EC-NR.: 2.7.2.8) kodiert,
ll) Polynukleotid (gdh-Gen), das für eine Glutamat-Dehydrogenase (Gdh, EC-Nr.: 1.4.1.3) kodiert,
mm) Polynukleotid (argJ-Gen), das für eine Glutamat N-Acetyltransferase (ArgJ, EC-Nr.: 2.3.1.35 und EC-Nr.: 2.3.1.1) kodiert,
nn) Polynukleotid (argC-Gen), das für eine N-Acetyl-Gamma-Glutamyl-Phosphat-Reduktase (ArgC, EC-Nr.: 1.2.1.38) kodiert, und
oo) Polynukleotid (argD-Gen), das für eine Acetyl-Ornithin Aminotransferase (ArgD, EC-Nr.: 2.6.1.11), kodiert.

17. Der Mikrorganismus gemäß einem der Ansprüche 12 bis 16, **dadurch gekennzeichnet, dass** der Mikroorganismus die Fahigkeit besitzt eine Feinchemikalie zu produzieren.

18. Der Mikrorganismus gemäß Anspruch 17, **dadurch gekennzeichnet, dass** es sich bei der Feinchemikalie um eine L-Aminosäure, vorzugsweise ausgewählt aus der Gruppe L-Isoleucin, L-Lysin, L-Methionin, L-Ornithin, L-Prolin, L-Valin und L-Tryptophan, oder eine organische Säure, vorzugsweise eine α-Ketosäure, insbesondere um eine α-Ketosäure ausgewählt aus der Gruppe α-Ketoisocapronsäure, α-Ketovaleriansäure und α-Keto-β-methylvaleriansäure, handelt.

19. Ein Verfahren zur Herstellung einer Feinchemikalie, **dadurch gekennzeichnet, dass** man folgende Schritte durchführt
a) Fermentation eines Mikrorganismus definiert in einem der Ansprüche 12 bis 18 in einem Fermentationsmedium unter Bildung einer Fermentationsbrühe
b) Akkumulation der Feinchemikalie in der Fermentationsbrühe aus a) und/oder in den Zellen des Mikrrorganismus.

20. Das Verfahren zur Herstellung einer Feinchemikalie nach Anspruch 19, **dadurch gekennzeichnet, dass** es sich um ein Verfahren ausgewählt aus der Gruppe Satzverfahren, Zulaufverfahren, repetitives Zulaufverfahren und kontinuierliches Verfahren handelt.

21. Das Verfahren nach Anspruch 19 bis 20, **dadurch gekennzeichnet, dass** man aus der Feinchemikaliehaltigen Fermentationsbrühe die Feinchemikalie oder ein flüssiges oder festes Feinchemikalie-haltiges Produkt gewinnt.

## Claims

1. An isolated polynucleotide having promoter activity, which comprises a polynucleotide having the nucleotide sequence depicted in SEQ ID NO:3 or SEQ ID NO:34.

2. The polynucleotide according to Claim 1, **characterized in that** it consists of the nucleotide sequence depicted in SEQ ID NO:3 or SEQ ID NO:34.

3. The polynucleotide according to Claim 2, **characterized in that** it is functionally linked at its 3' end, directly or by means of a linker oligonucleotide, or by means of a linker polynucleotide, preferably by means of a linker polynucleotide, to a second polynucleotide which contains at its 5' end an ATG or GTG start codon and codes for one or more polypeptide(s).

4. The polynucleotide according to Claim 3, **characterized in that** the polynucleotide of SEQ ID NO:3 or SEQ ID NO:34, via the adenosine nucleotide in position 461 at its 3' end, is linked by a linker oligonucleotide of 1, 2, 3, 4 or 5 nucleotides in length to the first nucleotide of the start codon of the second polynucleotide.

5. The polynucleotide according to Claim 3, **characterized in that** the polynucleotide of SEQ ID NO:3 or SEQ ID NO:34, via the adenosine nucleotide at its 3' end, is linked by a linker polynucleotide of from 6 to no more than (≤)600 nucleotides, preferably of 20, 21, 22, 23, 24 or 25 nucleotides, in length to the first nucleotide of the start codon of the second polynucleotide.

6. The polynucleotide according to Claim 5, **characterized in that** the linker polynucleotide comprises a nucleotide sequence which ensures translation of the synthesized RNA.

7. The polynucleotide according to Claims 5 to 6, **characterized in that** the sequence of the linker polynucleotide essentially consists of a nucleotide sequence depicted in SEQ ID NO:12 or SEQ ID NO:13.

8. The polynucleotide according to Claims 5 to 6, **characterized in that** the sequence of the linker polynucleotide consists of any of the nucleotide sequences depicted in SEQ ID NO:14, SEQ ID NO:15, SEQ ID NO:16, SEQ ID NO:17 or SEQ ID NO:18.

9. A polynucleotide according to Claim 2, **characterized in that** it is functionally linked via the adenosine nucleotide in position 461 at the 3' end of SEQ ID NO:3 or SEQ ID NO:34 to a linker oligonucleotide or to a linker polynucleotide, preferably to a linker polynucleotide, which ensures translation of RNA.

10. The polynucleotide according to Claims 3 to 8, **characterized in that** the second polynucleotide which codes for one or more polypeptide(s) and which is functionally linked to the polynucleotide according to Claim 2 consists of one or more of the polynucleotides selected from the group consisting of:
a) Polynucleotide (zwf gene) coding for the Zwf subunit of glucose-6-phosphate 1-dehydrogenase (Zwf, EC NO.: 1.1.1.49),
b) Polynucleotide (opcA gene) coding for the OpcA subunit of glucose-6-phosphate 1-dehydrogenase (OpcA, EC NO.: 1.1.1.49),
c) Polynucleotide (devB gene) coding for a 6-phosphogluconolactonase (DevB, EC NO.: 3.1.1.31),
d) Polynucleotide (tkt gene) coding for a transketolase (Tkt, EC NO.: 2.2.1.1),
e) Polynucleotide (tal gene) coding for a transaldolase (Tal, EC NO.: 2.2.1.2),
f) Polynucleotide (gnd gene) coding for a 6-phosphogluconate dehydrogenase (Gnd, EC NO.: 1.1.1.44),
g) Polynucleotide (rpe gene) coding for a ribulose-phosphate 3-epimerase (Rpe, EC NO.: 5.1.3.1),
h) Polynucleotide (rpi gene) coding for a ribose-5-phosphate isomerase B (Rpi, EC NO.: 5.3.1.6),
i) Polynucleotide (ppc gene) coding for a phosphoenolpyruvate carboxylase (Ppc, EC NO.: 4.1.1.31),
j) Polynucleotide (fbp gene) coding for a fructose 1,6-bisphosphatase (Fbp, EC NO.: 3.1.3.11),
k) Polynucleotide (pyc gene) coding for a pyruvate carboxylase (Pyc, EC NO.: 6.4.1.1),
l) Polynucleotide (dapA gene) coding for a dihydrodipicolinate synthase (DapA, EC No.: 4.2.1.52),
m) Polynucleotide (asd gene) coding for an aspartate-semialdehyde dehydrogenase (Asd, EC No.: 1.2.1.11),
n) Polynucleotide (ddh gene) coding for a *meso-*diaminopimelate dehydrogenase (Ddh, EC No.: 1.4.1.16),
o) Polynucleotide (lysA gene) coding for a diaminopimelate decarboxylase (LysA, EC No.: 4.1.1.20),
p) Polynucleotide (aat gene) coding for an aspartate aminotransferase (Aat, EC No.: 2.6.1.1),
q) Polynucleotide (lysE gene) coding for a polypeptide having L-lysine-export activity (LysE, lysine efflux permease),
r) Polynucleotide (dapB gene) coding for a dihydrodipicolinate reductase (DapB, EC No.: 1.3.1.26),
s) Polynucleotide (lysC gene) coding for an aspartate kinase (LysC, EC NO.: 2.7.2.4),
t) Polynucleotide (dapC gene) coding for a succinyldiaminopimelate aminotransferase, AT class I (DapC, EC No.: 2.6.1.17),
u) Polynucleotide (dapD gene) coding for a tetrahydrodipicolinate succinylase (DapD, EC NO.: 2.3.1.117),
v) Polynucleotide (dapE gene) coding for a succinyl-diaminopimelate desuccinylase (DapE, EC NO.: 3.5.1.18),
w) Polynucleotide (dapF gene) coding for a diaminopimelate epimerase (DapF, EC NO.: 5.1.1.7),
x) Polynucleotides (ilvB gene and ilvN gene) coding for the subunits of an acetolactate synthase (IlvBN, EC No.: 4.1.3.18),
y) Polynucleotide (ilvC gene) coding for an isomeroreductase (IlvC, EC No.: 1.1.1.86),
z) Polynucleotide (ilvD gene) coding for a dihydroxy-acid dehydratase (IlvD, EC No.: 4.2.1.9),
aa) Polynucleotide (ilvE gene) coding for a transaminase (IlvE, EC No.: 2.6.1.42),
bb) Polynucleotide (ilvA gene) coding for a threonine dehydratase (IlvA, EC No.: 4.3.1.19),
cc) Polynucleotide (hom gene) coding for a homoserine dehydrogenase (Hom, EC-No.: 1.2.1.11),
dd) Polynucleotide (thrB gene) coding for a homoserine kinase (ThrB, EC-No.: 2.7.1.39),
ee) Polynucleotide (thrC gene) coding for a threonine synthase (ThrC, EC-No.: 4.2.3.1),
ff) Polynucleotide (leuA gene) coding for an isopropylmalate synthase (LeuA, EC-No.: 2.3.3.13),
gg) Polynucleotide (leuB gene) coding for an isopropylmalate dehydrogenase (LeuB, EC-No.: 1.1.1.85),
hh) Polynucleotide (leuC gene) coding for the large subunit of an isopropylmalate isomerase (LeuC, EC-No.: 4.2.1.33),
ii) Polynucleotide (leuD gene) coding for the small subunit of an isopropylmalate isomerase (LeuD, EC-No.: 4.2.1.33),
jj) Polynucleotide (lysE gene) coding for a lysine/ornithine transporter (DE application number 102010003419.3),
kk) Polynucleotide (argB gene) coding for an N-acetylglutamate kinase (ArgB, EC-NO.: 2.7.2.8),
ll) Polynucleotide (gdh gene) coding for a glutamate dehydrogenase (Gdh, EC-No.: 1.4.1.3),
mm) Polynucleotide (argJ gene) coding for a glutamate N-acetyltransferase (ArgJ, EC-No.: 2.3.1.35 and EC-No.: 2.3.1.1),
nn) Polynucleotide (argC gene) coding for an N-acetyl-gamma-glutamyl-phosphate reductase (ArgC, EC-No.: 1.2.1.38), and
oo) Polynucleotide (argD gene) coding for an acetylornithine aminotransferase (ArgD, EC-No.: 2.6.1.11).

11. A vector comprising the polynucleotide according to any of Claims 1 to 10.

12. A microorganism comprising the polynucleotide according to SEQ ID NO:34.

13. The microorganism comprising the vector according to Claim 11.

14. The microorganism according to Claim 12, **characterized in that** the polynucleotide is integrated in the chromosome.

15. The microorganism according to any of Claims 12 to 14, **characterized in that** it is a bacterium of the genus *Corynebacterium,* preferably *Corynebacterium glutamicum.*

16. A microorganism comprising the isolated polynucleotide according to any of Claims 1 to 10, **characterized in that** the isolated polynucleotide having promoter activity according to any of Claims 1 to 10 in the chromosome
1. has been replaced with the native promoter at the native gene locus of the second polynucleotide, or
2. has been integrated with the second polynucleotide at the native gene locus of the latter or at another gene locus, wherein the second polynucleotide which codes for one or more polypeptide(s) consists of one or more of the polynucleotides selected from the group consisting of:
a) Polynucleotide (zwf gene) coding for the Zwf subunit of glucose-6-phosphate 1-dehydrogenase (Zwf, EC NO.: 1.1.1.49),
b) Polynucleotide (opcA gene) coding for the OpcA subunit of glucose-6-phosphate 1-dehydrogenase (OpcA, EC NO.: 1.1.1.49),
c) Polynucleotide (devB gene) coding for a 6-phosphogluconolactonase (DevB, EC NO.: 3.1.1.31),
d) Polynucleotide (tkt gene) coding for a transketolase (Tkt, EC NO.: 2.2.1.1),
e) Polynucleotide (tal gene) coding for a transaldolase (Tal, EC NO.: 2.2.1.2),
f) Polynucleotide (gnd gene) coding for a 6-phosphogluconate dehydrogenase (Gnd, EC NO.: 1.1.1.44),
g) Polynucleotide (rpe gene) coding for a ribulose-phosphate 3-epimerase (Rpe, EC NO.: 5.1.3.1),
h) Polynucleotide (rpi gene) coding for a ribose-5-phosphate isomerase B (Rpi, EC NO.: 5.3.1.6),
i) Polynucleotide (ppc gene) coding for a phosphoenolpyruvate carboxylase (Ppc, EC NO.: 4.1.1.31),
j) Polynucleotide (fbp gene) coding for a fructose 1,6-bisphosphatase (Fbp, EC NO.: 3.1.3.11),
k) Polynucleotide (pyc gene) coding for a pyruvate carboxylase (Pyc, EC NO.: 6.4.1.1),
l) Polynucleotide (dapA gene) coding for a dihydrodipicolinate synthase (DapA, EC No.: 4.2.1.52),
m) Polynucleotide (asd gene) coding for an aspartate-semialdehyde dehydrogenase (Asd, EC No.: 1.2.1.11),
n) Polynucleotide (ddh gene) coding for a *meso-*diaminopimelate dehydrogenase (Ddh, EC No.: 1.4.1.16),
o) Polynucleotide (lysA gene) coding for a diaminopimelate decarboxylase (LysA, EC No.: 4.1.1.20),
p) Polynucleotide (aat gene) coding for an aspartate aminotransferase (Aat, EC No.: 2.6.1.1),
q) Polynucleotide (lysE gene) coding for a polypeptide having L-lysine-export activity (LysE, lysine efflux permease),
r) Polynucleotide (dapB gene) coding for a dihydrodipicolinate reductase (DapB, EC No.: 1.3.1.26),
s) Polynucleotide (lysC gene) coding for an aspartate kinase (LysC, EC NO.: 2.7.2.4),
t) Polynucleotide (dapC gene) coding for a succinyldiaminopimelate aminotransferase, AT class I (DapC, EC No.: 2.6.1.17),
u) Polynucleotide (dapD gene) coding for a tetrahydrodipicolinate succinylase (DapD, EC NO.: 2.3.1.117),
v) Polynucleotide (dapE gene) coding for a succinyl-diaminopimelate desuccinylase (DapE, EC NO.: 3.5.1.18),
w) Polynucleotide (dapF gene) coding for a diaminopimelate epimerase (DapF, EC NO.: 5.1.1.7),
x) Polynucleotides (ilvB gene and ilvN gene) coding for the subunits of an acetolactate synthase (IlvBN, EC No.: 4.1.3.18),
y) Polynucleotide (ilvC gene) coding for an isomeroreductase (IlvC, EC No.: 1.1.1.86),
z) Polynucleotide (ilvD gene) coding for a dihydroxy-acid dehydratase (IlvD, EC No.: 4.2.1.9),
aa) Polynucleotide (ilvE gene) coding for a transaminase (IlvE, EC No.: 2.6.1.42),
bb) Polynucleotide (ilvA gene) coding for a threonine dehydratase (IlvA, EC No.: 4.3.1.19),
cc) Polynucleotide (hom gene) coding for a homoserine dehydrogenase (Hom, EC-No.: 1.2.1.11),
dd) Polynucleotide (thrB gene) coding for a homoserine kinase (ThrB, EC-No.: 2.7.1.39),
ee) Polynucleotide (thrC gene) coding for a threonine synthase (ThrC, EC-No.: 4.2.3.1),
ff) Polynucleotide (leuA gene) coding for an isopropylmalate synthase (LeuA, EC-No.: 2.3.3.13),
gg) Polynucleotide (leuB gene) coding for an isopropylmalate dehydrogenase (LeuB, EC-No.: 1.1.1.85),
hh) Polynucleotide (leuC gene) coding for the large subunit of an isopropylmalate isomerase (LeuC, EC-No.: 4.2.1.33),
ii) Polynucleotide (leuD gene) coding for the small subunit of an isopropylmalate isomerase (LeuD, EC-No.: 4.2.1.33),
jj) Polynucleotide (lysE gene) coding for a lysine/ornithine transporter (DE application number 102010003419.3),
kk) Polynucleotide (argB gene) coding for an N-acetylglutamate kinase (ArgB, EC-NO.: 2.7.2.8),
ll) Polynucleotide (gdh gene) coding for a glutamate dehydrogenase (Gdh, EC-No.: 1.4.1.3),
mm) Polynucleotide (argJ gene) coding for a glutamate N-acetyltransferase (ArgJ, EC-No.: 2.3.1.35 and EC-No.: 2.3.1.1),
nn) Polynucleotide (argC gene) coding for an N-acetyl-gamma-glutamyl-phosphate reductase (ArgC, EC-No.: 1.2.1.38), and
oo) Polynucleotide (argD gene) coding for an acetylornithine aminotransferase (ArgD, EC-No.: 2.6.1.11).

17. The microorganism according to any of Claims 12 to 16, **characterized in that** the microorganism is capable of producing a fine chemical.

18. The microorganism according to Claim 17, **characterized in that** the fine chemical is an L-amino acid, preferably selected from the group consisting of L-isoleucine, L-lysine, L-methionine, L-ornithine, L-proline, L-valine, and L-tryptophan, or an organic acid, preferably an α-keto acid, in particular an α-keto acid selected from the group consisting of α-ketoisocaproic acid, α-ketovaleric acid, and α-keto-β-methylvaleric acid.

19. A process for preparing a fine chemical, **characterized in that** it comprises the steps of
a) Fermenting a microorganism defined in any of Claims 12 to 18 in a fermentation medium to form a fermentation broth
b) Accumulating the fine chemical in the fermentation broth of a) and/or in the cells of the microorganism.

20. The process for preparing a fine chemical according to Claim 19, **characterized in that** said process is selected from the group consisting of batch process, fed-batch process, repeated fed-batch process, and continuous process.

21. The process according to Claims 19 to 20, **characterized in that** the fine chemical or a liquid or solid fine chemical-containing product is recovered from the fine chemical-containing fermentation broth.

## Revendications

1. Polynucléotide isolé présentant une activité de promoteur, qui comprend au moins un polynucléotide présentant la séquence nucléotidique représentée dans les séquences SEQ ID NO:3 ou SEQ ID NO:34.

2. Polynucléotide selon la revendication 1, **caractérisé en ce qu'**il est constitué par la séquence nucléotidique représentée dans les séquences SEQ ID NO:3 ou SEQ ID NO:34.

3. Polynucléotide selon la revendication 2, **caractérisé en ce qu'**il est relié fonctionnellement en l'extrémité 3' à un deuxième polynucléotide, qui contient en l'extrémité 5' un codon de départ ATG ou GTG et qui code pour un ou plusieurs polypeptide(s), directement, via un oligonucléotide de pontage ou via un polynucléotide de pontage, de préférence via un polynucléotide de pontage.

4. Polynucléotide selon la revendication 3, **caractérisé en ce que** le polynucléotide selon les séquences SEQ ID NO:3 ou SEQ ID NO:34 est relié avec le nucléotide d'adénosine en position 461 en l'extrémité 3' via un oligonucléotide de pontage présentant une longueur de 1, 2, 3, 4 ou 5 nucléotides au premier nucléotide du codon de départ du deuxième polynucléotide.

5. Polynucléotide selon la revendication 3, **caractérisé en ce que** le polynucléotide selon les séquences SEQ ID NO:3 ou SEQ ID NO:34 est relié avec le nucléotide d'adénosine en l'extrémité 3' via un polynucléotide de pontage présentant une longueur de 6 à au maximum (≤)600 nucléotides, de préférence une longueur de 20, 21, 22, 23, 24 ou 25 nucléotides, au premier nucléotide du codon de départ du deuxième polynucléotide.

6. Polynucléotide selon la revendication 5, **caractérisé en ce que** le polynucléotide de pontage comprend une séquence nucléotidique qui assure une traduction de l'ARN synthétisé.

7. Polynucléotide selon les revendications 5 à 6, **caractérisé en ce que** la séquence du polynucléotide de pontage est essentiellement constituée par une séquence nucléotidique représentée dans les séquences SEQ ID NO:12 ou SEQ ID NO:13.

8. Polynucléotide selon les revendications 5 à 6, **caractérisé en ce que** la séquence du polynucléotide de pontage est constituée par une des séquences nucléotidiques représentées dans les séquences SEQ ID NO:14, SEQ ID NO:15, SEQ ID NO:16, SEQ ID NO:17 ou SEQ ID NO:18.

9. Polynucléotide selon la revendication 2, **caractérisé en ce qu'**il est relié fonctionnellement avec le nucléotide d'adénosine en position 461 en l'extrémité 3' de la séquence SEQ ID NO:3 ou de la séquence SEQ ID NO:34 via un oligonucléotide de pontage ou un polynucléotide de pontage, de préférence avec un polynucléotide de pontage, qui assure la traduction de l'ARN.

10. Polynucléotide selon les revendications 3 à 8, **caractérisé en ce que** le deuxième polynucléotide, qui code pour un ou plusieurs polypeptide(s) et qui est relié fonctionnellement au polynucléotide selon la revendication 2, est constitué par un ou plusieurs des polynucléotides choisis dans le groupe formé par :
a) polynucléotide (gène zwf), qui code pour la sous-unité Zwf de la glucose-6-phosphate-1-déshydrogénase (Zwf, n° EC : 1.1.1.49),
b) polynucléotide (gène opcA), qui code pour la sous-unité OpcA de la glucose-6-phosphate-1-déshydrogénase (OpcA, n° EC : 1.1.1.49),
c) polynucléotide (gène devB), qui code pour une 6-phosphogluconolactonase (DevB, n° EC : 3.1.1.31),
d) polynucléotide (gène tkt), qui code pour une transcétolase (Tkt, n° EC : 2.2.1.1),
e) polynucléotide (gène tal), qui code pour une transaldolase (Tal, n° EC : 2.2.1.2),
f) polynucléotide (gène gnd), qui code pour une 6-phosphogluconate-déshydrogénase (Gnd, n° EC : 1.1.1.44),
g) polynucléotide (gène rpe), qui code pour une ribulose-phosphate-3-épimérase (Rpe, n° EC : 5.1.3.1),
h) polynucléotide (gène rpi), qui code pour une ribulose-5-phosphate-isomérase B (Rpi, n° EC : 5.3.1.6),
i) polynucléotide (gène ppc), qui code pour une phosphoénolpyruvate-carboxylase (Ppc, n° EC : 4.1.1.31),
j) polynucléotide (gène fbp), qui code pour une fructose-1,6-biphosphatase (Fbp, n° EC : 3.1.3.11),
k) polynucléotide (gène pyc), qui code pour une pyruvate-carboxylase (Pyc, n° EC : 6.4.1.1),
l) polynucléotide (gène dapA), qui code pour une dihydrodipicolinate-synthase (DapA, n° EC : 4.2.1.52),
m) polynucléotide (gène asd), qui code pour une aspartate-semi-aldéhyde-déshydrogénase (Asd, n° EC : 1.2.1.11),
n) polynucléotide (gène ddh), qui code pour une méso-diaminopimélate-déshydrogénase (Ddh, n° EC : 1.4.1.16),
o) polynucléotide (gène lysA), qui code pour une diaminopimélate-décarboxylase (LysA, n° EC : 4.1.1.20),
p) polynucléotide (gène aat), qui code pour une aspartate-aminotransférase (Aat, n° EC : 2.6.1.1),
q) polynucléotide (gène lysE), qui code pour un polypeptide présentant une activité L-lysine-export (LysE, lysine efflux perméase),
r) polynucléotide (gène dapB), qui code pour une dihydrodipicolinate-réductase (DapB, n° EC : 1.3.1.26),
s) polynucléotide (gène lysC), qui code pour une aspartate-kinase (LysC, n° EC : 2.7.2.4),
t) polynucléotide (gène dapC), qui code pour une succinyldiaminopimélate aminotransférase classe AT I (DapC, n° EC : 2.6.1.17),
u) polynucléotide (gène dapD), qui code pour une tétrahydrodipicolinate-succinylase (DapD, n° EC : 2.3.1.117),
v) polynucléotide (gène dapE), qui code pour une succinyldiaminopimélate désuccinylase (DapE n° EC : 3.5.1.18),
w) polynucléotide (gène dapF), qui code pour une diaminopimélate-épimérase (DapF, n° EC : 5.1.1.7),
x) polynucléotides (gène ilvB et gène ilvN), qui codent pour les sous-unités d'une acétolactate-synthase (IlvBN, n° EC : 4.1.3.18),
y) polynucléotide (gène ilvC), qui code pour une isoméroréductase (IlvC, n° EC : 1.1.1.86),
z) polynucléotide (gène ilvD), qui code pour une dihydroxyacide-déshydratase (IlvD, n° EC : 4.2.1.9),
aa) polynucléotide (gène ilvE), qui code pour une transaminase (IlvE, n° EC : 2.6.1.42),
bb) polynucléotide (gène ilvA), qui code pour une thréonine-déshydratase (IlvA, n° EC : 4.3.1.19),
cc) polynucléotide (gène hom), qui code pour une homosérine-déshydrogénase (Hom, n° EC : 1.2.1.11),
dd) polynucléotide (gène thrB), qui code pour une homosérine-kinase (ThrB, n° EC : 2.7.1.39),
ee) polynucléotide (gène thrC), qui code pour une thréonine-synthase (ThrC, n° EC : 4.2.3.1),
ff) polynucléotide (gène leuA), qui code pour une isopropylmalate-synthase (LeuA, n° EC : 2.3.3.13),
gg) polynucléotide (gène leuB), qui code pour une isopropylmalate-déshydrogénase (LeuB, n° EC : 1.1.1.85),
hh) polynucléotide (gène leuC), qui code pour la grande sous-unité d'une isopropylmalate-isomérase (LeuC, n° EC : 4.2.1.33),
ii) polynucléotide (gène leuD), qui code pour la petite sous-unité d'une isopropylmalate-isomérase (LeuD, n° EC : 4.2.1.33),
jj) polynucléotide (gène lysE), qui code pour un lysine/ornithine-transporteur (demande DE 102010003419.3),
kk) polynucléotide (gène argB), qui code pour une N-acétylglutamate-kinase (ArgB, n° EC : 2.7.2.8),
ll) polynucléotide (gène gdh), qui code pour une glutamate-déshydrogénase (Gdh, n° EC : 1.4.1.3),
mm) polynucléotide (gène argJ), qui code pour une glutamate-N-acétyl-transférase (ArgJ, n° EC : 2.3.1.35 et n° EC : 2.3.1.1),
nn) polynucléotide (gène argC), qui code pour une N-acétyl-gamma-glutamyl-phosphate-réductase (ArgC, n° EC : 1.2.1.38) et
oo) polynucléotide (gène argD), qui code pour une acétyl-ornithine-aminotransférase (ArgD, n° EC : 2.6.1.11).

11. Vecteur, contenant le polynucléotide selon l'une quelconque des revendications 1 à 10.

12. Micro-organisme contenant le polynucléotide selon la séquence SEQ ID NO:34.

13. Micro-organisme contenant le vecteur selon la revendication 11.

14. Micro-organisme selon la revendication 12, **caractérisé en ce que** le polynucléotide est intégré dans le chromosome.

15. Micro-organisme selon l'une quelconque des revendications 12 à 14, **caractérisé en ce qu'**il s'agit d'une bactérie du genre Corynebacterium, de préférence Corynebacterium glutamicum.

16. Micro-organisme contenant le polynucléotide isolé selon l'une quelconque des revendications 1 à 10, **caractérisé en ce que** le polynucléotide isolé présentant une activité de promoteur selon l'une quelconque des revendications 1 à 10 dans le chromosome 1. est remplacé par le promoteur naturel au niveau du site génique naturel du deuxième polynucléotide ou 2. **en ce qu'**il est intégré avec le deuxième polynucléotide en son site génique naturel ou en une autre site génique, le deuxième polynucléotide, qui code pour un ou plusieurs polypeptide(s) étant constitué par un ou plusieurs des polynucléotides choisis dans le groupe formé par :
a) polynucléotide (gène zwf), qui code pour la sous-unité Zwf de la glucose-6-phosphate-1-déshydrogénase (Zwf, n° EC : 1.1.1.49),
b) polynucléotide (gène opcA), qui code pour la sous-unité OpcA de la glucose-6-phosphate-1-déshydrogénase (OpcA, n° EC : 1.1.1.49),
c) polynucléotide (gène devB), qui code pour une 6-phosphogluconolactonase (DevB, n° EC : 3.1.1.31),
d) polynucléotide (gène tkt), qui code pour une transcétolase (Tkt, n° EC : 2.2.1.1),
e) polynucléotide (gène tal), qui code pour une transaldolase (Tal, n° EC : 2.2.1.2),
f) polynucléotide (gène gnd), qui code pour une 6-phosphogluconate-déshydrogénase (Gnd, n° EC : 1.1.1.44),
g) polynucléotide (gène rpe), qui code pour une ribulose-phosphate-3-épimérase (Rpe, n° EC : 5.1.3.1),
h) polynucléotide (gène rpi), qui code pour une ribulose-5-phosphate-isomérase B (Rpi, n° EC : 5.3.1.6),
i) polynucléotide (gène ppc), qui code pour une phosphoénolpyruvate-carboxylase (Ppc, n° EC : 4.1.1.31),
j) polynucléotide (gène fbp), qui code pour une fructose-1,6-biphosphatase (Fbp, n° EC : 3.1.3.11),
k) polynucléotide (gène pyc), qui code pour une pyruvate-carboxylase (Pyc, n° EC : 6.4.1.1),
l) polynucléotide (gène dapA), qui code pour une dihydrodipicolinate-synthase (DapA, n° EC : 4.2.1.52),
m) polynucléotide (gène asd), qui code pour une aspartate-semi-aldéhyde-déshydrogénase (Asd, n° EC : 1.2.1.11),
n) polynucléotide (gène ddh), qui code pour une méso-diaminopimélate-déshydrogénase (Ddh, n° EC : 1.4.1.16),
o) polynucléotide (gène lysA), qui code pour une diaminopimélate-décarboxylase (LysA, n° EC : 4.1.1.20),
p) polynucléotide (gène aat), qui code pour une aspartate-aminotransférase (Aat, n° EC : 2.6.1.1),
q) polynucléotide (gène lysE), qui code pour un polypeptide présentant une activité L-lysine-export (LysE, lysine efflux perméase),
r) polynucléotide (gène dapB), qui code pour une dihydrodipicolinate-réductase (DapB, n° EC : 1.3.1.26),
s) polynucléotide (gène lysC), qui code pour une aspartate-kinase (LysC, n° EC : 2.7.2.4),
t) polynucléotide (gène dapC), qui code pour une succinyldiaminopimélate aminotransférase classe AT I (DapC, n° EC : 2.6.1.17),
u) polynucléotide (gène dapD), qui code pour une tétrahydrodipicolinate-succinylase (DapD, n° EC : 2.3.1.117),
v) polynucléotide (gène dapE), qui code pour une succinyldiaminopimélate désuccinylase (DapE n° EC : 3.5.1.18),
w) polynucléotide (gène dapF), qui code pour une diaminopimélate-épimérase (DapF, n° EC : 5.1.1.7),
x) polynucléotides (gène ilvB et gène ilvN), qui codent pour les sous-unités d'une acétolactate-synthase (IlvBN, n° EC : 4.1.3.18),
y) polynucléotide (gène ilvC), qui code pour une isoméroréductase (IlvC, n° EC : 1.1.1.86),
z) polynucléotide (gène ilvD), qui code pour une dihydroxyacide-déshydratase (IlvD, n° EC : 4.2.1.9),
aa) polynucléotide (gène ilvE), qui code pour une transaminase (IlvE, n° EC : 2.6.1.42),
bb) polynucléotide (gène ilvA), qui code pour une thréonine-déshydratase (IlvA, n° EC : 4.3.1.19),
cc) polynucléotide (gène hom), qui code pour une homosérine-déshydrogénase (Hom, n° EC : 1.2.1.11),
dd) polynucléotide (gène thrB), qui code pour une homosérine-kinase (ThrB, n° EC : 2.7.1.39),
ee) polynucléotide (gène thrC), qui code pour une thréonine-synthase (ThrC, n° EC : 4.2.3.1),
ff) polynucléotide (gène leuA), qui code pour une isopropylmalate-synthase (LeuA, n° EC : 2.3.3.13),
gg) polynucléotide (gène leuB), qui code pour une isopropylmalate-déshydrogénase (LeuB, n° EC : 1.1.1.85),
hh) polynucléotide (gène leuC), qui code pour la grande sous-unité d'une isopropylmalate-isomérase (LeuC, n° EC : 4.2.1.33),
ii) polynucléotide (gène leuD), qui code pour la petite sous-unité d'une isopropylmalate-isomérase (LeuD, n° EC : 4.2.1.33),
jj) polynucléotide (gène lysE), qui code pour un lysine/ornithine-transporteur (demande DE 102010003419.3),
kk) polynucléotide (gène argB), qui code pour une N-acétylglutamate-kinase (ArgB, n° EC : 2.7.2.8),
ll) polynucléotide (gène gdh), qui code pour une glutamate-déshydrogénase (Gdh, n° EC : 1.4.1.3),
mm) polynucléotide (gène argJ), qui code pour une glutamate-N-acétyl-transférase (ArgJ, n° EC : 2.3.1.35 et n° EC : 2.3.1.1),
nn) polynucléotide (gène argC), qui code pour une N-acétyl-gamma-glutamyl-phosphate-réductase (ArgC, n° EC : 1.2.1.38) et
oo) polynucléotide (gène argD), qui code pour une acétyl-ornithine-aminotransférase (ArgD, n° EC : 2.6.1.11).

17. Micro-organisme selon l'une quelconque des revendications 12 à 16, **caractérisé en ce que** le micro-organisme présente l'aptitude à produire un produit chimique fin.

18. Micro-organisme selon la revendication 17, **caractérisé en ce qu'**il s'agit, pour le produit chimique fin, d'un acide L-aminé, de préférence choisi dans le groupe formé par la L-isoleucine, la L-lysine, la L-méthionine, la L-ornithine, la L-proline, la L-valine et le L-tryptophane, ou d'un acide organique, de préférence un α-cétoacide, en particulier un α-cétoacide choisi dans le groupe formé par l'acide α-céto-isocaproïque, l'acide α-cétovalérianique et l'acide α-céto-ß-méthylvalérianique.

19. Procédé pour la préparation d'un produit chimique fin, **caractérisé en ce qu'**on réalise les étapes suivantes :
a) fermentation d'un micro-organisme défini dans l'une quelconque des revendications 12 à 18 dans un milieu de fermentation avec formation d'un bouillon de fermentation
b) accumulation du produit chimique fin dans le bouillon de fermentation de a) et/ou dans les cellules du micro-organisme.

20. Procédé pour la préparation d'un produit chimique fin selon la revendication 19, **caractérisé en ce qu'**il s'agit d'un procédé choisi dans le groupe formé par les procédés par lots, les procédés à alimentation, les procédés à alimentation répétitifs et les procédés continus.

21. Procédé selon la revendication 19 à 20, **caractérisé en ce qu'**on obtient le produit chimique fin ou un produit liquide ou solide contenant le produit chimique fin à partir du bouillon de fermentation contenant le produit chimique fin.


### SEQUENCE LISTING

<110> Evonik Degussa GmbH
<120> Varianten des Promotors Pgap
<130> 2010E00462
<160> 50
<170> PatentIn version 3.5
<210> 1
   <211> 484
   <212> DNA
   <213> Corynebacterium glutamicum ATCC13032
<220>
   <221> misc_feature
   <222> (1)..(484)
   <223> SEQ ID NO: 20 aus US 2008/0050786
<220>
   <221> misc_feature
   <222> (247)..(346)
   <223> Sequenz des Promotors P-gap nach Patek et al. (Journal of Biotechnology 104, 311-323 (2003))
<220>
   <221> misc_feature
   <222> (289) .. (294)
   <223> - 10 Region des Promotors P-gap nach Patek et al.
<220>
   <221> misc_feature
   <222> (301)..(301)
   <223> Transkriptionsstart
<220>
   <221> misc_feature
   <222> (363)..(363)
   <223> Nukleobase Guanin
<220>
   <221> RBS
   <222> (473)..(478)
   <223> Ribosomenbindestelle
<400> 1
   ttgaagccta aaaacgaccg agcctattgg gattaccatt gaagccagtg tgagttgcat      60
   cacattggct tcaaatctga gactttaatt tgtggattca cgggggtgta atgtagttca     120
   taattaaccc cattcggggg agcagatcgt agtgcgaacg atttcaggtt cgttccctgc     180
   aaaaactatt tagcgcaagt gttggaaatg cccccgtttg gggtcaatgt ccatttttga     240
   atgtgtctgt atgattttgc atctgctgcg aaatctttgt ttccccgcta aagttgagga     300
   caggttgaca cggagttgac tcgacgaatt atccaatgtg agtaggtttg gtgcgtgagt     360
   tggaaaaatt cgccatactc gcccttgggt tctgtcagct caagaattct tgagtgaccg     420
   atgctctgat tgacctaact gcttgacaca ttgcatttcc tacaatcttt agaggagaca     480
   caac                                                                  484
<210> 2
   <211> 461
   <212> DNA
   <213> Corynebacterium glutamicum ATCC13032
<220>
   <221> promoter
   <222> (1)..(461)
<220>
   <221> misc_feature
   <222> (362)..(362)
   <223> Nukleobase Guanin (g)
<400> 2
   tgaagcctaa aaacgaccga gcctattggg attaccattg aagccagtgt gagttgcatc 60
   acattggctt caaatctgag actttaattt gtggattcac gggggtgtaa tgtagttcat 120
   aattaacccc attcggggga gcagatcgta gtgcgaacga tttcaggttc gttccctgca 180
   aaaactattt agcgcaagtg ttggaaatgc ccccgtttgg ggtcaatgtc catttttgaa 240
   tgtgtctgta tgattttgca tctgctgcga aatctttgtt tccccgctaa agttgaggac 300
   aggttgacac ggagttgact cgacgaatta tccaatgtga gtaggtttgg tgcgtgagtt 360
   ggaaaaattc gccatactcg cccttgggtt ctgtcagctc aagaattctt gagtgaccga 420
   tgctctgatt gacctaactg cttgacacat tgcatttcct a 461
<210> 3
   <211> 461
   <212> DNA
   <213> Corynebacterium glutamicum ATCC13032
<220>
   <221> promoter
   <222> (1)..(461)
<220>
   <221> mutation
   <222> (362)..(362)
   <223> Nukleobase Adenin (a)
<400> 3
   tgaagcctaa aaacgaccga gcctattggg attaccattg aagccagtgt gagttgcatc 60
   acattggctt caaatctgag actttaattt gtggattcac gggggtgtaa tgtagttcat 120
   aattaacccc attcggggga gcagatcgta gtgcgaacga tttcaggttc gttccctgca 180
   aaaactattt agcgcaagtg ttggaaatgc ccccgtttgg ggtcaatgtc catttttgaa 240
   tgtgtctgta tgattttgca tctgctgcga aatctttgtt tccccgctaa agttgaggac 300
   aggttgacac ggagttgact cgacgaatta tccaatgtga gtaggtttgg tgcgtgagtt 360
   gaaaaaattc gccatactcg cccttgggtt ctgtcagctc aagaattctt gagtgaccga 420
   tgctctgatt gacctaactg cttgacacat tgcatttcct a 461
<210> 4
   <211> 10
   <212> DNA
   <213> Corynebacterium glutamicum
<220>
   <221> RBS
   <222> (1)..(10)
   <223> Shine-Dalgarno Sequenz gemäß Amador et al. (Microbiology, 145, 915-924 (1999))
<400> 4
   agaaaggagg 10
<210> 5
   <211> 9
   <212> DNA
   <213> Corynebacterium glutamicum
<220>
   <221> RBS
   <222> (1)..(9)
   <223> Shine-Dalgarno Sequenz gemäß Martin et. al. (Journal of Biotechnology 104, 41 - 53 (2003))
<400> 5
   gaaaggagg 9
<210> 6
   <211> 9
   <212> DNA
   <213> Corynebacterium glutamicum
<220>
   <221> RBS
   <222> (1)..(9)
   <223> Ribosomenbindestelle des Gens ppc nach O'Regan et al. (Gene 77, 237-251 (1989))
<400> 6
   gaaagagtg 9
<210> 7
   <211> 7
   <212> DNA
   <213> Corynebacterium glutamicum
<220>
   <221> RBS
   <222> (1)..(7)
   <223> Ribosomenbindestelle des Gens aceA nach Reinscheid et al. (Journal of Bacteriology 176 (12), 3474-3483 (1994))
<400> 7
   aaggaag 7
<210> 8
   <211> 7
   <212> DNA
   <213> Corynebacterium glutamicum
<220>
   <221> RBS
   <222> (1)..(7)
   <223> Ribosomenbindestelle des Gens thiX nach Reinscheid et al. (Journal of Bacteriology 176 (12), 3474-3483 (1994))
<400> 8
   gaaagga 7
<210> 9
   <211> 7
   <212> DNA
   <213> Corynebacterium glutamicum
<220>
   <221> RBS
   <222> (1)..(7)
   <223> Ribosomenbindestelle des Gens sod gemäß WO2005/059144
<400> 9
   gaaagga 7
<210> 10
   <211> 7
   <212> DNA
   <213> Corynebacterium glutamicum
<220>
   <221> RBS
   <222> (1)..(7)
   <223> Ribosomenbinstelle des Gens tuf gemäß WO2005/059093
<400> 10
   aggagga 7
<210> 11
   <211> 7
   <212> DNA
   <213> Corynebacterium glutamicum
<220>
   <221> RBS
   <222> (1)..(7)
   <223> Ribosomenbindestelle gemäß SEQ ID NO:44 von EP 1 918 378 A1
<400> 11
   gaaagga 7
<210> 12
   <211> 7
   <212> DNA
   <213> Corynebacterium glutamicum
<220>
   <221> RBS
   <222> (1)..(7)
   <223> Ribosomenbindestelle des Gens fbp
<400> 12
   gaggagg 7
<210> 13
   <211> 6
   <212> DNA
   <213> Corynebacterium glutamicum
<220>
   <221> RBS
   <222> (1)..(6)
   <223> Ribosomenbindestelle des Gens gap nach Eikmanns (Journal of Bacteriology 174 (19), 6076-6086 (1992))
<400> 13
   aggaga 6
<210> 14
   <211> 22
   <212> DNA
   <213> Corynebacterium glutamicum
<220>
   <221> RBS
   <222> (11)..(16)
   <223> Ribosomenbindestelle des Gens gap
<400> 14
   caatctttag aggagacaca ac 22
<210> 15
   <211> 22
   <212> DNA
   <213> Corynebacterium glutamicum
<220>
   <221> misc_feature
   <222> (2)..(7)
   <223> SspI-Schnittstelle
<220>
   <221> RBS
   <222> (11)..(16)
   <223> Ribosomenbindestelle des Gens gap
<400> 15
   caatatttag aggagacaca ac 22
<210> 16
   <211> 21
   <212> DNA
   <213> Corynebacterium glutamicum
<220>
   <221> misc_feature
   <222> (4)..(8)
   <223> Bereich der NruI-Schnittstelle
<220>
   <221> mutation
   <222> (7)..(7)
   <223> Deletion des Nukleotids Cytosin (c)
<220>
   <221> RBS
   <222> (10)..(15)
   <223> Ribosomenbindestelle des Gens gap
<400> 16
   caatcggaga ggagacacaa c 21
<210> 17
   <211> 22
   <212> DNA
   <213> Corynebacterium glutamicum
<220>
   <221> misc_feature
   <222> (4)..(9)
   <223> NruI-Schnittstelle
<220>
   <221> RBS
   <222> (11)..(16)
   <223> Ribosomenbindestelle des Gens gap
<400> 17
   caatcgcgag aggagacaca ac 22
<210> 18
   <211> 22
   <212> DNA
   <213> Corynebacterium glutamicum
<220>
   <221> misc_feature
   <222> (4)..(9)
   <223> NruI-Schnittstelle
<220>
   <221> RBS
   <222> (8)..(14)
   <223> Ribosomenbindestelle des Gens fbp
<400> 18
   caatcgcgag gaggcccttc ag 22
<210> 19
   <211> 1266
   <212> DNA
   <213> Corynebacterium glutamicum
<220>
   <221> CDS
   <222> (1)..(1266)
   <223> lysC Kodierregion
<400> 19
   gtg gcc ctg gtc gta cag aaa tat ggc ggt tcc tcg ctt gag agt gcg        48
   Val Ala Leu Val Val Gln Lys Tyr Gly Gly Ser Ser Leu Glu Ser Ala
   1               5                   10                  15
   gaa cgc att aga aac gtc gct gaa cgg atc gtt gcc acc aag aag gct        96
   Glu Arg Ile Arg Asn Val Ala Glu Arg Ile Val Ala Thr Lys Lys Ala
               20                  25                  30
   gga aat gat gtc gtg gtt gtc tgc tcc gca atg gga gac acc acg gat       144
   Gly Asn Asp Val Val Val Val Cys Ser Ala Met Gly Asp Thr Thr Asp
           35                  40                  45
   gaa ctt cta gaa ctt gca gcg gca gtg aat ccc gtt ccg cca gct cgt       192
   Glu Leu Leu Glu Leu Ala Ala Ala Val Asn Pro Val Pro Pro Ala Arg
       50                  55                  60
   gaa atg gat atg ctc ctg act gct ggt gag cgt att tct aac gct ctc       240
   Glu Met Asp Met Leu Leu Thr Ala Gly Glu Arg Ile Ser Asn Ala Leu
   65                  70                  75                  80
   gtc gcc atg gct att gag tcc ctt ggc gca gaa gcc caa tct ttc acg       288
   Val Ala Met Ala Ile Glu Ser Leu Gly Ala Glu Ala Gln Ser Phe Thr
                   85                  90                  95
   ggc tct cag gct ggt gtg ctc acc acc gag cgc cac gga aac gca cgc       336
   Gly Ser Gln Ala Gly Val Leu Thr Thr Glu Arg His Gly Asn Ala Arg
               100                 105                 110
   att gtt gat gtc act cca ggt cgt gtg cgt gaa gca ctc gat gag ggc       384
   Ile Val Asp Val Thr Pro Gly Arg Val Arg Glu Ala Leu Asp Glu Gly
           115                 120                 125
   aag atc tgc att gtt gct ggt ttc cag ggt gtt aat aaa gaa acc cgc       432
   Lys Ile Cys Ile Val Ala Gly Phe Gln Gly Val Asn Lys Glu Thr Arg
       130                 135                 140
   gat gtc acc acg ttg ggt cgt ggt ggt tct gac acc act gca gtt gcg       480
   Asp Val Thr Thr Leu Gly Arg Gly Gly Ser Asp Thr Thr Ala Val Ala
   145                 150                 155                 160
   ttg gca gct gct ttg aac gct gat gtg tgt gag att tac tcg gac gtt       528
   Leu Ala Ala Ala Leu Asn Ala Asp Val Cys Glu Ile Tyr Ser Asp Val
                   165                 170                 175
   gac ggt gtg tat acc gct gac ccg cgc atc gtt cct aat gca cag aag       576
   Asp Gly Val Tyr Thr Ala Asp Pro Arg Ile Val Pro Asn Ala Gln Lys
               180                 185                 190
   ctg gaa aag ctc agc ttc gaa gaa atg ctg gaa ctt gct gct gtt ggc       624
   Leu Glu Lys Leu Ser Phe Glu Glu Met Leu Glu Leu Ala Ala Val Gly
           195                 200                 205
   tcc aag att ttg gtg ctg cgc agt gtt gaa tac gct cgt gca ttc aat       672
   Ser Lys Ile Leu Val Leu Arg Ser Val Glu Tyr Ala Arg Ala Phe Asn
       210                 215                 220
   gtg cca ctt cgc gta cgc tcg tct tat agt aat gat ccc ggc act ttg       720
   Val Pro Leu Arg Val Arg Ser Ser Tyr Ser Asn Asp Pro Gly Thr Leu
   225                 230                 235                 240
   att gcc ggc tct atg gag gat att cct gtg gaa gaa gca gtc ctt acc       768
   Ile Ala Gly Ser Met Glu Asp Ile Pro Val Glu Glu Ala Val Leu Thr
                   245                 250                 255
   ggt gtc gca acc gac aag tcc gaa gcc aaa gta acc gtt ctg ggt att       816
   Gly Val Ala Thr Asp Lys Ser Glu Ala Lys Val Thr Val Leu Gly Ile
               260                 265                 270
   tcc gat aag cca ggc gag gct gcg aag gtt ttc cgt gcg ttg gct gat       864
   Ser Asp Lys Pro Gly Glu Ala Ala Lys Val Phe Arg Ala Leu Ala Asp
           275                 280                 285
   gca gaa atc aac att gac atg gtt ctg cag aac gtc tct tct gta gaa       912
   Ala Glu Ile Asn Ile Asp Met Val Leu Gln Asn Val Ser Ser Val Glu
       290                 295                 300
   gac ggc acc acc gac atc acc ttc acc tgc cct cgt tcc gac ggc cgc       960
   Asp Gly Thr Thr Asp Ile Thr Phe Thr Cys Pro Arg Ser Asp Gly Arg
   305                 310                 315                 320
   cgc gcg atg gag atc ttg aag aag ctt cag gtt cag ggc aac tgg acc      1008
   Arg Ala Met Glu Ile Leu Lys Lys Leu Gln Val Gln Gly Asn Trp Thr
                   325                 330                 335
   aat gtg ctt tac gac gac cag gtc ggc aaa gtc tcc ctc gtg ggt gct      1056
   Asn Val Leu Tyr Asp Asp Gln Val Gly Lys Val Ser Leu Val Gly Ala
               340                 345                 350
   ggc atg aag tct cac cca ggt gtt acc gca gag ttc atg gaa gct ctg      1104
   Gly Met Lys Ser His Pro Gly Val Thr Ala Glu Phe Met Glu Ala Leu
           355                 360                 365
   cgc gat gtc aac gtg aac atc gaa ttg att tcc acc tct gag att cgt      1152
   Arg Asp Val Asn Val Asn Ile Glu Leu Ile Ser Thr Ser Glu Ile Arg
       370                 375                 380
   att tcc gtg ctg atc cgt gaa gat gat ctg gat gct gct gca cgt gca      1200
   Ile Ser Val Leu Ile Arg Glu Asp Asp Leu Asp Ala Ala Ala Arg Ala
   385                 390                 395                 400
   ttg cat gag cag ttc cag ctg ggc ggc gaa gac gaa gcc gtc gtt tat      1248
   Leu His Glu Gln Phe Gln Leu Gly Gly Glu Asp Glu Ala Val Val Tyr
                   405                 410                 415
   gca ggc acc gga cgc taa                                             1266
   Ala Gly Thr Gly Arg
               420
<210> 20
   <211> 421
   <212> PRT
   <213> Corynebacterium glutamicum
<400> 20
   Val Ala Leu Val Val Gln Lys Tyr Gly Gly Ser Ser Leu Glu Ser Ala
   1               5                   10                  15
   Glu Arg Ile Arg Asn Val Ala Glu Arg Ile Val Ala Thr Lys Lys Ala
               20                  25                  30
   Gly Asn Asp Val Val Val Val Cys Ser Ala Met Gly Asp Thr Thr Asp
           35                  40                  45
   Glu Leu Leu Glu Leu Ala Ala Ala Val Asn Pro Val Pro Pro Ala Arg
       50                  55                  60
   Glu Met Asp Met Leu Leu Thr Ala Gly Glu Arg Ile Ser Asn Ala Leu
   65                  70                  75                  80
   Val Ala Met Ala Ile Glu Ser Leu Gly Ala Glu Ala Gln Ser Phe Thr
                   85                  90                  95
   Gly Ser Gln Ala Gly Val Leu Thr Thr Glu Arg His Gly Asn Ala Arg
               100                 105                 110
   Ile Val Asp Val Thr Pro Gly Arg Val Arg Glu Ala Leu Asp Glu Gly
           115                 120                 125
   Lys Ile Cys Ile Val Ala Gly Phe Gln Gly Val Asn Lys Glu Thr Arg
       130                 135                 140
   Asp Val Thr Thr Leu Gly Arg Gly Gly Ser Asp Thr Thr Ala Val Ala
   145                 150                 155                 160
   Leu Ala Ala Ala Leu Asn Ala Asp Val Cys Glu Ile Tyr Ser Asp Val
                   165                 170                 175
   Asp Gly Val Tyr Thr Ala Asp Pro Arg Ile Val Pro Asn Ala Gln Lys
               180                 185                 190
   Leu Glu Lys Leu Ser Phe Glu Glu Met Leu Glu Leu Ala Ala Val Gly
           195                 200                 205
   Ser Lys Ile Leu Val Leu Arg Ser Val Glu Tyr Ala Arg Ala Phe Asn
       210                 215                 220
   Val Pro Leu Arg Val Arg Ser Ser Tyr Ser Asn Asp Pro Gly Thr Leu
   225                 230                 235                 240
   Ile Ala Gly Ser Met Glu Asp Ile Pro Val Glu Glu Ala Val Leu Thr
                   245                 250                 255
   Gly Val Ala Thr Asp Lys Ser Glu Ala Lys Val Thr Val Leu Gly Ile
               260                 265                 270
   Ser Asp Lys Pro Gly Glu Ala Ala Lys Val Phe Arg Ala Leu Ala Asp
           275                 280                 285
   Ala Glu Ile Asn Ile Asp Met Val Leu Gln Asn Val Ser Ser Val Glu
       290                 295                 300
   Asp Gly Thr Thr Asp Ile Thr Phe Thr Cys Pro Arg Ser Asp Gly Arg
   305                 310                 315                 320
   Arg Ala Met Glu Ile Leu Lys Lys Leu Gln Val Gln Gly Asn Trp Thr
                   325                 330                 335
   Asn Val Leu Tyr Asp Asp Gln Val Gly Lys Val Ser Leu Val Gly Ala
               340                 345                 350
   Gly Met Lys Ser His Pro Gly Val Thr Ala Glu Phe Met Glu Ala Leu
           355                 360                 365
   Arg Asp Val Asn Val Asn Ile Glu Leu Ile Ser Thr Ser Glu Ile Arg
       370                 375                 380
   Ile Ser Val Leu Ile Arg Glu Asp Asp Leu Asp Ala Ala Ala Arg Ala
   385                 390                 395                 400
   Leu His Glu Gln Phe Gln Leu Gly Gly Glu Asp Glu Ala Val Val Tyr
                   405                 410                 415
   Ala Gly Thr Gly Arg
               420
<210> 21
   <211> 1266
   <212> DNA
   <213> Corynebacterium glutamicum
<220>
   <221> CDS
   <222> (1)..(1266)
   <223> lysC Kodierregion
<220>
   <221> mutation
   <222> (932)..(932)
   <223> Mutation T311I; Nukleobase Cytosin (c) zu Thymin (t)
<400> 21
   gtg gcc ctg gtc gta cag aaa tat ggc ggt tcc tcg ctt gag agt gcg        48
   Val Ala Leu Val Val Gln Lys Tyr Gly Gly Ser Ser Leu Glu Ser Ala
   1               5                   10                  15
   gaa cgc att aga aac gtc gct gaa cgg atc gtt gcc acc aag aag gct        96
   Glu Arg Ile Arg Asn Val Ala Glu Arg Ile Val Ala Thr Lys Lys Ala
               20                  25                  30
   gga aat gat gtc gtg gtt gtc tgc tcc gca atg gga gac acc acg gat       144
   Gly Asn Asp Val Val Val Val Cys Ser Ala Met Gly Asp Thr Thr Asp
           35                  40                  45
   gaa ctt cta gaa ctt gca gcg gca gtg aat ccc gtt ccg cca gct cgt       192
   Glu Leu Leu Glu Leu Ala Ala Ala Val Asn Pro Val Pro Pro Ala Arg
       50                  55                  60
   gaa atg gat atg ctc ctg act gct ggt gag cgt att tct aac gct ctc       240
   Glu Met Asp Met Leu Leu Thr Ala Gly Glu Arg Ile Ser Asn Ala Leu
   65                  70                  75                  80
   gtc gcc atg gct att gag tcc ctt ggc gca gaa gcc caa tct ttc acg       288
   Val Ala Met Ala Ile Glu Ser Leu Gly Ala Glu Ala Gln Ser Phe Thr
                   85                  90                  95
   ggc tct cag gct ggt gtg ctc acc acc gag cgc cac gga aac gca cgc       336
   Gly Ser Gln Ala Gly Val Leu Thr Thr Glu Arg His Gly Asn Ala Arg
               100                 105                 110
   att gtt gat gtc act cca ggt cgt gtg cgt gaa gca ctc gat gag ggc       384
   Ile Val Asp Val Thr Pro Gly Arg Val Arg Glu Ala Leu Asp Glu Gly
           115                 120                 125
   aag atc tgc att gtt gct ggt ttc cag ggt gtt aat aaa gaa acc cgc       432
   Lys Ile Cys Ile Val Ala Gly Phe Gln Gly Val Asn Lys Glu Thr Arg
       130                 135                 140
   gat gtc acc acg ttg ggt cgt ggt ggt tct gac acc act gca gtt gcg       480
   Asp Val Thr Thr Leu Gly Arg Gly Gly Ser Asp Thr Thr Ala Val Ala
   145                 150                 155                 160
   ttg gca gct gct ttg aac gct gat gtg tgt gag att tac tcg gac gtt       528
   Leu Ala Ala Ala Leu Asn Ala Asp Val Cys Glu Ile Tyr Ser Asp Val
                   165                 170                 175
   gac ggt gtg tat acc gct gac ccg cgc atc gtt cct aat gca cag aag       576
   Asp Gly Val Tyr Thr Ala Asp Pro Arg Ile Val Pro Asn Ala Gln Lys
               180                 185                 190
   ctg gaa aag ctc agc ttc gaa gaa atg ctg gaa ctt gct gct gtt ggc       624
   Leu Glu Lys Leu Ser Phe Glu Glu Met Leu Glu Leu Ala Ala Val Gly
           195                 200                 205
   tcc aag att ttg gtg ctg cgc agt gtt gaa tac gct cgt gca ttc aat        672
   Ser Lys Ile Leu Val Leu Arg Ser Val Glu Tyr Ala Arg Ala Phe Asn
       210                 215                 220
   gtg cca ctt cgc gta cgc tcg tct tat agt aat gat ccc ggc act ttg        720
   Val Pro Leu Arg Val Arg Ser Ser Tyr Ser Asn Asp Pro Gly Thr Leu
   225                 230                 235                 240
   att gcc ggc tct atg gag gat att cct gtg gaa gaa gca gtc ctt acc        768
   Ile Ala Gly Ser Met Glu Asp Ile Pro Val Glu Glu Ala Val Leu Thr
                   245                 250                 255
   ggt gtc gca acc gac aag tcc gaa gcc aaa gta acc gtt ctg ggt att        816
   Gly Val Ala Thr Asp Lys Ser Glu Ala Lys Val Thr Val Leu Gly Ile
               260                 265                 270
   tcc gat aag cca ggc gag gct gcg aag gtt ttc cgt gcg ttg gct gat        864
   Ser Asp Lys Pro Gly Glu Ala Ala Lys Val Phe Arg Ala Leu Ala Asp
           275                 280                 285
   gca gaa atc aac att gac atg gtt ctg cag aac gtc tct tct gta gaa        912
   Ala Glu Ile Asn Ile Asp Met Val Leu Gln Asn Val Ser Ser Val Glu
       290                 295                 300
   gac ggc acc acc gac atc atc ttc acc tgc cct cgt tcc gac ggc cgc        960
   Asp Gly Thr Thr Asp Ile Ile Phe Thr Cys Pro Arg Ser Asp Gly Arg
   305                 310                 315                 320
   cgc gcg atg gag atc ttg aag aag ctt cag gtt cag ggc aac tgg acc       1008
   Arg Ala Met Glu Ile Leu Lys Lys Leu Gln Val Gln Gly Asn Trp Thr
                   325                 330                 335
   aat gtg ctt tac gac gac cag gtc ggc aaa gtc tcc ctc gtg ggt gct       1056
   Asn Val Leu Tyr Asp Asp Gln Val Gly Lys Val Ser Leu Val Gly Ala
               340                 345                 350
   ggc atg aag tct cac cca ggt gtt acc gca gag ttc atg gaa gct ctg       1104
   Gly Met Lys Ser His Pro Gly Val Thr Ala Glu Phe Met Glu Ala Leu
           355                 360                 365
   cgc gat gtc aac gtg aac atc gaa ttg att tcc acc tct gag att cgt       1152
   Arg Asp Val Asn Val Asn Ile Glu Leu Ile Ser Thr Ser Glu Ile Arg
       370                 375                 380
   att tcc gtg ctg atc cgt gaa gat gat ctg gat gct gct gca cgt gca       1200
   Ile Ser Val Leu Ile Arg Glu Asp Asp Leu Asp Ala Ala Ala Arg Ala
   385                 390                 395                 400
   ttg cat gag cag ttc cag ctg ggc ggc gaa gac gaa gcc gtc gtt tat       1248
   Leu His Glu Gln Phe Gln Leu Gly Gly Glu Asp Glu Ala Val Val Tyr
                   405                 410                 415
   gca ggc acc gga cgc taa                                               1266
   Ala Gly Thr Gly Arg
               420
<210> 22
   <211> 421
   <212> PRT
   <213> Corynebacterium glutamicum
<400> 22
   Val Ala Leu Val Val Gln Lys Tyr Gly Gly Ser Ser Leu Glu Ser Ala
   1               5                   10                  15
   Glu Arg Ile Arg Asn Val Ala Glu Arg Ile Val Ala Thr Lys Lys Ala
               20                  25                  30
   Gly Asn Asp Val Val Val Val Cys Ser Ala Met Gly Asp Thr Thr Asp
           35                  40                  45
   Glu Leu Leu Glu Leu Ala Ala Ala Val Asn Pro Val Pro Pro Ala Arg
       50                  55                  60
   Glu Met Asp Met Leu Leu Thr Ala Gly Glu Arg Ile Ser Asn Ala Leu
   65                  70                  75                  80
   Val Ala Met Ala Ile Glu Ser Leu Gly Ala Glu Ala Gln Ser Phe Thr
                   85                  90                  95
   Gly Ser Gln Ala Gly Val Leu Thr Thr Glu Arg His Gly Asn Ala Arg
               100                 105                 110
   Ile Val Asp Val Thr Pro Gly Arg Val Arg Glu Ala Leu Asp Glu Gly
           115                 120                 125
   Lys Ile Cys Ile Val Ala Gly Phe Gln Gly Val Asn Lys Glu Thr Arg
       130                 135                 140
   Asp Val Thr Thr Leu Gly Arg Gly Gly Ser Asp Thr Thr Ala Val Ala
   145                 150                 155                 160
   Leu Ala Ala Ala Leu Asn Ala Asp Val Cys Glu Ile Tyr Ser Asp Val
                   165                 170                 175
   Asp Gly Val Tyr Thr Ala Asp Pro Arg Ile Val Pro Asn Ala Gln Lys
               180                 185                 190
   Leu Glu Lys Leu Ser Phe Glu Glu Met Leu Glu Leu Ala Ala Val Gly
           195                 200                 205
   Ser Lys Ile Leu Val Leu Arg Ser Val Glu Tyr Ala Arg Ala Phe Asn
       210                 215                 220
   Val Pro Leu Arg Val Arg Ser Ser Tyr Ser Asn Asp Pro Gly Thr Leu
   225                 230                 235                 240
   Ile Ala Gly Ser Met Glu Asp Ile Pro Val Glu Glu Ala Val Leu Thr
                   245                 250                 255
   Gly Val Ala Thr Asp Lys Ser Glu Ala Lys Val Thr Val Leu Gly Ile
               260                 265                 270
   Ser Asp Lys Pro Gly Glu Ala Ala Lys Val Phe Arg Ala Leu Ala Asp
           275                 280                 285
   Ala Glu Ile Asn Ile Asp Met Val Leu Gln Asn Val Ser Ser Val Glu
       290                 295                 300
   Asp Gly Thr Thr Asp Ile Ile Phe Thr Cys Pro Arg Ser Asp Gly Arg
   305                 310                 315                 320
   Arg Ala Met Glu Ile Leu Lys Lys Leu Gln Val Gln Gly Asn Trp Thr
                   325                 330                 335
   Asn Val Leu Tyr Asp Asp Gln Val Gly Lys Val Ser Leu Val Gly Ala
               340                 345                 350
   Gly Met Lys Ser His Pro Gly Val Thr Ala Glu Phe Met Glu Ala Leu
           355                 360                 365
   Arg Asp Val Asn Val Asn Ile Glu Leu Ile Ser Thr Ser Glu Ile Arg
       370                 375                 380
   Ile Ser Val Leu Ile Arg Glu Asp Asp Leu Asp Ala Ala Ala Arg Ala
   385                 390                 395                 400
   Leu His Glu Gln Phe Gln Leu Gly Gly Glu Asp Glu Ala Val Val Tyr
                   405                 410                 415
   Ala Gly Thr Gly Arg
               420
<210> 23
   <211> 2324
   <212> DNA
   <213> Corynebacterium glutamicum
<220>
   <221> gene
   <222> (1)..(1266)
   <223> lysC Kodierregion
<220>
   <221> mutation
   <222> (932)..(932)
   <223> T311I, c zu t
<220>
   <221> gene
   <222> (1290)..(2324)
   <223> asd Kodierregion
<400> 23
   gtggccctgg tcgtacagaa atatggcggt tcctcgcttg agagtgcgga acgcattaga      60
   aacgtcgctg aacggatcgt tgccaccaag aaggctggaa atgatgtcgt ggttgtctgc     120
   tccgcaatgg gagacaccac ggatgaactt ctagaacttg cagcggcagt gaatcccgtt     180
   ccgccagctc gtgaaatgga tatgctcctg actgctggtg agcgtatttc taacgctctc     240
   gtcgccatgg ctattgagtc ccttggcgca gaagcccaat ctttcacggg ctctcaggct     300
   ggtgtgctca ccaccgagcg ccacggaaac gcacgcattg ttgatgtcac tccaggtcgt     360
   gtgcgtgaag cactcgatga gggcaagatc tgcattgttg ctggtttcca gggtgttaat     420
   aaagaaaccc gcgatgtcac cacgttgggt cgtggtggtt ctgacaccac tgcagttgcg     480
   ttggcagctg ctttgaacgc tgatgtgtgt gagatttact cggacgttga cggtgtgtat     540
   accgctgacc cgcgcatcgt tcctaatgca cagaagctgg aaaagctcag cttcgaagaa     600
   atgctggaac ttgctgctgt tggctccaag attttggtgc tgcgcagtgt tgaatacgct     660
   cgtgcattca atgtgccact tcgcgtacgc tcgtcttata gtaatgatcc cggcactttg     720
   attgccggct ctatggagga tattcctgtg gaagaagcag tccttaccgg tgtcgcaacc     780
   gacaagtccg aagccaaagt aaccgttctg ggtatttccg ataagccagg cgaggctgcg     840
   aaggttttcc gtgcgttggc tgatgcagaa atcaacattg acatggttct gcagaacgtc     900
   tcttctgtag aagacggcac caccgacatc atcttcacct gccctcgttc cgacggccgc     960
   cgcgcgatgg agatcttgaa gaagcttcag gttcagggca actggaccaa tgtgctttac    1020
   gacgaccagg tcggcaaagt ctccctcgtg ggtgctggca tgaagtctca cccaggtgtt    1080
   accgcagagt tcatggaagc tctgcgcgat gtcaacgtga acatcgaatt gatttccacc    1140
   tctgagattc gtatttccgt gctgatccgt gaagatgatc tggatgctgc tgcacgtgca    1200
   ttgcatgagc agttccagct gggcggcgaa gacgaagccg tcgtttatgc aggcaccgga    1260
   cgctaaagtt ttaaaggagt agttttacaa tgaccaccat cgcagttgtt ggtgcaaccg    1320
   gccaggtcgg ccaggttatg cgcacccttt tggaagagcg caatttccca gctgacactg    1380
   ttcgtttctt tgcttcccca cgttccgcag gccgtaagat tgaattccgt ggcacggaaa    1440
   tcgaggtaga agacattact caggcaaccg aggagtccct caaggacatc gacgttgcgt    1500
   tgttctccgc tggaggcacc gcttccaagc agtacgctcc actgttcgct gctgcaggcg    1560
   cgactgttgt ggataactct tctgcttggc gcaaggacga cgaggttcca ctaatcgtct    1620
   ctgaggtgaa cccttccgac aaggattccc tggtcaaggg cattattgcg aaccctaact    1680
   gcaccaccat ggctgcgatg ccagtgctga agccacttca cgatgccgct ggtcttgtaa 1740
   agcttcacgt ttcctcttac caggctgttt ccggttctgg tcttgcaggt gtggaaacct 1800
   tggcaaagca ggttgctgca gttggagacc acaacgttga gttcgtccat gatggacagg 1860
   ctgctgacgc aggcgatgtc ggaccttatg tttcaccaat cgcttacaac gtgctgccat 1920
   tcgccggaaa cctcgtcgat gacggcacct tcgaaaccga tgaagagcag aagctgcgca 1980
   acgaatcccg caagattctc ggtctcccag acctcaaggt ctcaggcacc tgcgtccgcg 2040
   tgccggtttt caccggccac acgctgacca ttcacgccga attcgacaag gcaatcaccg 2100
   tggaccaggc gcaggagatc ttgggtgccg cttcaggcgt caagcttgtc gacgtcccaa 2160
   ccccacttgc agctgccggc attgacgaat ccctcgttgg acgcatccgt caggactcca 2220
   ctgtcgacga taaccgcggt ctggttctcg tcgtatctgg cgacaacctc cgcaagggtg 2280
   ctgcgctaaa caccatccag atcgctgagc tgctggttaa gtaa 2324
<210> 24
   <211> 1266
   <212> DNA
   <213> Corynebacterium glutamicum
<220>
   <221> mutation
   <222> (1)..(1)
   <223> Nukleobase Guanin (g) zu Adenin (a)
<220>
   <221> gene
   <222> (1)..(1266)
   <223> lysC Kodierregion
<220>
   <221> mutation
   <222> (932)..(932)
   <223> Mutation T311I; Nukleobase Cytosin (c) zu Thymin (t)
<220>
   <221> mutation
   <222> (1265)..(1265)
   <223> Nukleobase Adenin (a) zu Guanin (g)
<400> 24
   atggccctgg tcgtacagaa atatggcggt tcctcgcttg agagtgcgga acgcattaga      60
   aacgtcgctg aacggatcgt tgccaccaag aaggctggaa atgatgtcgt ggttgtctgc     120
   tccgcaatgg gagacaccac ggatgaactt ctagaacttg cagcggcagt gaatcccgtt     180
   ccgccagctc gtgaaatgga tatgctcctg actgctggtg agcgtatttc taacgctctc     240
   gtcgccatgg ctattgagtc ccttggcgca gaagcccaat ctttcacggg ctctcaggct     300
   ggtgtgctca ccaccgagcg ccacggaaac gcacgcattg ttgatgtcac tccaggtcgt     360
   gtgcgtgaag cactcgatga gggcaagatc tgcattgttg ctggtttcca gggtgttaat     420
   aaagaaaccc gcgatgtcac cacgttgggt cgtggtggtt ctgacaccac tgcagttgcg     480
   ttggcagctg ctttgaacgc tgatgtgtgt gagatttact cggacgttga cggtgtgtat     540
   accgctgacc cgcgcatcgt tcctaatgca cagaagctgg aaaagctcag cttcgaagaa     600
   atgctggaac ttgctgctgt tggctccaag attttggtgc tgcgcagtgt tgaatacgct     660
   cgtgcattca atgtgccact tcgcgtacgc tcgtcttata gtaatgatcc cggcactttg     720
   attgccggct ctatggagga tattcctgtg gaagaagcag tccttaccgg tgtcgcaacc     780
   gacaagtccg aagccaaagt aaccgttctg ggtatttccg ataagccagg cgaggctgcg     840
   aaggttttcc gtgcgttggc tgatgcagaa atcaacattg acatggttct gcagaacgtc     900
   tcttctgtag aagacggcac caccgacatc atcttcacct gccctcgttc cgacggccgc     960
   cgcgcgatgg agatcttgaa gaagcttcag gttcagggca actggaccaa tgtgctttac    1020
   gacgaccagg tcggcaaagt ctccctcgtg ggtgctggca tgaagtctca cccaggtgtt    1080
   accgcagagt tcatggaagc tctgcgcgat gtcaacgtga acatcgaatt gatttccacc    1140
   tctgagattc gtatttccgt gctgatccgt gaagatgatc tggatgctgc tgcacgtgca    1200
   ttgcatgagc agttccagct gggcggcgaa gacgaagccg tcgtttatgc aggcaccgga    1260
   cgctga                                                               1266
<210> 25
   <211> 2808
   <212> DNA
   <213> Corynebacterium glutamicum
<220>
   <221> misc_feature
   <222> (1)..(6)
   <223> Bereich der NsiI-Schnittstelle
<220>
   <221> promoter
   <222> (7)..(467)
   <223> Pgap3 Promotor wie SEQ ID Nr. 3
<220>
   <221> misc_feature
   <222> (468)..(488)
   <223> gap RBS wie SEQ ID Nr. 16
<220>
   <221> gene
   <222> (489)..(1754)
   <223> lysC Kodierregion mit Mutation T311I, atg Start und tga Stopp wie SEQ ID Nr. 24
<220>
   <221> misc_feature
   <222> (1755)..(1773)
   <223> RBS gap wie SEQ ID Nr. 26
<220>
   <221> gene
   <222> (1774)..(2808)
   <223> asd Kodierregion
<400> 25
   atgcattgaa gcctaaaaac gaccgagcct attgggatta ccattgaagc cagtgtgagt      60
   tgcatcacat tggcttcaaa tctgagactt taatttgtgg attcacgggg gtgtaatgta     120
   gttcataatt aaccccattc gggggagcag atcgtagtgc gaacgatttc aggttcgttc     180
   cctgcaaaaa ctatttagcg caagtgttgg aaatgccccc gtttggggtc aatgtccatt     240
   tttgaatgtg tctgtatgat tttgcatctg ctgcgaaatc tttgtttccc cgctaaagtt     300
   gaggacaggt tgacacggag ttgactcgac gaattatcca atgtgagtag gtttggtgcg     360
   tgagttgaaa aaattcgcca tactcgccct tgggttctgt cagctcaaga attcttgagt     420
   gaccgatgct ctgattgacc taactgcttg acacattgca tttcctacaa tcggagagga     480
   gacacaacat ggccctggtc gtacagaaat atggcggttc ctcgcttgag agtgcggaac     540
   gcattagaaa cgtcgctgaa cggatcgttg ccaccaagaa ggctggaaat gatgtcgtgg     600
   ttgtctgctc cgcaatggga gacaccacgg atgaacttct ggaacttgca gcggcagtga     660
   atcccgttcc gccagctcgt gaaatggata tgctcctgac tgctggtgag cgtatttcta     720
   acgctctcgt cgccatggct attgagtccc ttggcgcaga agcccaatct ttcacgggct     780
   ctcaggctgg tgtgctcacc accgagcgcc acggaaacgc acgcattgtt gatgtcactc     840
   caggtcgtgt gcgtgaagca ctcgatgagg gcaagatctg cattgttgct ggtttccagg     900
   gtgttaataa agaaacccgc gatgtcacca cgttgggtcg tggtggttct gacaccactg     960
   cagttgcgtt ggcagctgct ttgaacgctg atgtgtgtga gatttactcg gacgttgacg    1020
   gtgtgtatac cgctgacccg cgcatcgttc ctaatgcaca gaagctggaa aagctcagct    1080
   tcgaagaaat gctggaactt gctgctgttg gctccaagat tttggtgctg cgcagtgttg    1140
   aatacgctcg tgcattcaat gtgccacttc gcgtacgctc gtcttatagt aatgatcccg    1200
   gcactttgat tgccggctct atggaggata ttcctgtgga agaagcagtc cttaccggtg    1260
   tcgcaaccga caagtccgaa gccaaagtaa ccgttctggg tatttccgat aagccaggcg    1320
   aggctgcgaa ggttttccgt gcgttggctg atgcagaaat caacattgac atggttctgc    1380
   agaacgtctc ttctgtagaa gacggcacca ccgacatcat cttcacctgc cctcgttccg    1440
   acggccgccg cgcgatggag atcttgaaga agcttcaggt tcagggcaac tggaccaatg    1500
   tgctttacga cgaccaggtc ggcaaagtct ccctcgtggg tgctggcatg aagtctcacc    1560
   caggtgttac cgcagagttc atggaagctc tgcgcgatgt caacgtgaac atcgaattga    1620
   tttccacctc tgagattcgt atttccgtgc tgatccgtga agatgatctg gatgctgctg    1680
   cacgtgcatt gcatgagcag ttccagctgg gcggcgaaga cgaagccgtc gtttatgcag 1740
   gcaccggacg ctgacgtcta gaggagacac aacatgacca ccatcgcagt tgttggtgca 1800
   accggccagg tcggccaggt tatgcgcacc cttttggaag agcgcaattt cccagctgac 1860
   actgttcgtt tctttgcttc cccacgttcc gcaggccgta agattgaatt ccgtggcacg 1920
   gaaatcgagg tagaagacat tactcaggca accgaggagt ccctcaagga catcgacgtt 1980
   gcgttgttct ccgctggagg caccgcttcc aagcagtacg ctccactgtt cgctgctgca 2040
   ggcgcgactg ttgtggataa ctcttctgct tggcgcaagg acgacgaggt tccactaatc 2100
   gtctctgagg tgaacccttc cgacaaggat tccctggtca agggcattat tgcgaaccct 2160
   aactgcacca ccatggctgc gatgccagtg ctgaagccac ttcacgatgc cgctggtctt 2220
   gtaaagcttc acgtttcctc ttaccaggct gtttccggtt ctggtcttgc aggtgtggaa 2280
   accttggcaa agcaggttgc tgcagttgga gaccacaacg ttgagttcgt ccatgatgga 2340
   caggctgctg acgcaggcga tgtcggacct tatgtttcac caatcgctta caacgtgctg 2400
   ccattcgccg gaaacctcgt cgatgacggc accttcgaaa ccgatgaaga gcagaagctg 2460
   cgcaacgaat cccgcaagat tctcggtctc ccagacctca aggtctcagg cacctgcgtc 2520
   cgcgtgccgg ttttcaccgg ccacacgctg accattcacg ccgaattcga caaggcaatc 2580
   accgtggacc aggcgcagga gatcttgggt gccgcttcag gcgtcaagct tgtcgatgtc 2640
   ccaaccccac ttgcagctgc cggcattgac gaatccctcg ttggacgcat ccgtcaggac 2700
   tccactgtcg acgataaccg cggtctggtt ctcgtcgtat ctggcgacaa cctccgcaag 2760
   ggtgctgcgc taaacaccat ccagatcgct gagctgctgg ttaagtaa 2808
<210> 26
   <211> 22
   <212> DNA
   <213> Corynebacterium glutamicum
<220>
   <221> misc_feature
   <222> (2)..(7)
   <223> AatII-Schnittstelle
<220>
   <221> misc_feature
   <222> (6)..(11)
   <223> XbaI-Schnittstelle
<220>
   <221> RBS
   <222> (11)..(16)
   <223> Ribosomenbindestelle des Gens gap
<400> 26
   tgacgtctag aggagacaca ac 22
<210> 27
   <211> 4496
   <212> DNA
   <213> Corynebacterium glutamicum
<220>
   <221> misc_feature
   <222> (1)..(207)
   <223> Teil der Kodierregion für eine Serin/Threonin-Proteinkinase (cg0057)
<220>
   <221> gene
   <222> (322)..(594)
   <223> Kodierregion für ein putatives Protein (septation inhibitor protein, cg0055)
<220>
   <221> misc_feature
   <222> (812)..(817)
   <223> Bereich der NsiI-Schnittstelle
<220>
   <221> promoter
   <222> (818)..(1278)
   <223> Pgap3 Promotor wie SEQ ID Nr.3
<220>
   <221> misc_feature
   <222> (1279)..(1299)
   <223> gap RBS wie SEQ ID Nr. 16
<220>
   <221> gene
   <222> (1300)..(2565)
   <223> lysC Kodierregion mit Mutation T311I, atg Start und tga Stopp wie SEQ ID Nr. 24
<220>
   <221> misc_feature
   <222> (2566)..(2584)
   <223> RBS gap wie SEQ ID Nr. 26
<220>
   <221> gene
   <222> (2585)..(3619)
   <223> asd Kodierregion
<220>
   <221> terminator
   <222> (3623)..(3667)
   <223> gap Terminator
<220>
   <221> misc_feature
   <222> (3674)..(3679)
   <223> Bereich der AvrII-Schnittstelle
<220>
   <221> misc_feature
   <222> (3834)..(4496)
   <223> Teil der Kodierregion für ein iron-chelator utilization protein (cg0054)
<400> 27
   accgaacagg ccatcagtgc cctccgcgct gctggctgga ccgccccaga tcaatccctg      60
   atcgtcggcg accccatcca caccgcagcc ctcgtggatc aaaacaaaat cggattccaa     120
   tccccaaccc ctgcaaccct cttccgcaaa gacgcccaag tgcaagtgcg actcttcgaa     180
   ttcgatctcg ctgcactcgt gcaatagcca acaaggaaac cgtcaaggta gctggcccgg     240
   caactgatac gttaagctca aacaagataa gtaccagttg ctggggtttt tccaagacaa     300
   taaattatga aggtgtgaac aatgccaaag gcaagagtaa ctaaaaacga gaccgcaccg     360
   gtttcaagca acccaagcgc aaaccgcacc ccggttaaga tcaattccgc cggaacccca     420
   atgtggtaca aggtcatcat gtttgccttc atgatcgtcg gcctagcctg gttgatcatt     480
   aactacctcg tgggcccaca gatcccattc atggctgatc ttggtgcatg gaactatggc     540
   atcggcttcg gtctgatgat catcggccta ctcatgacca tgggttggcg ttaatccttc     600
   aaaaaagtga ctgccgcagc acagcgttga ttcgttgtgc tgcggcagtt gttttgtgcg     660
   ggcggatcca gatttagccc ctcatgggca ctctcgtttg gcagttcggg gcactcaaaa     720
   aggtaaaacc agactagcgt gcccaagaac tgaacttttt gccagccgtg ggcactcctg     780
   ttcggtttga gagccgagaa accagacagg catgcattga agcctaaaaa cgaccgagcc     840
   tattgggatt accattgaag ccagtgtgag ttgcatcaca ttggcttcaa atctgagact     900
   ttaatttgtg gattcacggg ggtgtaatgt agttcataat taaccccatt cgggggagca     960
   gatcgtagtg cgaacgattt caggttcgtt ccctgcaaaa actatttagc gcaagtgttg    1020
   gaaatgcccc cgtttggggt caatgtccat ttttgaatgt gtctgtatga ttttgcatct    1080
   gctgcgaaat ctttgtttcc ccgctaaagt tgaggacagg ttgacacgga gttgactcga    1140
   cgaattatcc aatgtgagta ggtttggtgc gtgagttgaa aaaattcgcc atactcgccc    1200
   ttgggttctg tcagctcaag aattcttgag tgaccgatgc tctgattgac ctaactgctt    1260
   gacacattgc atttcctaca atcggagagg agacacaaca tggccctggt cgtacagaaa    1320
   tatggcggtt cctcgcttga gagtgcggaa cgcattagaa acgtcgctga acggatcgtt    1380
   gccaccaaga aggctggaaa tgatgtcgtg gttgtctgct ccgcaatggg agacaccacg    1440
   gatgaacttc tggaacttgc agcggcagtg aatcccgttc cgccagctcg tgaaatggat    1500
   atgctcctga ctgctggtga gcgtatttct aacgctctcg tcgccatggc tattgagtcc    1560
   cttggcgcag aagcccaatc tttcacgggc tctcaggctg gtgtgctcac caccgagcgc    1620
   cacggaaacg cacgcattgt tgatgtcact ccaggtcgtg tgcgtgaagc actcgatgag    1680
   ggcaagatct gcattgttgc tggtttccag ggtgttaata aagaaacccg cgatgtcacc    1740
   acgttgggtc gtggtggttc tgacaccact gcagttgcgt tggcagctgc tttgaacgct 1800
   gatgtgtgtg agatttactc ggacgttgac ggtgtgtata ccgctgaccc gcgcatcgtt 1860
   cctaatgcac agaagctgga aaagctcagc ttcgaagaaa tgctggaact tgctgctgtt 1920
   ggctccaaga ttttggtgct gcgcagtgtt gaatacgctc gtgcattcaa tgtgccactt 1980
   cgcgtacgct cgtcttatag taatgatccc ggcactttga ttgccggctc tatggaggat 2040
   attcctgtgg aagaagcagt ccttaccggt gtcgcaaccg acaagtccga agccaaagta 2100
   accgttctgg gtatttccga taagccaggc gaggctgcga aggttttccg tgcgttggct 2160
   gatgcagaaa tcaacattga catggttctg cagaacgtct cttctgtaga agacggcacc 2220
   accgacatca tcttcacctg ccctcgttcc gacggccgcc gcgcgatgga gatcttgaag 2280
   aagcttcagg ttcagggcaa ctggaccaat gtgctttacg acgaccaggt cggcaaagtc 2340
   tccctcgtgg gtgctggcat gaagtctcac ccaggtgtta ccgcagagtt catggaagct 2400
   ctgcgcgatg tcaacgtgaa catcgaattg atttccacct ctgagattcg tatttccgtg 2460
   ctgatccgtg aagatgatct ggatgctgct gcacgtgcat tgcatgagca gttccagctg 2520
   ggcggcgaag acgaagccgt cgtttatgca ggcaccggac gctgacgtct agaggagaca 2580
   caacatgacc accatcgcag ttgttggtgc aaccggccag gtcggccagg ttatgcgcac 2640
   ccttttggaa gagcgcaatt tcccagctga cactgttcgt ttctttgctt ccccacgttc 2700
   cgcaggccgt aagattgaat tccgtggcac ggaaatcgag gtagaagaca ttactcaggc 2760
   aaccgaggag tccctcaagg acatcgacgt tgcgttgttc tccgctggag gcaccgcttc 2820
   caagcagtac gctccactgt tcgctgctgc aggcgcgact gttgtggata actcttctgc 2880
   ttggcgcaag gacgacgagg ttccactaat cgtctctgag gtgaaccctt ccgacaagga 2940
   ttccctggtc aagggcatta ttgcgaaccc taactgcacc accatggctg cgatgccagt 3000
   gctgaagcca cttcacgatg ccgctggtct tgtaaagctt cacgtttcct cttaccaggc 3060
   tgtttccggt tctggtcttg caggtgtgga aaccttggca aagcaggttg ctgcagttgg 3120
   agaccacaac gttgagttcg tccatgatgg acaggctgct gacgcaggcg atgtcggacc 3180
   ttatgtttca ccaatcgctt acaacgtgct gccattcgcc ggaaacctcg tcgatgacgg 3240
   caccttcgaa accgatgaag agcagaagct gcgcaacgaa tcccgcaaga ttctcggtct 3300
   cccagacctc aaggtctcag gcacctgcgt ccgcgtgccg gttttcaccg gccacacgct 3360
   gaccattcac gccgaattcg acaaggcaat caccgtggac caggcgcagg agatcttggg 3420
   tgccgcttca ggcgtcaagc ttgtcgatgt cccaacccca cttgcagctg ccggcattga 3480
   cgaatccctc gttggacgca tccgtcagga ctccactgtc gacgataacc gcggtctggt 3540
   tctcgtcgta tctggcgaca acctccgcaa gggtgctgcg ctaaacacca tccagatcgc 3600
   tgagctgctg gttaagtaat attcccaata gcccggggtg tgcctcggcg caccccgggc 3660
   tattttgata tcacctagga ctgaagacac gacatttggg cacgccagtc tggttccccc 3720
   aaaatcagtc cacattcagc tccgaatccc aaaaatcatg ccctcccaga atcgcttcta 3780
   agagctcatc agacgccaat caatgcaaac acccattcta aaaactcgac cccttaaatc 3840
   tccaatgaga ggtcgtcgaa gttttccagc tgtagccctt cagagagcat cttcatgtag 3900
   ttttctttgc ggactgcatc gatctgagtg ttggtggtgt tgtatttcca gtacccaaac 3960
   acggccactg catctttttc caatcccacc tgcttgcgga agtgcctgcg gatggatttt 4020
   atttcatctc gctcgccggc tgcccacacc acgtaagttt ctgggttttc caggtcatat 4080
   gcctgttggg caagtggctg aacagttgga tctgatcccg gcgcgagcag gttcagttcc 4140
   agtccgtcga tgctgggtag ttcatcgaag gctgcgggat cgtcagtgac aacccatcct 4200
   ttgccacgaa gatctgttgg ccattgatcc aaaatagcgg cgagagcggg gatggcggtg 4260
   tcatcaagga agagtgctgc ttgttcgcct tcggggatga caaagtgtgg gcggggtccg 4320
   gtgagcacga gggtgtcgtt gatttttacg gtgtctgacc agcgcatcat ggggctttcg 4380
   tggtggtgtt gaaccacatc caccacgatg ctttcagttg cggcatcaaa ggatcggacg 4440
   gtgtagacgc gggtggcgtc gtcgaattct tcgctgaggt aaagcctgat tgccac 4496
<210> 28
   <211> 2531
   <212> DNA
   <213> Corynebacterium glutamicum
<220>
   <221> misc_feature
   <222> (1)..(1013)
   <223> Upstream-Bereich der hom Kodierregion
<220>
   <221> misc_feature
   <222> (1012)..(1017)
   <223> Bereich der NsiI-Schnittstelle
<220>
   <221> promoter
   <222> (1018)..(1478)
   <223> Pgap3 Promotor wie SEQ ID Nr.3
<220>
   <221> misc_feature
   <222> (1479)..(1500)
   <223> gap RBS wie SEQ ID Nr. 17
<220>
   <221> misc_feature
   <222> (1482)..(1487)
   <223> Bereich der NruI-Schnittstelle
<220>
   <221> gene
   <222> (1501)..(2531)
   <223> hom Kodierregion
<400> 28
   ctgcagtcga ggcttcagag gttttattgc tcgacttcac gtgtgctgac ttcgcgtgct 60
   caacttctca tgaggcgaca tcagtcgaaa agccggtcat tgtcgtatga agtctgcgca 120
   cgagaagttg agcggaagaa gtcggtgtta agaagtcgac cgcgtgaagt cgccctttag 180
   gagaattctg actaactgga gccaaaactt gatccactcg agagctgtgc agtctctttt 240
   tccttcaatt ctgcctgctc gagctcgtag aagtagaggt ctacttcagt tggttcacct 300
   tgcacacaag catgaagtag tgggtaggtc gagttgttaa atgcggtgta gaaggggagt 360
   agttcgctag caaaggttaa tttggagtcg ctgtactgcg ggttctcggg tggagtattc 420
   ccggaggatt caagaaatct tgacgcatct ttgatgaggt atgtttggaa ttcgtcggca 480
   ccttcctcgc cggagaggta gtaggagttg tcgtaatttg gaacccagat ggcaaatcgt 540
   gcgttttcga ttgcgtccag gagttcctct acgttgtatc tcgcacttgt tgcagcggaa 600
   gcgactcggt tgccgatgtc tccgtatgca gtgagcgtgg cgtttccgag gggaacttga 660
   tcagaggaat acaccatgga gccgatgtca gaggcgactg cgggcagatc cttttgaagc 720
   tgtttcacaa tttctttgcc cagttcgcgg cggatctgga accacttttg catgcgatcg 780
   tcgtcagagt ggttcatgtg aaaaatacac tcaccatctc aatggtcatg gtgaaggcct 840
   gtactggctg cgacagcatg gaactcagtg caatggctgt aaggcctgca ccaacaatga 900
   ttgagcgaag ctccaaaatg tcctccccgg gttgatatta gatttcataa atatactaaa 960
   aatcttgaga gtttttccgt tgaaaactaa aaagctggga aggtgaatcg aatgcattga 1020
   agcctaaaaa cgaccgagcc tattgggatt accattgaag ccagtgtgag ttgcatcaca 1080
   ttggcttcaa atctgagact ttaatttgtg gattcacggg ggtgtaatgt agttcataat 1140
   taaccccatt cgggggagca gatcgtagtg cgaacgattt caggttcgtt ccctgcaaaa 1200
   actatttagc gcaagtgttg gaaatgcccc cgtttggggt caatgtccat ttttgaatgt 1260
   gtctgtatga ttttgcatct gctgcgaaat ctttgtttcc ccgctaaagt tgaggacagg 1320
   ttgacacgga gttgactcga cgaattatcc aatgtgagta ggtttggtgc gtgagttgaa 1380
   aaaattcgcc atactcgccc ttgggttctg tcagctcaag aattcttgag tgaccgatgc 1440
   tctgattgac ctaactgctt gacacattgc atttcctaca atcgcgagag gagacacaac 1500
   atgacctcag catctgcccc aagctttaac cccggcaagg gtcccggctc agcagtcgga 1560
   attgcccttt taggattcgg aacagtcggc actgaggtga tgcgtctgat gaccgagtac 1620
   ggtgatgaac ttgcgcaccg cattggtggc ccactggagg ttcgtggcat tgctgtttct 1680
   gatatctcaa agccacgtga aggcgttgca cctgagctgc tcactgagga cgcttttgca 1740
   ctcatcgagc gcgaggatgt tgacatcgtc gttgaggtta tcggcggcat tgagtaccca 1800
   cgtgaggtag ttctcgcagc tctgaaggcc ggcaagtctg ttgttaccgc caataaggct      1860
   cttgttgcag ctcactctgc tgagcttgct gatgcagcgg aagccgcaaa cgttgacctg      1920
   tacttcgagg ctgctgttgc aggcgcaatt ccagtggttg gcccactgcg tcgctccctg      1980
   gctggcgatc agatccagtc tgtgatgggc atcgttaacg gcaccaccaa cttcatcttg      2040
   gacgccatgg attccaccgg cgctgactat gcagattctt tggctgaggc aactcgtttg      2100
   ggttacgccg aagctgatcc aactgcagac gtcgaaggcc atgacgccgc atccaaggct      2160
   gcaattttgg catccatcgc tttccacacc cgtgttaccg cggatgatgt gtactgcgaa      2220
   ggtatcagca acatcagcgc tgccgacatt gaggcagcac agcaggcagg ccacaccatc      2280
   aagttgttgg ccatctgtga gaagttcacc aacaaggaag gaaagtcggc tatttctgct      2340
   cgcgtgcacc cgactctatt acctgtgtcc cacccactgg cgtcggtaaa caagtccttt      2400
   aatgcaatct ttgttgaagc agaagcagct ggtcgcctga tgttctacgg aaacggtgca      2460
   ggtggcgcgc caaccgcgtc tgctgtgctt ggcgacgtcg ttggtgccgc acgaaacaag      2520
   gtgcacggtg g                                                           2531
<210> 29
   <211> 2722
   <212> DNA
   <213> Corynebacterium glutamicum
<220>
   <221> misc_feature
   <222> (2)..(7)
   <223> Restriktionsschnittstelle SphI
<220>
   <221> promoter
   <222> (10)..(470)
   <223> Pgap3 Promotor wie SEQ ID Nr.3
<220>
   <221> gene
   <222> (529)..(1695)
   <223> argJ Kodierregion
<220>
   <221> gene
   <222> (1741)..(2694)
   <223> argB Kodierregion
<220>
   <221> gene
   <222> (2695)..(2708)
   <223> Teil der argD Kodierregion
<220>
   <221> misc_feature
   <222> (2716) .. (2721)
   <223> Restriktionsschnittstelle BlnI
<400> 29
   ggcatgcatt gaagcctaaa aacgaccgag cctattggga ttaccattga agccagtgtg      60
   agttgcatca cattggcttc aaatctgaga ctttaatttg tggattcacg ggggtgtaat      120
   gtagttcata attaacccca ttcgggggag cagatcgtag tgcgaacgat ttcaggttcg     180
   ttccctgcaa aaactattta gcgcaagtgt tggaaatgcc cccgtttggg gtcaatgtcc     240
   atttttgaat gtgtctgtat gattttgcat ctgctgcgaa atctttgttt ccccgctaaa     300
   gttgaggaca ggttgacacg gagttgactc gacgaattat ccaatgtgag taggtttggt     360
   gcgtgagttg aaaaaattcg ccatactcgc ccttgggttc tgtcagctca agaattcttg     420
   agtgaccgat gctctgattg acctaactgc ttgacacatt gcatttccta caatcgactc     480
   cctaaccagc aaacacaaca aacacatcta attcagtagg agttccacat ggcagaaaaa     540
   ggcattaccg cgccgaaagg cttcgttgct tctgcaacga ccgcgggtat taaagcttct     600
   ggcaatcctg acatggcgtt ggtggttaac cagggtccag agttttccgc agcggccgtg     660
   tttacacgta accgagtttt cgcagcgcct gtgaaggtga gccgagagaa cgttgctgat     720
   ggccagatca gggctgtttt gtacaacgct ggtaatgcta atgcgtgtaa tggtctgcag     780
   ggtgagaagg atgctcgtga gtctgtttct catctagctc aaaatttggg cttggaggat     840
   tccgatattg gtgtgtgttc cactggtctt attggtgagt tgcttccgat ggataagctc     900
   aatgcaggta ttgatcagct gaccgctgag ggcgctttgg gtgacaatgg tgcagctgct     960
   gccaaggcga tcatgaccac tgacacggtg gataaggaaa ccgtcgtgtt tgctgatggt    1020
   tggactgtcg gcggaatggg caagggcgtg ggcatgatgg cgccgtctct tgccaccatg    1080
   ctggtctgct tgaccactga tgcatccgtt actcaggaaa tggctcagat cgcgctggct    1140
   aatgctacgg ccgttacgtt tgacaccctg gatattgatg gatcaacctc caccaatgac    1200
   accgtgttcc tgctggcatc tggcgctagc ggaatcaccc caactcagga tgaactcaac    1260
   gatgcggtgt acgcagcttg ttctgatatc gcagcgaagc ttcaggctga tgcagagggt    1320
   gtgaccaagc gcgttgctgt gacagtggtg ggaaccacca acaacgagca ggcgattaat    1380
   gcggctcgca ctgttgctcg tgacaatttg ttcaagtgcg caatgtttgg atctgatcca    1440
   aactggggtc gcgtgttggc tgcagtcggc atggctgatg ctgatatgga accagagaag    1500
   atttctgtgt tcttcaatgg tcaagcagta tgccttgatt ccactggcgc tcctggtgct    1560
   cgtgaggtgg atctttccgg cgctgacatt gatgtccgaa ttgatttggg caccagtggg    1620
   gaaggccagg caacagttcg aaccactgac ctgagcttct cctacgtgga gatcaactcc    1680
   gcgtacagct cttaaaaaga aacagcactc caactaacaa gcagggaaaa gggcacaggc    1740
   atgaatgact tgatcaaaga tttaggctct gaggtgcgcg caaatgtcct cgctgaggcg    1800
   ttgccatggt tgcagcactt ccgcgacaag attgttgtcg tgaaatatgg cggaaacgcc    1860
   atggtggatg atgatctcaa ggctgctttt gctgccgaca tggtcttctt gcgcaccgtg      1920
   ggcgcaaaac cagtggtggt gcacggtggt ggacctcaga tttctgagat gctaaaccgt      1980
   gtgggtctcc agggcgagtt caagggtggt ttccgtgtga ccactcctga ggtcatggac      2040
   attgtgcgca tggtgctctt tggtcaggtc ggtcgcgatt tagttggttt gatcaactct      2100
   catggccctt acgctgtggg aacctccggt gaggatgccg gcctgtttac cgcgcagaag      2160
   cgcatggtca acatcgatgg cgtacccact gatattggtt tggtcggaga catcattaat      2220
   gtcgatgcct cttccttgat ggatatcatc gaggccggtc gcattcctgt ggtctctacg      2280
   attgctccag gcgaagacgg ccagatttac aacattaacg ccgataccgc agcaggtgct      2340
   ttggctgcag cgattggtgc agaacgcctg ctggttctca ccaatgtgga aggtctgtac      2400
   accgattggc ctgataagag ctcactggtg tccaagatca aggccaccga gctggaggcc      2460
   attcttccgg gacttgattc cggcatgatt ccaaagatgg agtcttgctt gaacgcggtg      2520
   cgtgggggag taagcgctgc tcatgtcatt gacggccgca tcgcgcactc ggtgttgctg      2580
   gagcttttga ccatgggtgg aattggcacg atggtgctgc cggatgtttt tgatcgggag      2640
   aattatcctg aaggcaccgt ttttagaaaa gacgacaagg atggggaact gtaaatgagc      2700
   acgctggaat attggcctag ga                                              2722
<210> 30
   <211> 2005
   <212> DNA
   <213> Corynebacterium glutamicum
<220>
   <221> misc_feature
   <222> (379)..(379)
   <223> Nukleobase Guanin (g)
<220>
   <221> gene
   <222> (501)..(1505)
   <223> Kodierregion gap
<400> 30
   gaagaaattt agatgattga agcctaaaaa cgaccgagcc tattgggatt accattgaag      60
   ccagtgtgag ttgcatcaca ttggcttcaa atctgagact ttaatttgtg gattcacggg     120
   ggtgtaatgt agttcataat taaccccatt cgggggagca gatcgtagtg cgaacgattt     180
   caggttcgtt ccctgcaaaa actatttagc gcaagtgttg gaaatgcccc cgtttggggt     240
   caatgtccat ttttgaatgt gtctgtatga ttttgcatct gctgcgaaat ctttgtttcc     300
   ccgctaaagt tgaggacagg ttgacacgga gttgactcga cgaattatcc aatgtgagta     360
   ggtttggtgc gtgagttgga aaaattcgcc atactcgccc ttgggttctg tcagctcaag     420
   aattcttgag tgaccgatgc tctgattgac ctaactgctt gacacattgc atttcctaca      480
   atctttagag gagacacaac atgaccattc gtgttggtat taacggattt ggccgtatcg      540
   gacgtaactt cttccgcgca gttctggagc gcagcgacga tctcgaggta gttgcagtca      600
   acgacctcac cgacaacaag accctttcca cccttctcaa gttcgactcc atcatgggcc      660
   gccttggcca ggaagttgaa tacgacgatg actccatcac cgttggtggc aagcgcatcg      720
   ctgtttacgc agagcgcgat ccaaagaacc tggactgggc tgcacacaac gttgacatcg      780
   tgatcgagtc caccggcttc ttcaccgatg caaacgcggc taaggctcac atcgaagcag      840
   gtgccaagaa ggtcatcatc tccgcaccag caagcaacga agacgcaacc ttcgtttacg      900
   gtgtgaacca cgagtcctac gatcctgaga accacaacgt gatctccggc gcatcttgca      960
   ccaccaactg cctcgcacca atggcaaagg tcctaaacga caagttcggc atcgagaacg     1020
   gcctcatgac caccgttcac gcatacactg gcgaccagcg cctgcacgat gcacctcacc     1080
   gcgacctgcg tcgtgcacgt gcagcagcag tcaacatcgt tcctacctcc accggtgcag     1140
   ctaaggctgt tgctctggtt ctcccagagc tcaagggcaa gcttgacggc tacgcacttc     1200
   gcgttccagt tatcaccggt tccgcaaccg acctgacctt caacaccaag tctgaggtca     1260
   ccgttgagtc catcaacgct gcaatcaagg aagctgcagt cggcgagttc ggcgagaccc     1320
   tggcttactc cgaagagcca ctggtttcca ccgacatcgt ccacgattcc cacggctcca     1380
   tcttcgacgc tggcctgacc aaggtctccg gcaacaccgt caaggttgtt tcctggtacg     1440
   acaacgagtg gggctacacc tgccagctcc tgcgtctgac cgagctcgta gcttccaagc     1500
   tctaattagt tcacatcgct aacgtgggcg atcgatgctc acggtgatgt gtcatcccaa     1560
   tagcccgggg tgtgcctcgg cgcaccccgg gctattttgt gtctttaatc aatacaattg     1620
   aataccggtg ccagcgccac acaatgtgtg gcaatctggg acagtgcatc acattgcacc     1680
   agaagaattt tttaaacaat caaatctcca aggagtacgg catggctgtt aagaccctca     1740
   aggacttgct cgacgaaggc gtagacggac gccacgtcat cgttcgatct gacttcaatg     1800
   ttcccctcaa cgatgaccgc gagatcaccg ataagggccg aatcattgcc tccctaccaa     1860
   cccttaaagc actgagcgaa ggtggcgcaa aggtcatcgt catggctcac cttggccgcc     1920
   caaagggcga ggtcaacgag aagtactccc tcgcacctgt cgctgaggca ctctccgatg     1980
   agcttggcca gtacgttgca cttgc                                           2005
<210> 31
   <211> 20
   <212> DNA
   <213> Corynebacterium glutamicum
<220>
   <221> misc_feature
   <222> (1)..(20)
   <223> Primer Pgap3-1
<400> 31
   cgtttggggt caatgtccat 20
<210> 32
   <211> 18
   <212> DNA
   <213> Corynebacterium glutamicum
<400> 32
   cccagtccag gttctttg 18
<210> 33
   <211> 2005
   <212> DNA
   <213> Corynebacterium glutamicum
<220>
   <221> mutation
   <222> (379)..(379)
   <223> Nukleobase Adenin (a)
<220>
   <221> gene
   <222> (501)..(1505)
   <223> Kodierregion gap
<400> 33
   gaagaaattt agatgattga agcctaaaaa cgaccgagcc tattgggatt accattgaag      60
   ccagtgtgag ttgcatcaca ttggcttcaa atctgagact ttaatttgtg gattcacggg     120
   ggtgtaatgt agttcataat taaccccatt cgggggagca gatcgtagtg cgaacgattt     180
   caggttcgtt ccctgcaaaa actatttagc gcaagtgttg gaaatgcccc cgtttggggt     240
   caatgtccat ttttgaatgt gtctgtatga ttttgcatct gctgcgaaat ctttgtttcc     300
   ccgctaaagt tgaggacagg ttgacacgga gttgactcga cgaattatcc aatgtgagta     360
   ggtttggtgc gtgagttgaa aaaattcgcc atactcgccc ttgggttctg tcagctcaag     420
   aattcttgag tgaccgatgc tctgattgac ctaactgctt gacacattgc atttcctaca     480
   atctttagag gagacacaac atgaccattc gtgttggtat taacggattt ggccgtatcg     540
   gacgtaactt cttccgcgca gttctggagc gcagcgacga tctcgaggta gttgcagtca     600
   acgacctcac cgacaacaag accctttcca cccttctcaa gttcgactcc atcatgggcc     660
   gccttggcca ggaagttgaa tacgacgatg actccatcac cgttggtggc aagcgcatcg     720
   ctgtttacgc agagcgcgat ccaaagaacc tggactgggc tgcacacaac gttgacatcg     780
   tgatcgagtc caccggcttc ttcaccgatg caaacgcggc taaggctcac atcgaagcag     840
   gtgccaagaa ggtcatcatc tccgcaccag caagcaacga agacgcaacc ttcgtttacg     900
   gtgtgaacca cgagtcctac gatcctgaga accacaacgt gatctccggc gcatcttgca 960
   ccaccaactg cctcgcacca atggcaaagg tcctaaacga caagttcggc atcgagaacg 1020
   gcctcatgac caccgttcac gcatacactg gcgaccagcg cctgcacgat gcacctcacc 1080
   gcgacctgcg tcgtgcacgt gcagcagcag tcaacatcgt tcctacctcc accggtgcag 1140
   ctaaggctgt tgctctggtt ctcccagagc tcaagggcaa gcttgacggc tacgcacttc 1200
   gcgttccagt tatcaccggt tccgcaaccg acctgacctt caacaccaag tctgaggtca 1260
   ccgttgagtc catcaacgct gcaatcaagg aagctgcagt cggcgagttc ggcgagaccc 1320
   tggcttactc cgaagagcca ctggtttcca ccgacatcgt ccacgattcc cacggctcca 1380
   tcttcgacgc tggcctgacc aaggtctccg gcaacaccgt caaggttgtt tcctggtacg 1440
   acaacgagtg gggctacacc tgccagctcc tgcgtctgac cgagctcgta gcttccaagc 1500
   tctaattagt tcacatcgct aacgtgggcg atcgatgctc acggtgatgt gtcatcccaa 1560
   tagcccgggg tgtgcctcgg cgcaccccgg gctattttgt gtctttaatc aatacaattg 1620
   aataccggtg ccagcgccac acaatgtgtg gcaatctggg acagtgcatc acattgcacc 1680
   agaagaattt tttaaacaat caaatctcca aggagtacgg catggctgtt aagaccctca 1740
   aggacttgct cgacgaaggc gtagacggac gccacgtcat cgttcgatct gacttcaatg 1800
   ttcccctcaa cgatgaccgc gagatcaccg ataagggccg aatcattgcc tccctaccaa 1860
   cccttaaagc actgagcgaa ggtggcgcaa aggtcatcgt catggctcac cttggccgcc 1920
   caaagggcga ggtcaacgag aagtactccc tcgcacctgt cgctgaggca ctctccgatg 1980
   agcttggcca gtacgttgca cttgc 2005
<210> 34
   <211> 461
   <212> DNA
   <213> Corynebacterium glutamicum
<220>
   <221> promoter
   <222> (1)..(461)
   <223> Pg3N3 Promoter
<220>
   <221> mutation
   <222> (265)..(265)
   <223> Nukleobase Thymin (t)
<220>
   <221> mutation
   <222> (269) .. (269)
   <223> Nukleobase Cytosin (c)
<220>
   <221> mutation
   <222> (290)..(290)
   <223> Nukleobase Thymin (t)
<220>
   <221> mutation
   <222> (292)..(292)
   <223> Nukleobase Adenin (a)
<220>
   <221> mutation
   <222> (362)..(362)
   <223> Nukleobase Adenin (a)
<400> 34
   tgaagcctaa aaacgaccga gcctattggg attaccattg aagccagtgt gagttgcatc      60
   acattggctt caaatctgag actttaattt gtggattcac gggggtgtaa tgtagttcat     120
   aattaacccc attcggggga gcagatcgta gtgcgaacga tttcaggttc gttccctgca     180
   aaaactattt agcgcaagtg ttggaaatgc ccccgtttgg ggtcaatgtc catttttgaa     240
   tgtgtctgta tgattttgca tctgttgcca aatctttgtt tccccgctat aattgaggac     300
   aggttgacac ggagttgact cgacgaatta tccaatgtga gtaggtttgg tgcgtgagtt     360
   gaaaaaattc gccatactcg cccttgggtt ctgtcagctc aagaattctt gagtgaccga     420
   tgctctgatt gacctaactg cttgacacat tgcatttcct a                         461
<210> 35
   <211> 1660
   <212> DNA
   <213> Corynebacterium glutamicum
<400> 35
   gccaattgcg tggcaatcag gctttacctc agcgaagaat tcgacgacgc cacccgcgtc 60
   tacaccgtcc gatcctttga tgccgcaact gaaagcatcg tggtggatgt ggttcaacac 120
   caccacgaaa gccccatgat gcgctggtca gacaccgtaa aaatcaacga caccctcgtg 180
   ctcaccggac cccgcccaca ctttgtcatc cccgaaggcg aacaagcagc actcttcctt 240
   gatgacaccg ccatccccgc tctcgccgct attttggatc aatggccaac agatcttcgt 300
   ggcaaaggat gggttgtcac tgacgatccc gcagccttcg atgaactacc cagcatcgac 360
   ggactggaac tgaacctgct cgcgccggga tcagatccaa ctgttcagcc acttgcccaa 420
   caggcatatg acctggaaaa cccagaaact tacgtggtgt gggcagccgg cgagcgagat 480
   gaaataaaat ccatccgcag gcacttccgc aagcaggtgg gattggaaaa agatgcagtg 540
   gccgtgtttg ggtactggaa atacaacacc accaacactc agatcgatgc agtccgcaaa 600
   gaaaactaca tgaagatgct ctctgaaggg ctacagctgg aaaacttcga cgacctctca 660
   ttggagattt aaggggtcga gtttttagaa tgggtgtttg cattgattgg cgtctgatga 720
   gctcttagaa gcgattctgg gagggcatga tttttgggat tcggagctga atgtggactg 780
   attttggggg aaccagactg gcgtgcccaa atgtcgtgtc ttcagtccta ggcgatgcat 840
   gcctgtctgg tttctcggct ctcaaaccga acaggagtgc ccacggctgg caaaaagttc 900
   agttcttggg cacgctagtc tggttttacc tttttgagtg ccccgaactg ccaaacgaga 960
   gtgcccatga ggggctaaat ctggatccgc ccgcacaaaa caactgccgc agcacaacga 1020
   atcaacgctg tgctgcggca gtcacttttt tgaaggatta acgccaaccc atggtcatga 1080
   gtaggccgat gatcatcaga ccgaagccga tgccatagtt ccatgcacca agatcagcca 1140
   tgaatgggat ctgtgggccc acgaggtagt taatgatcaa ccaggctagg ccgacgatca 1200
   tgaaggcaaa catgatgacc ttgtaccaca ttggggttcc ggcggaattg atcttaaccg 1260
   gggtgcggtt tgcgcttggg ttgcttgaaa ccggtgcggt ctcgttttta gttactcttg 1320
   cctttggcat tgttcacacc ttcataattt attgtcttgg aaaaacccca gcaactggta 1380
   cttatcttgt ttgagcttaa cgtatcagtt gccgggccag ctaccttgac ggtttccttg 1440
   ttggctattg cacgagtgca gcgagatcga attcgaagag tcgcacttgc acttgggcgt 1500
   ctttgcggaa gagggttgca ggggttgggg attggaatcc gattttgttt tgatccacga 1560
   gggctgcggt gtggatgggg tcgccgacga tcagggattg atctggggcg gtccagccag 1620
   cagcgcggag ggcactgatg gcctgttcgg tgaagcttgc 1660
<210> 36
   <211> 2872
   <212> DNA
   <213> Corynebacterium glutamicum
<220>
   <221> misc_feature
   <222> (3)..(8)
   <223> restriction site NsiI
<220>
   <221> promoter
   <222> (9) .. (469)
   <223> gap promoter according to SEQ ID NO. 2
<220>
   <221> RBS
   <222> (470)..(490)
   <223> RBS gap according to SEQ ID No. 16
<220>
   <221> gene
   <222> (491)..(1756)
   <223> coding sequence lysC
<220>
   <221> RBS
   <222> (1754)..(1775)
   <223> RBS gap according to SEQ ID No. 26
<220>
   <221> gene
   <222> (1776)..(2810)
   <223> coding sequence asd
<220>
   <221> terminator
   <222> (2814)..(2858)
   <223> gap terminator
<220>
   <221> misc_feature
   <222> (2865)..(2870)
   <223> restriction site AvrII
<400> 36
   cgatgcattg aagcctaaaa acgaccgagc ctattgggat taccattgaa gccagtgtga      60
   gttgcatcac attggcttca aatctgagac tttaatttgt ggattcacgg gggtgtaatg     120
   tagttcataa ttaaccccat tcgggggagc agatcgtagt gcgaacgatt tcaggttcgt     180
   tccctgcaaa aactatttag cgcaagtgtt ggaaatgccc ccgtttgggg tcaatgtcca     240
   tttttgaatg tgtctgtatg attttgcatc tgctgcgaaa tctttgtttc cccgctaaag     300
   ttgaggacag gttgacacgg agttgactcg acgaattatc caatgtgagt aggtttggtg     360
   cgtgagttgg aaaaattcgc catactcgcc cttgggttct gtcagctcaa gaattcttga     420
   gtgaccgatg ctctgattga cctaactgct tgacacattg catttcctac aatcggagag     480
   gagacacaac atggccctgg tcgtacagaa atatggcggt tcctcgcttg agagtgcgga     540
   acgcattaga aacgtcgctg aacggatcgt tgccaccaag aaggctggaa atgatgtcgt     600
   ggttgtctgc tccgcaatgg gagacaccac ggatgaactt ctggaacttg cagcggcagt     660
   gaatcccgtt ccgccagctc gtgaaatgga tatgctcctg actgctggtg agcgtatttc     720
   taacgctctc gtcgccatgg ctattgagtc ccttggcgca gaagcccaat ctttcacggg     780
   ctctcaggct ggtgtgctca ccaccgagcg ccacggaaac gcacgcattg ttgatgtcac     840
   tccaggtcgt gtgcgtgaag cactcgatga gggcaagatc tgcattgttg ctggtttcca     900
   gggtgttaat aaagaaaccc gcgatgtcac cacgttgggt cgtggtggtt ctgacaccac     960
   tgcagttgcg ttggcagctg ctttgaacgc tgatgtgtgt gagatttact cggacgttga    1020
   cggtgtgtat accgctgacc cgcgcatcgt tcctaatgca cagaagctgg aaaagctcag    1080
   cttcgaagaa atgctggaac ttgctgctgt tggctccaag attttggtgc tgcgcagtgt    1140
   tgaatacgct cgtgcattca atgtgccact tcgcgtacgc tcgtcttata gtaatgatcc    1200
   cggcactttg attgccggct ctatggagga tattcctgtg gaagaagcag tccttaccgg    1260
   tgtcgcaacc gacaagtccg aagccaaagt aaccgttctg ggtatttccg ataagccagg    1320
   cgaggctgcg aaggttttcc gtgcgttggc tgatgcagaa atcaacattg acatggttct    1380
   gcagaacgtc tcttctgtag aagacggcac caccgacatc atcttcacct gccctcgttc    1440
   cgacggccgc cgcgcgatgg agatcttgaa gaagcttcag gttcagggca actggaccaa    1500
   tgtgctttac gacgaccagg tcggcaaagt ctccctcgtg ggtgctggca tgaagtctca      1560
   cccaggtgtt accgcagagt tcatggaagc tctgcgcgat gtcaacgtga acatcgaatt      1620
   gatttccacc tctgagattc gtatttccgt gctgatccgt gaagatgatc tggatgctgc      1680
   tgcacgtgca ttgcatgagc agttccagct gggcggcgaa gacgaagccg tcgtttatgc      1740
   aggcaccgga cgctgacgtc tagaggagac acaacatgac caccatcgca gttgttggtg      1800
   caaccggcca ggtcggccag gttatgcgca cccttttgga agagcgcaat ttcccagctg      1860
   acactgttcg tttctttgct tccccacgtt ccgcaggccg taagattgaa ttccgtggca      1920
   cggaaatcga ggtagaagac attactcagg caaccgagga gtccctcaag gacatcgacg      1980
   ttgcgttgtt ctccgctgga ggcaccgctt ccaagcagta cgctccactg ttcgctgctg      2040
   caggcgcgac tgttgtggat aactcttctg cttggcgcaa ggacgacgag gttccactaa      2100
   tcgtctctga ggtgaaccct tccgacaagg attccctggt caagggcatt attgcgaacc      2160
   ctaactgcac caccatggct gcgatgccag tgctgaagcc acttcacgat gccgctggtc      2220
   ttgtaaagct tcacgtttcc tcttaccagg ctgtttccgg ttctggtctt gcaggtgtgg      2280
   aaaccttggc aaagcaggtt gctgcagttg gagaccacaa cgttgagttc gtccatgatg      2340
   gacaggctgc tgacgcaggc gatgtcggac cttatgtttc accaatcgct tacaacgtgc      2400
   tgccattcgc cggaaacctc gtcgatgacg gcaccttcga aaccgatgaa gagcagaagc      2460
   tgcgcaacga atcccgcaag attctcggtc tcccagacct caaggtctca ggcacctgcg      2520
   tccgcgtgcc ggttttcacc ggccacacgc tgaccattca cgccgaattc gacaaggcaa      2580
   tcaccgtgga ccaggcgcag gagatcttgg gtgccgcttc aggcgtcaag cttgtcgatg      2640
   tcccaacccc acttgcagct gccggcattg acgaatccct cgttggacgc atccgtcagg      2700
   actccactgt cgacgataac cgcggtctgg ttctcgtcgt atctggcgac aacctccgca      2760
   agggtgctgc gctaaacacc atccagatcg ctgagctgct ggttaagtaa tattcccaat      2820
   agcccggggt gtgcctcggc gcaccccggg ctattttgat atcacctagg cg              2872
<210> 37
   <211> 2872
   <212> DNA
   <213> Corynebacterium glutamicum
<220>
   <221> misc_feature
   <222> (3)..(8)
   <223> restriction site NsiI
<220>
   <221> promoter
   <222> (9) .. (469)
   <223> gap promoter according to SEQ ID NO. 3
<220>
   <221> RBS
   <222> (470)..(490)
   <223> RBS gap according to SEQ ID No. 16
<220>
   <221> gene
   <222> (491)..(1756)
   <223> coding sequence lysC
<220>
   <221> RBS
   <222> (1754)..(1775)
   <223> RBS gap according to SEQ ID No. 26
<220>
   <221> gene
   <222> (1776)..(2810)
   <223> coding sequence asd
<220>
   <221> terminator
   <222> (2814)..(2858)
   <223> gap terminator
<220>
   <221> misc_feature
   <222> (2865)..(2870)
   <223> restriction site AvrII
<400> 37
   cgatgcattg aagcctaaaa acgaccgagc ctattgggat taccattgaa gccagtgtga      60
   gttgcatcac attggcttca aatctgagac tttaatttgt ggattcacgg gggtgtaatg     120
   tagttcataa ttaaccccat tcgggggagc agatcgtagt gcgaacgatt tcaggttcgt     180
   tccctgcaaa aactatttag cgcaagtgtt ggaaatgccc ccgtttgggg tcaatgtcca     240
   tttttgaatg tgtctgtatg attttgcatc tgctgcgaaa tctttgtttc cccgctaaag     300
   ttgaggacag gttgacacgg agttgactcg acgaattatc caatgtgagt aggtttggtg     360
   cgtgagttga aaaaattcgc catactcgcc cttgggttct gtcagctcaa gaattcttga     420
   gtgaccgatg ctctgattga cctaactgct tgacacattg catttcctac aatcggagag     480
   gagacacaac atggccctgg tcgtacagaa atatggcggt tcctcgcttg agagtgcgga     540
   acgcattaga aacgtcgctg aacggatcgt tgccaccaag aaggctggaa atgatgtcgt     600
   ggttgtctgc tccgcaatgg gagacaccac ggatgaactt ctggaacttg cagcggcagt     660
   gaatcccgtt ccgccagctc gtgaaatgga tatgctcctg actgctggtg agcgtatttc     720
   taacgctctc gtcgccatgg ctattgagtc ccttggcgca gaagcccaat ctttcacggg     780
   ctctcaggct ggtgtgctca ccaccgagcg ccacggaaac gcacgcattg ttgatgtcac     840
   tccaggtcgt gtgcgtgaag cactcgatga gggcaagatc tgcattgttg ctggtttcca     900
   gggtgttaat aaagaaaccc gcgatgtcac cacgttgggt cgtggtggtt ctgacaccac     960
   tgcagttgcg ttggcagctg ctttgaacgc tgatgtgtgt gagatttact cggacgttga 1020
   cggtgtgtat accgctgacc cgcgcatcgt tcctaatgca cagaagctgg aaaagctcag 1080
   cttcgaagaa atgctggaac ttgctgctgt tggctccaag attttggtgc tgcgcagtgt 1140
   tgaatacgct cgtgcattca atgtgccact tcgcgtacgc tcgtcttata gtaatgatcc 1200
   cggcactttg attgccggct ctatggagga tattcctgtg gaagaagcag tccttaccgg 1260
   tgtcgcaacc gacaagtccg aagccaaagt aaccgttctg ggtatttccg ataagccagg 1320
   cgaggctgcg aaggttttcc gtgcgttggc tgatgcagaa atcaacattg acatggttct 1380
   gcagaacgtc tcttctgtag aagacggcac caccgacatc atcttcacct gccctcgttc 1440
   cgacggccgc cgcgcgatgg agatcttgaa gaagcttcag gttcagggca actggaccaa 1500
   tgtgctttac gacgaccagg tcggcaaagt ctccctcgtg ggtgctggca tgaagtctca 1560
   cccaggtgtt accgcagagt tcatggaagc tctgcgcgat gtcaacgtga acatcgaatt 1620
   gatttccacc tctgagattc gtatttccgt gctgatccgt gaagatgatc tggatgctgc 1680
   tgcacgtgca ttgcatgagc agttccagct gggcggcgaa gacgaagccg tcgtttatgc 1740
   aggcaccgga cgctgacgtc tagaggagac acaacatgac caccatcgca gttgttggtg 1800
   caaccggcca ggtcggccag gttatgcgca cccttttgga agagcgcaat ttcccagctg 1860
   acactgttcg tttctttgct tccccacgtt ccgcaggccg taagattgaa ttccgtggca 1920
   cggaaatcga ggtagaagac attactcagg caaccgagga gtccctcaag gacatcgacg 1980
   ttgcgttgtt ctccgctgga ggcaccgctt ccaagcagta cgctccactg ttcgctgctg 2040
   caggcgcgac tgttgtggat aactcttctg cttggcgcaa ggacgacgag gttccactaa 2100
   tcgtctctga ggtgaaccct tccgacaagg attccctggt caagggcatt attgcgaacc 2160
   ctaactgcac caccatggct gcgatgccag tgctgaagcc acttcacgat gccgctggtc 2220
   ttgtaaagct tcacgtttcc tcttaccagg ctgtttccgg ttctggtctt gcaggtgtgg 2280
   aaaccttggc aaagcaggtt gctgcagttg gagaccacaa cgttgagttc gtccatgatg 2340
   gacaggctgc tgacgcaggc gatgtcggac cttatgtttc accaatcgct tacaacgtgc 2400
   tgccattcgc cggaaacctc gtcgatgacg gcaccttcga aaccgatgaa gagcagaagc 2460
   tgcgcaacga atcccgcaag attctcggtc tcccagacct caaggtctca ggcacctgcg 2520
   tccgcgtgcc ggttttcacc ggccacacgc tgaccattca cgccgaattc gacaaggcaa 2580
   tcaccgtgga ccaggcgcag gagatcttgg gtgccgcttc aggcgtcaag cttgtcgatg 2640
   tcccaacccc acttgcagct gccggcattg acgaatccct cgttggacgc atccgtcagg 2700
   actccactgt cgacgataac cgcggtctgg ttctcgtcgt atctggcgac aacctccgca 2760
   agggtgctgc gctaaacacc atccagatcg ctgagctgct ggttaagtaa tattcccaat 2820
   agcccggggt gtgcctcggc gcaccccggg ctattttgat atcacctagg cg 2872
<210> 38
   <211> 2872
   <212> DNA
   <213> Corynebacterium glutamicum
<220>
   <221> misc_feature
   <222> (3)..(8)
   <223> restriction site NsiI
<220>
   <221> promoter
   <222> (9) .. (469)
   <223> gap promoter according to SEQ ID NO. 34
<220>
   <221> RBS
   <222> (470)..(490)
   <223> RBS gap according to SEQ ID No. 16
<220>
   <221> gene
   <222> (491)..(1756)
   <223> coding sequence lysC
<220>
   <221> RBS
   <222> (1754)..(1775)
   <223> RBS gap according to SEQ ID No. 26
<220>
   <221> gene
   <222> (1776)..(2810)
   <223> coding sequence asd
<220>
   <221> terminator
   <222> (2814)..(2858)
   <223> gap terminator
<220>
   <221> misc_feature
   <222> (2865)..(2870)
   <223> restriction site AvrII
<400> 38
   cgatgcattg aagcctaaaa acgaccgagc ctattgggat taccattgaa gccagtgtga      60
   gttgcatcac attggcttca aatctgagac tttaatttgt ggattcacgg gggtgtaatg     120
   tagttcataa ttaaccccat tcgggggagc agatcgtagt gcgaacgatt tcaggttcgt     180
   tccctgcaaa aactatttag cgcaagtgtt ggaaatgccc ccgtttgggg tcaatgtcca     240
   tttttgaatg tgtctgtatg attttgcatc tgttgccaaa tctttgtttc cccgctataa     300
   ttgaggacag gttgacacgg agttgactcg acgaattatc caatgtgagt aggtttggtg     360
   cgtgagttga aaaaattcgc catactcgcc cttgggttct gtcagctcaa gaattcttga     420
   gtgaccgatg ctctgattga cctaactgct tgacacattg catttcctac aatcggagag 480
   gagacacaac atggccctgg tcgtacagaa atatggcggt tcctcgcttg agagtgcgga 540
   acgcattaga aacgtcgctg aacggatcgt tgccaccaag aaggctggaa atgatgtcgt 600
   ggttgtctgc tccgcaatgg gagacaccac ggatgaactt ctggaacttg cagcggcagt 660
   gaatcccgtt ccgccagctc gtgaaatgga tatgctcctg actgctggtg agcgtatttc 720
   taacgctctc gtcgccatgg ctattgagtc ccttggcgca gaagcccaat ctttcacggg 780
   ctctcaggct ggtgtgctca ccaccgagcg ccacggaaac gcacgcattg ttgatgtcac 840
   tccaggtcgt gtgcgtgaag cactcgatga gggcaagatc tgcattgttg ctggtttcca 900
   gggtgttaat aaagaaaccc gcgatgtcac cacgttgggt cgtggtggtt ctgacaccac 960
   tgcagttgcg ttggcagctg ctttgaacgc tgatgtgtgt gagatttact cggacgttga 1020
   cggtgtgtat accgctgacc cgcgcatcgt tcctaatgca cagaagctgg aaaagctcag 1080
   cttcgaagaa atgctggaac ttgctgctgt tggctccaag attttggtgc tgcgcagtgt 1140
   tgaatacgct cgtgcattca atgtgccact tcgcgtacgc tcgtcttata gtaatgatcc 1200
   cggcactttg attgccggct ctatggagga tattcctgtg gaagaagcag tccttaccgg 1260
   tgtcgcaacc gacaagtccg aagccaaagt aaccgttctg ggtatttccg ataagccagg 1320
   cgaggctgcg aaggttttcc gtgcgttggc tgatgcagaa atcaacattg acatggttct 1380
   gcagaacgtc tcttctgtag aagacggcac caccgacatc atcttcacct gccctcgttc 1440
   cgacggccgc cgcgcgatgg agatcttgaa gaagcttcag gttcagggca actggaccaa 1500
   tgtgctttac gacgaccagg tcggcaaagt ctccctcgtg ggtgctggca tgaagtctca 1560
   cccaggtgtt accgcagagt tcatggaagc tctgcgcgat gtcaacgtga acatcgaatt 1620
   gatttccacc tctgagattc gtatttccgt gctgatccgt gaagatgatc tggatgctgc 1680
   tgcacgtgca ttgcatgagc agttccagct gggcggcgaa gacgaagccg tcgtttatgc 1740
   aggcaccgga cgctgacgtc tagaggagac acaacatgac caccatcgca gttgttggtg 1800
   caaccggcca ggtcggccag gttatgcgca cccttttgga agagcgcaat ttcccagctg 1860
   acactgttcg tttctttgct tccccacgtt ccgcaggccg taagattgaa ttccgtggca 1920
   cggaaatcga ggtagaagac attactcagg caaccgagga gtccctcaag gacatcgacg 1980
   ttgcgttgtt ctccgctgga ggcaccgctt ccaagcagta cgctccactg ttcgctgctg 2040
   caggcgcgac tgttgtggat aactcttctg cttggcgcaa ggacgacgag gttccactaa 2100
   tcgtctctga ggtgaaccct tccgacaagg attccctggt caagggcatt attgcgaacc 2160
   ctaactgcac caccatggct gcgatgccag tgctgaagcc acttcacgat gccgctggtc 2220
   ttgtaaagct tcacgtttcc tcttaccagg ctgtttccgg ttctggtctt gcaggtgtgg 2280
   aaaccttggc aaagcaggtt gctgcagttg gagaccacaa cgttgagttc gtccatgatg 2340
   gacaggctgc tgacgcaggc gatgtcggac cttatgtttc accaatcgct tacaacgtgc 2400
   tgccattcgc cggaaacctc gtcgatgacg gcaccttcga aaccgatgaa gagcagaagc 2460
   tgcgcaacga atcccgcaag attctcggtc tcccagacct caaggtctca ggcacctgcg 2520
   tccgcgtgcc ggttttcacc ggccacacgc tgaccattca cgccgaattc gacaaggcaa 2580
   tcaccgtgga ccaggcgcag gagatcttgg gtgccgcttc aggcgtcaag cttgtcgatg 2640
   tcccaacccc acttgcagct gccggcattg acgaatccct cgttggacgc atccgtcagg 2700
   actccactgt cgacgataac cgcggtctgg ttctcgtcgt atctggcgac aacctccgca 2760
   agggtgctgc gctaaacacc atccagatcg ctgagctgct ggttaagtaa tattcccaat 2820
   agcccggggt gtgcctcggc gcaccccggg ctattttgat atcacctagg cg 2872
<210> 39
   <211> 20
   <212> DNA
   <213> Corynebacterium glutamicum
<220>
   <221> misc_feature
   <222> (1)..(20)
   <223> Primer cg0054_9.p
<400> 39
   ccacccatca ccctcacttc 20
<210> 40
   <211> 20
   <212> DNA
   <213> Corynebacterium glutamicum
<220>
   <221> misc_feature
   <222> (1)..(20)
   <223> Primer cg0054_10.p
<400> 40
   gcactctcgt ttggcagttc 20
<210> 41
   <211> 20
   <212> DNA
   <213> Corynebacterium glutamicum
<220>
   <221> misc_feature
   <222> (1)..(20)
   <223> Primer QlysC_WT_P2
<400> 41
   aagtgccggg atcattacta 20
<210> 42
   <211> 2549
   <212> DNA
   <213> Corynebacterium glutamicum
<220>
   <221> misc_feature
   <222> (2)..(7)
   <223> Schnittstelle BamHI
<220>
   <221> misc_feature
   <222> (2543)..(2548)
   <223> Schnittstelle XbaI
<400> 42
   gggatcccgc tgcagtcgag gcttcagagg ttttattgct cgacttcacg tgtgctgact      60
   tcgcgtgctc aacttctcat gaggcgacat cagtcgaaaa gccggtcatt gtcgtatgaa     120
   gtctgcgcac gagaagttga gcggaagaag tcggtgttaa gaagtcgacc gcgtgaagtc     180
   gccctttagg agaattctga ctaactggag ccaaaacttg atccactcga gagctgtgca     240
   gtctcttttt ccttcaattc tgcctgctcg agctcgtaga agtagaggtc tacttcagtt     300
   ggttcacctt gcacacaagc atgaagtagt gggtaggtcg agttgttaaa tgcggtgtag     360
   aaggggagta gttcgctagc aaaggttaat ttggagtcgc tgtactgcgg gttctcgggt     420
   ggagtattcc cggaggattc aagaaatctt gacgcatctt tgatgaggta tgtttggaat     480
   tcgtcggcac cttcctcgcc ggagaggtag taggagttgt cgtaatttgg aacccagatg     540
   gcaaatcgtg cgttttcgat tgcgtccagg agttcctcta cgttgtatct cgcacttgtt     600
   gcagcggaag cgactcggtt gccgatgtct ccgtatgcag tgagcgtggc gtttccgagg     660
   ggaacttgat cagaggaata caccatggag ccgatgtcag aggcgactgc gggcagatcc     720
   ttttgaagct gtttcacaat ttctttgccc agttcgcggc ggatctggaa ccacttttgc     780
   atgcgatcgt cgtcagagtg gttcatgtga aaaatacact caccatctca atggtcatgg     840
   tgaaggcctg tactggctgc gacagcatgg aactcagtgc aatggctgta aggcctgcac     900
   caacaatgat tgagcgaagc tccaaaatgt cctccccggg ttgatattag atttcataaa     960
   tatactaaaa atcttgagag tttttccgtt gaaaactaaa aagctgggaa ggtgaatcga    1020
   atgcattgaa gcctaaaaac gaccgagcct attgggatta ccattgaagc cagtgtgagt    1080
   tgcatcacat tggcttcaaa tctgagactt taatttgtgg attcacgggg gtgtaatgta    1140
   gttcataatt aaccccattc gggggagcag atcgtagtgc gaacgatttc aggttcgttc    1200
   cctgcaaaaa ctatttagcg caagtgttgg aaatgccccc gtttggggtc aatgtccatt    1260
   tttgaatgtg tctgtatgat tttgcatctg ctgcgaaatc tttgtttccc cgctaaagtt    1320
   gaggacaggt tgacacggag ttgactcgac gaattatcca atgtgagtag gtttggtgcg 1380
   tgagttgaaa aaattcgcca tactcgccct tgggttctgt cagctcaaga attcttgagt 1440
   gaccgatgct ctgattgacc taactgcttg acacattgca tttcctacaa tcgcgagagg 1500
   agacacaaca tgacctcagc atctgcccca agctttaacc ccggcaaggg tcccggctca 1560
   gcagtcggaa ttgccctttt aggattcgga acagtcggca ctgaggtgat gcgtctgatg 1620
   accgagtacg gtgatgaact tgcgcaccgc attggtggcc cactggaggt tcgtggcatt 1680
   gctgtttctg atatctcaaa gccacgtgaa ggcgttgcac ctgagctgct cactgaggac 1740
   gcttttgcac tcatcgagcg cgaggatgtt gacatcgtcg ttgaggttat cggcggcatt 1800
   gagtacccac gtgaggtagt tctcgcagct ctgaaggccg gcaagtctgt tgttaccgcc 1860
   aataaggctc ttgttgcagc tcactctgct gagcttgctg atgcagcgga agccgcaaac 1920
   gttgacctgt acttcgaggc tgctgttgca ggcgcaattc cagtggttgg cccactgcgt 1980
   cgctccctgg ctggcgatca gatccagtct gtgatgggca tcgttaacgg caccaccaac 2040
   ttcatcttgg acgccatgga ttccaccggc gctgactatg cagattcttt ggctgaggca 2100
   actcgtttgg gttacgccga agctgatcca actgcagacg tcgaaggcca tgacgccgca 2160
   tccaaggctg caattttggc atccatcgct ttccacaccc gtgttaccgc ggatgatgtg 2220
   tactgcgaag gtatcagcaa catcagcgct gccgacattg aggcagcaca gcaggcaggc 2280
   cacaccatca agttgttggc catctgtgag aagttcacca acaaggaagg aaagtcggct 2340
   atttctgctc gcgtgcaccc gactctatta cctgtgtccc acccactggc gtcggtaaac 2400
   aagtccttta atgcaatctt tgttgaagca gaagcagctg gtcgcctgat gttctacgga 2460
   aacggtgcag gtggcgcgcc aaccgcgtct gctgtgcttg gcgacgtcgt tggtgccgca 2520
   cgaaacaagg tgcacggtgg gctctagag 2549
<210> 43
   <211> 2442
   <212> DNA
   <213> Corynebacterium glutamicum
<220>
   <221> misc_feature
   <222> (2)..(7)
   <223> Schnittstelle BamHI
<220>
   <221> misc_feature
   <222> (2436)..(2441)
   <223> Schnittstelle XbaI
<400> 43
   gggatcccgc ctggttgctg atcgtatcgt tcagttcgcc aagcttgttg gccctgagaa 60
   cgtcattgcg tccactgact gtggtctggg cggacgtctg cattcccaga tcgcatgggc 120
   aaagctggag tccctagtag agggcgctcg cattgcatca aaggaactgt tctaagctag 180
   acaacgaggg ttgctagtct aagcagcaaa atgagcggct gttgttcctt caggaaaatt 240
   atctgaagga acaatagccg ctcattttat gtcagtgtgc ttttaagcgt cgacgttgat 300
   gccaaactgg gtgagcatgt cacgcagagt ctgcttgaac gatgggtcga cggtcaccat 360
   gaaggatgct gcggtggaag caagagtggt gattgcgagc aatccctgca gtaccgcaaa 420
   aggaatggca agccattctg gtggggagac aatggaacca atcactggca tttcggacaa 480
   ccgatcgatc agtgcttgct gctcttcgct gatttcatcg tcatcatcgc tggtgtcttt 540
   gccggaggag ctcaagcttg gggccgcagg aatgtcgcca ttgctgagca ttgagctgcc 600
   ttcagagctg cctggccagg tttcgtttcc atcgactgga tttccatcat catcaaggat 660
   ctgtgatgag gtgatgttgt ctgagagctg tgtcagtgcg tcagaggact gagcctgggc 720
   aactggagtg aacacggaca atgccacagc gcttgctgta acaagggtca aagtacttcg 780
   acgcaaagac aaaacttttc tcctggcaat aaatatgcgg atttactatg gaaacaagat 840
   agaagattgg atagcgaaag ctatcctcaa ctcgtggaaa gtgtagtgcc acaaccacag 900
   tattggctag aaaacaatct atagcattgt tctacaaaga gctatgcatt gaagcctaaa 960
   aacgaccgag cctattggga ttaccattga agccagtgtg agttgcatca cattggcttc 1020
   aaatctgaga ctttaatttg tggattcacg ggggtgtaat gtagttcata attaacccca 1080
   ttcgggggag cagatcgtag tgcgaacgat ttcaggttcg ttccctgcaa aaactattta 1140
   gcgcaagtgt tggaaatgcc cccgtttggg gtcaatgtcc atttttgaat gtgtctgtat 1200
   gattttgcat ctgctgcgaa atctttgttt ccccgctaaa gttgaggaca ggttgacacg 1260
   gagttgactc gacgaattat ccaatgtgag taggtttggt gcgtgagttg aaaaaattcg 1320
   ccatactcgc ccttgggttc tgtcagctca agaattcttg agtgaccgat gctctgattg 1380
   acctaactgc ttgacacatt gcatttccta caatcgcgat ttagaggaga cacaacatga 1440
   gtgaaacata cgtgtctgag aaaagtccag gagtgatggc tagcggagcg gagctgattc 1500
   gtgccgccga cattcaaacg gcgcaggcac gaatttcctc cgtcattgca ccaactccat 1560
   tgcagtattg ccctcgtctt tctgaggaaa ccggagcgga aatctacctt aagcgtgagg 1620
   atctgcagga tgttcgttcc tacaagatcc gcggtgcgct gaactctgga gcgcagctca 1680
   cccaagagca gcgcgatgca ggtatcgttg ccgcatctgc aggtaaccat gcccagggcg 1740
   tggcctatgt gtgcaagtcc ttgggcgttc agggacgcat ctatgttcct gtgcagactc 1800
   caaagcaaaa gcgtgaccgc atcatggttc acggcggaga gtttgtctcc ttggtggtca 1860
   ctggcaataa cttcgacgaa gcatcggctg cagcgcatga agatgcagag cgcaccggcg 1920
   caacgctgat cgagcctttc gatgctcgca acaccgtcat cggtcagggc accgtggctg 1980
   ctgagatctt gtcgcagctg acttccatgg gcaagagtgc agatcacgtg atggttccag     2040
   tcggcggtgg cggacttctt gcaggtgtgg tcagctacat ggctgatatg gcacctcgca     2100
   ctgcgatcgt tggtatcgaa ccagcgggag cagcatccat gcaggctgca ttgcacaatg     2160
   gtggaccaat cactttggag actgttgatc cctttgtgga cggcgcagca gtcaaacgtg     2220
   tcggagatct caactacacc atcgtggaga agaaccaggg tcgcgtgcac atgatgagcg     2280
   cgaccgaggg cgctgtgtgt actgagatgc tcgatcttta ccaaaacgaa ggcatcatcg     2340
   cggagcctgc tggcgcgctg tctatcgctg ggttgaagga aatgtccttt gcacctggtt     2400
   ctgtcgtggt gtgcatcatc tctggtggca acagctctag ag                        2442
<210> 44
   <211> 2072
   <212> DNA
   <213> Corynebacterium glutamicum
<220>
   <221> misc_feature
   <222> (2)..(7)
   <223> Schnittstelle HindIII
<220>
   <221> misc_feature
   <222> (2066)..(2071)
   <223> Schnittstelle BamHI
<400> 44
   caagcttcac catccggcca tatcaaggtt gaccgcgtct cccgcaccaa catccccggt      60
   gtgtacgcag caggtgactg tactgaccta ttcccactgg cgtccgttgc agcgatgcag     120
   ggccgtatcg ccatgtatca cgcactcggt gaaggcgtga gccccatccg tttgaagact     180
   gttgccaccg cagtgtttac ccgcccagag atcgcagcag taggtatcac ccatgcacaa     240
   gttgattccg gcgaagtgtc tgctcgcgtg attgtgcttc ctttggctac taacccacgc     300
   gccaagatgc gttccctgcg ccacggtttt gtgaagctgt tctgccgccg taactctggc     360
   ctgatcatcg gtggtgtcgt ggtggcaccg accgcgtctg agctgatcct accgatcgct     420
   gtggcagtga ccaaccgtct gacagttgct gatctggctg ataccttcgc ggtgtaccca     480
   tcattgtcag gttcgattac tgaagcagca cgtcagctgg ttcaacatga tgatctaggc     540
   taatttttct gagtcttaga ttttgagaaa acccaggatt gctttgtgca ctcctgggtt     600
   ttcactttgt taagcagttt tggggaaaag tgcaaagttt gcaaagttta gaaatatttt     660
   aagaggtaag atgtctgcag gtggaagcgt ttaaatgcgt taaacttggc caaatgtggc     720
   aacctttgca aggtgaaaaa ctggggcggg gttagatcct gggggtttta tttcattcac   780
   tttggcttga agtcgtgcag gatactatgc attgaagcct aaaaacgacc gagcctattg     840
   ggattaccat tgaagccagt gtgagttgca tcacattggc ttcaaatctg agactttaat 900
   ttgtggattc acgggggtgt aatgtagttc ataattaacc ccattcgggg gagcagatcg 960
   tagtgcgaac gatttcaggt tcgttccctg caaaaactat ttagcgcaag tgttggaaat 1020
   gcccccgttt ggggtcaatg tccatttttg aatgtgtctg tatgattttg catctgctgc 1080
   gaaatctttg tttccccgct aaagttgagg acaggttgac acggagttga ctcgacgaat 1140
   tatccaatgt gagtaggttt ggtgcgtgag ttgaaaaaat tcgccatact cgcccttggg 1200
   ttctgtcagc tcaagaattc ttgagtgacc gatgctctga ttgacctaac tgcttgacac 1260
   attgcatttc ctacaatctt tagaggagac acaacatgtc gactcacaca tcttcaacgc 1320
   ttccagcatt caaaaagatc ttggtagcaa accgcggcga aatcgcggtc cgtgctttcc 1380
   gtgcagcact cgaaaccggt gcagccacgg tagctattta cccccgtgaa gatcggggat 1440
   cattccaccg ctcttttgct tctgaagctg tccgcattgg taccgaaggc tcaccagtca 1500
   aggcgtacct ggacatcgat gaaattatcg gtgcagctaa aaaagttaaa gcagatgcca 1560
   tttacccggg atacggcttc ctgtctgaaa atgcccagct tgcccgcgag tgtgcggaaa 1620
   acggcattac ttttattggc ccaaccccag aggttcttga tctcaccggt gataagtctc 1680
   gcgcggtaac cgccgcgaag aaggctggtc tgccagtttt ggcggaatcc accccgagca 1740
   aaaacatcga tgagatcgtt aaaagcgctg aaggccagac ttaccccatc tttgtgaagg 1800
   cagttgccgg tggtggcgga cgcggtatgc gttttgttgc ttcacctgat gagcttcgca 1860
   aattagcaac agaagcatct cgtgaagctg aagcggcttt cggcgatggc gcggtatatg 1920
   tcgaacgtgc tgtgattaac cctcagcata ttgaagtgca gatccttggc gatcacactg 1980
   gagaagttgt acacctttat gaacgtgact gctcactgca gcgtcgtcac caaaaagttg 2040
   tcgaaattgc gccagcacag catttggatc cc 2072
<210> 45
   <211> 28
   <212> DNA
   <213> Corynebacterium glutamicum
<220>
   <221> misc_feature
   <222> (1)..(28)
   <223> Primer Hom_Xl-A1
<400> 45
   gatctagacg tccaggagtt cctctacg 28
<210> 46
   <211> 20
   <212> DNA
   <213> Corynebacterium glutamicum
<220>
   <221> misc_feature
   <222> (1)..(20)
   <223> Primer LC-hom2
<400> 46
   tggcgtccaa gatgaagttg 20
<210> 47
   <211> 33
   <212> DNA
   <213> Corynebacterium glutamicum
<220>
   <221> misc_feature
   <222> (1)..(33)
   <223> Primer Pgap3_1.p
<400> 47
   cgagtactat gcattgaagc ctaaaaacga ccg 33
<210> 48
   <211> 20
   <212> DNA
   <213> Corynebacterium glutamicum
<220>
   <221> misc_feature
   <222> (1)..(20)
   <223> Primer ilvA-int-rev2
<400> 48
   accgcggatc ttgtaggaac 20
<210> 49
   <211> 18
   <212> DNA
   <213> Corynebacterium glutamicum
<220>
   <221> misc_feature
   <222> (1)..(18)
   <223> Primer pycUP_3.p
<400> 49
   agcacgtcag ctggttca 18
<210> 50
   <211> 20
   <212> DNA
   <213> Corynebacterium glutamicum
<220>
   <221> misc_feature
   <222> (1)..(20)
   <223> Primer 2xpyc-1
<400> 50
   aggtacgcct tgactggtga 20